# EUROPEAN PATENT SPECIFICATION

(11) **EP 0 734 445 B1**
(45) Date of publication and mention of the grant of the patent: **05.07.2006**
(21) Application number: 95903539.5
(22) Date of filing: 21.11.1994
(51) Int. Cl.: C12N 15/82, C12N 15/60, C12N 15/11, A01H 5/00, A01H 5/10

(54) **CHIMERIC GENES AND METHODS FOR INCREASING THE LYSINE CONTENT OF THE SEEDS OF CORN, SOYBEAN AND RAPESEED PLANTS**
CHIMAERE GENE UND VERFAHREN ZUR STEIGERUNG DES LYSIN-GEHALTS DER SAMEN VON MAIS, SOJA UND RAPS PFLANZEN
GENES CHIMERES ET PROCEDES D'AUGMENTATION DE LA TENEUR EN LYSINE DE SEMENCES DE BLE, DE SOJA ET DE COLZA

(30) Priority: 30.11.1993 US 160117; 17.06.1994 US 261661
(43) Date of publication of application: 02.10.1996
(73) Proprietor: E.I. DU PONT DE NEMOURS AND COMPANY, Wilmington, Delaware 19898 (US)
(72) Inventor: FALCO, Saverio, Carl, Arden, DE 19810 (US); KEELER, Sharon, Jo, Newark, DE 19711-2636 (US); RICE, Janet, Ann, Wilmington, DE 19803 (US)
(74) Representative: Dzieglewska, Hanna Eva
(86) International application number: PCT/US1994/013190
(87) International publication number: WO 1995/015392

(56) References cited:
- WO-A-93/03160
- WO-A-93/19190
- PLANT PHYSIOLOGY SUPPLEMENT, vol.105, no.1, May 1994 page 115 SANDERS, C., ET AL. 'Overproduction of lysine in seeds of Brassica napus cv westar by genetic engineering of the biosynthetic pathway'
- JOURNAL OF CELLULAR BIOCHEMISTRY SUPPLEMENT, vol.18A, 1994 page 79 FALCO, S.C., ET AL. 'Transgenic crops with improved amino acid composition' & KEYSTONE SYMPOSIUM ON IMPROVED CROP AND PLANT PRODUCTS THROUGH BIOTECHNOLOGY, KEYSTONE, COLORADO, USA, JANUARY 9-16, 1994.
- JOURNAL OF CELLULAR BIOCHEMISTRY SUPPLEMENT, vol.18A, 1994 page 107 RICE, J.A., ET AL. 'Expression of synthetic high lysine seed storage proteins can significantly increase the accumulated levels of lysine in mature seeds of transgenic crop plants' & KEYSTONE SYMPOSIUM ON IMPROVED CROP AND PLANT PRODUCTS THROUGH BIOTECHNOLOGY, KEYSTONE, COLORADO, USA, JANUARY 9-16, 1994.

## Description

### TECHNICAL FIELD

This invention relates to three chimeric genes, the first encoding dihydrodipicolinic acid synthase (DHDPS), which is insensitive to inhibition by lysine and operably linked to a plant chloroplast transit sequence, a second encoding a lysine-rich protein, and a third encoding a plant lysine ketoglutarate reductase, all operably linked to plant seed-specific regulatory sequences. Methods for their use to produce increased levels of lysine in the seeds of transformed plants are provided. Also provided are transformed corn, rapeseed and soybean plants wherein the seeds accumulate lysine to higher levels than untransformed plants.

### BACKGROUND OF THE INVENTION

Human food and animal feed derived from many grains are deficient in some of the ten essential amino acids which are required in the animal diet. In corn (Zea mays L.), lysine is the most limiting amino acid for the dietary requirements of many animals. Meal derived from other crop plants, e.g., soybean (Glycine max L.) or Canola (Brassica napus), is used as an additive to corn based animal feeds to supplement this lysine deficiency. Also, additional lysine, produced via fermentation of microbes, is used as a supplement in animal feeds. An increase in the lysine content of meal derived from plant sources would reduce or eliminate the need to supplement mixed grain feeds with microbially produced lysine.

The amino acid content of seeds is determined primarily (90-99%) by the amino acid composition of the proteins in the seed and to a lesser extent (1-10%) by the free amino acid pools. The quantity of total protein in seeds varies from about 10% of the dry weight in cereals to 20-40% of the dry weight of legumes. Much of the protein-bound amino acids is contained in the seed storage proteins which are synthesized during seed development and which serve as a major nutrient reserve following germination. In many seeds the storage proteins account for 50% or more of the total protein.

To improve the amino acid composition of seeds genetic engineering technology is being used to isolate, and express genes for storage proteins in transgenic plants. For example, a gene from Brazil nut for a seed 2S albumin composed of 26% sulfur-containing amino acids has been isolated [Altenbach et al. (1987) Plant Mol. Biol. 8:239-250] and expressed in the seeds of transformed tobacco under the control of the regulatory sequences from a bean phaseolin storage protein gene. The accumulation of the sulfur-rich protein in the tobacco seeds resulted in an up to 30% increase in the level of methionine in the seeds [Altenbach et al. (1989) Plant Mol. Biol. 13:513-522]. However, no plant seed storage proteins similarly enriched in lysine relative to average lysine content of plant proteins have been identified to date, preventing this approach from being used to increase lysine.

An alternative approach is to increase the production and accumulation of lysine via genetic engineering technology. Lysine, along with threonine, methionine and isoleucine, are amino acids derived from aspartate, and regulation of the biosynthesis of each member of this family is complex, interconnected, and not well understood, especially in plants. Regulation of the metabolic flow in the pathway appears to be primarily via end products in plants. The aspartate family pathway is also regulated at the branch-point reactions. For lysine this is the condensation of aspartyl β-semialdehyde with pyruvate catalyzed by dihydrodipicolinic acid synthase (DHDPS).

The E. coli dapA gene encodes a DHDPS enzyme that is about 20-fold less sensitive to inhibition by lysine than than a typical plant DHDPS enzyme, e.g., wheat germ DHDPS. The E. coli dapA gene has been linked to the 35S promoter of Cauliflower Mosaic Virus and a plant chloroplast transit sequence. The chimeric gene was introduced into tobacco cells via transformation and shown to cause a substantial increase in free lysine levels in leaves [Glassman et al. (1989) PCT Patent Appl. PCT/US89/01309, Shaul et al. (1992) Plant Jour. 2:203-209, Galili et al. (1992). EPO Patent Appl. 91119328.2, Falco, PCT/US93/02480 (International Publication Number WO 93/19190). However, the lysine content of the seeds was not increased in any of the transformed plants described in these studies. The same chimeric gene was also introduced into potato cells and lead to small increases in free lysine in leaves, roots and tubers of regenerated plants [Galili et al. (1992) EPO Patent Appl. 91119328.2, Perl et al. (1992) Plant Mol. Biol. 19:815-823].

Falco, PCT/US93/02480 (International Publication Number WO 93/19190, linked the E. coli dapA gene to the bean phaseolin promoter and a plant chloroplast transit sequence to increase expression in seeds, but still observed no increase in the lysine level in seeds. As noted above, the first step in the lysine biosynthetic pathway is catalyzed by aspartokinase (AK), and this enzyme has been found to be an important target for regulation in many organisms. Falco isolated a mutant of the E. coli lysC gene, which encoded a lysine-feedback-insensitive AK, and linked it to the bean phaseolin promoter and a plant chloroplast transit sequence. Expression of this chimeric gene in the seeds of transformed tobacco lead to a substantial increase in the level of threonine, but not lysine. Galili et al. (1992) EPO Patent Appl. 91119328.2 suggest that transforming plants with chimeric genes linking seed-specific promoters to a plant chloroplast transit sequence/E. coli dapA gene and plant chloroplast transit sequence/mutant E. coli lysC gene will lead to increased lysine levels in seeds. Falco, PCT/US93/02480 (International Publication Number WO 93/19190) carried out this experiment by transforming tobacco with a construct containing both the chimeric genes, bean phaseolin promoter/plant chloroplast transit sequence/E. coli dapA gene and bean phaseolin promoter/plant chloroplast transit sequence/mutant E. coli lysC gene. Simultaneous expression of both genes had no significant effect on the lysine content of the seeds. However, it was noted that a breakdown product of lysine, α-amino adipic acid, built up in the seeds. This suggested that the accumulation of free lysine in seeds was prevented because of lysine catabolism. In an effort to increase the rate of biosynthesis of lysine, Falco, PCT/US93/02480 (International Publication Number WO 93/19190, isolated the Corynebacterium glutamicum dapA gene which encodes a completely lysine insensitive DHDPS enzyme. Falco transformed tobacco with a construct containing the chimeric gene, bean phaseolin promoter/plant chloroplast transit sequence/Corynebacterium glutamicum dapA gene linked to bean phaseolin promoter/plant chloroplast transit sequence/mutant E. coli lysC gene. Simultaneous expression of both these lysine-insensitve enzymes still had no significant effect on the lysine content of the seeds.

Thus, it is clear that the limited understanding of the details of the regulation of the lysine biosynthetic pathway in plants, particularly in seeds, makes the application of genetic engineering technology to increase lysine content uncertain. It is not known, for most plants, whether lysine is synthesized in seeds or transported to the seeds from leaves. In addition, little is known about storage or catabolism of lysine in seeds. Because free amino acids make up only a small fraction of the total amino acid content of seeds, over-accumulation must be many-fold in order to significantly affect the total amino acid composition of the seeds. In addition, the effects of over-accumulation of a free amino acid such as lysine on seed development and viability is not known.

No method to increase the lysine content of seeds via genetic engineering and no examples of seeds having increased lysine levels obtained via genetic engineering were known before the invention described herein.

### SUMMARY OF THE INVENTION

This invention concerns a novel chimeric gene, and plants transformed using said novel gene, wherein a nucleic acid fragment encoding dihydrodipicolinic acid synthase, which is insensitive to inhibition by lysine, is operably linked to a monocot plant chloroplast transit sequence and to a monocot plant seed-specific regulatory sequence. In a preferred embodiment, the nucleic acid fragment encoding dihydrodipicolinic acid synthase comprises the nucleotide sequence shown in SEQ ID NO:3: encoding dihydrodipicolinic acid synthase from Corynebacterium glutamicum. In especially preferred embodiments, the plant chloroplast transit sequence is derived from a gene encoding the small subunit of ribulose 1,5-bisphosphate carboxylase, and the seed-specific regulatory sequence is from the gene encoding the β subunit of the seed storage protein phaseolin from the bean Phaseolus vulgaris, the Kunitz trypsin inhibitor 3 gene of Glycine max_{f} or a monocot embryo-specific promoter, preferably from the globulin 1 gene from Zea maize.

The genes described may be used, for example, for transforming plants, preferably corn plants. Also claimed are seeds obtained from the transformed plants. The invention can produce transformed plants wherein the seeds of the plants accumulate lysine to a level at least ten percent higher than in seeds of untransformed plants, preferably ten to four hundred percent higher than in untransformed plants.

The invention further concerns a method for obtaining a plant, preferably a corn, rapeseed or soybean plant wherein the seeds of the plants accumulate lysine to a level from ten percent to four hundred percent higher than seeds of untransformed plants comprising:
(a) transforming plant cells, preferably corn, rapeseed or soybean cells, with a chimeric gene comprising a nucleic acid fragment encoding dihydrodipicolinic acid synthase which is insensitive to inhibition by lysine operably linked to a plant chloroplast transit sequence and to a plant seed specific regulatory sequence;
(b) regenerating fertile mature plants from the transformed plant cells obtained from step (a) under conditions suitable to obtain seeds;
(c) screening the progeny seed of step (b) for lysine content; and
(d) selecting those lines whose seeds contain increased levels of lysine. Transformed plants obtained from this method are also claimed.

The invention additionally concerns a nucleic acid fragment comprising
(a) a first chimeric gene wherein a nucleic acid fragment encoding dihydrodipicolinic acid synthase which is insensitive to inhibition by lysine is operably linked to a plant chloroplast transit sequence and to a plant seed-specific regulatory sequence and
(b) a second chimeric gene wherein a nucleic acid fragment encoding a lysine-rich protein, wherein the weight percent lysine is at least 15%, is operably linked to a plant seed-specific regulatory sequence.

Also described is a nucleic acid fragment comprising
(a) a first chimeric gene wherein a nucleic acid fragment encoding dihydrodipicolinic acid synthase which is insensitive to inhibition by lysine is operably linked to a plant chloroplast transit sequence and to a plant seed-specific regulatory sequence; and
(b) a second chimeric gene wherein a nucleic acid fragment encoding a lysine-rich protein comprises a nucleic acid sequence encoding a protein comprising n heptad units (d e f g a b c), each heptad being either the same or different, wherein:
   n is at least 4;
   a and d are independently selected from the group consisting of Met, Leu, Val, Ile and Thr;
   e and g are independently selected from the group consisting of the acid/base pairs Glu/Lys, Lys/Glu, Arg/Glu, Arg/Asp, Lys/Asp, Glu/Arg, Asp/Arg and Asp/Lys; and
   b, c and f are independently any amino acids except Gly or Pro and at least two amino acids of b, c and f in each heptad are selected from the group consisting of Glu, Lys, Asp, Arg, His, Thr, Ser, Asn, Ala, Gln and Cys,
said nucleic acid fragment is operably linked to a plant seed-specific regulatory sequence.

Further described herein is a nucleic acid fragment comprising
(a) a first chimeric gene, wherein a nucleic acid fragment encoding dihydrodipicolinic acid synthase which is insensitive to inhibition by lysine is operably linked to a plant chloroplast transit sequence and to a plant seed-specific regulatory sequence; and
(b) a second chimeric gene wherein a nucleic acid fragment encoding a lysine/rich protein comprises a nucleic acid sequence encoding a protein having the amino acid sequence (MEEKLKA)₆(MEEKMKA)₂ is operably linked to a plant seed-specific regulatory sequence.

Also claimed herein are plants containing various embodiments of the described first chimeric genes and second chimeric genes and the described nucleic acid fragments and seeds obtained from such plants.

The invention further concerns a nucleic acid fragment comprising
(a) a first chimeric gene wherein a nucleic acid fragment encoding dihydrodipicolinic acid synthase which is insensitive to inhibition by lysine is operably linked to a plant chloroplast transit sequence and to a plant seed-specific regulatory sequence; and
(b) a second chimeric gene wherein a nucleic acid fragment encoding a lysine ketoglutarate reductase is operably linked in the sense or antisense orientation to a plant seed-specific regulatory sequence. Also claimed is a plant comprising in its genome that nucleic acid fragment and a seed obtained from such plant.

### BRIEF DESCRIPTION OF THE DRAWINGS AND SEQUENCE DESCRIPTIONS

The invention can be more fully understood from the following detailed description and the accompanying drawings and the sequence descriptions which form a part of this application.

Figure 1 shows an alpha helix from the side and top views.

Figure 2 shows end (Figure 2a) and side (Figure 2b) views of an alpha helical coiled-coil structure.

Figure 3 shows the chemical structure of leucine and methionine emphasizing their similar shapes.

Figure 4 shows a schematic representation of a seed-specific gene expression cassette.

Figure 5A shows a map of the binary plasmid vector pZS199, Figure 5B shows a map of the binary plasmid vector pFS926.

Figure 6A shows a map of the plasmid vector pBT603; Figure 6B shows a map of the plasmid vector pBT614.

Figure 7 depicts the strategy for creating a vector (pSK5) for use in construction and expression of the SSP gene sequences.

Figure 8 shows the strategy for inserting oligonucleotide sequences into the unique Ear I site of the base gene sequence.

Figure 9 shows the insertion of the base gene oligonucleotides into the Nco I/EcoR I sites of pSK5 to create the plasmid pSK6. This base gene sequence was used as in Figure 8 to insert the various SSP coding regions at the unique Ear I site to create the cloned segments listed.

Figure 10 shows the insertion of the 63 bp "segment" oligonucleotides used to create non-repetitive gene sequences for use in the duplication scheme in Figure 11.

Figure 11 (A and B) shows the strategy for multiplying non-repetitive gene "segments" utilizing in-frame fusions.

Figure 12 shows the vectors containing seed specific promoter and 3' sequence cassettes. SSP sequences were inserted into these vectors using the Nco I and Asp718 sites.

Figure 13 shows a map of the binary plasmid vector pZS97.

Figure 14 shows a map of the plasmid vector pML63.

Figure 15 shows a map of the plasmid vector pML102 carrying a chimeric gene wherein seed specific regulatory sequences (from the soybean Kunitz trypsin inhibitor 3 gene) are linked to a chloroplast transit sequence (from the small subunit of soybean ribulose bis-phosphate carboxylase) and the coding sequence for lysine-insensitive dihydrodipicolinic acid synthase (the dapA gene from Corynebacterium glutamicum).

SEQ ID NOS:1 and 2 were used in Example 1 as PCR primers for the isolation of the Corynebacterium dapA gene.

SEQ ID NO:3 shows the nucleotide and amino acid sequence of the coding region of the wild type Corynebacterium dapA gene, which encodes lysine-insensitive DHDPS, described in Example 1.

SEQ ID NO:4 shows an oligonucleotide used in Example 2 to create an Nco I site at the translation start codon of the E. coli dapA gene.

SEQ ID NO:5 shows the nucleotide and amino acid sequence of the coding region of the wild type E. coli lysC gene, which encodes AKIII, described in Example 3.

SEQ ID NOS:6 and 7 were used in Example 3 to create an Nco I site at the translation start codon of the E. coli lysC gene.

SEQ ID NOS:8, 9, 10 and 11 were used in Example 4 to create a chloroplast transit sequence and link the sequence to the E. coli lysC-M4, E. coli dapA and Corynebacteria dapA genes.

SEQ ID NOS:12 and 13 were used in Example 4 to create a Kpn I site immediately following the translation stop codon of the E. coli dapA gene.

SEQ ID NOS:14 and 15 were used in Example 4 as PCR primers to create a soybean chloroplast transit sequence and link the sequence to the Corynebacterium dapA gene.

SEQ ID NOS:16-92 represent nucleic acid fragments and the polypeptides they encode that are used to create chimeric genes for lysine-rich synthetic seed storage proteins suitable for expression in the seeds of plants.

SEQ ID NOS:93-98 were used in Example 12 to create a corn chloroplast transit sequence.

SEQ ID NOS:99 and 100 were used in Example 12 as PCR primers to create a corn chloroplast transit sequence and link the sequence to the E. coli dapA gene.

The Sequence Descriptions contain the one letter code for nucleotide sequence characters and the three letter codes for amino acids as defined in conformity with the IUPAC-IYUB standards described in Nucleic Acids Research 13:3021-3030(1985) and in the Biochemical Journal 219 (No. 2):345-373(1984), which are incorporated by reference herein.

### DETAILED DESCRIPTION OF THE INVENTION

The teachings below describe nucleic acid fragments and procedures useful for increasing the accumulation of lysine in the seeds of transformed plants, as compared to levels of lysine in untransformed plants. In order to increase the accumulation of free lysine in the seeds of plants via genetic engineering, a determination was made of which enyzmes in this pathway controlled the pathway in the seeds of plants. In order to accomplish this, genes encoding enzymes in the pathway were isolated from bacteria. Intracellular localization sequences and suitable regulatory sequences for expression in the seeds of plants were linked to create chimeric genes. The chimeric genes were then introduced into plants via transformation and assessed for their ability to elicit accumulation of the lysine in seeds. Expression of lysine-insensitive dihydrodipicolinic acid synthase (DHDPS), under control of a strong seed-specific promoter, is shown to increase free lysine levels 10 to 100 fold in corn, rapeseed and soybean seeds.

It has been discovered that the full potential for accumulation of excess free lysine in seeds is reduced by lysine catabolism. Provided herein are two alternative routes to prevent the loss of excess lysine due to catabolism. In the first approach, lysine catabolism is prevented through reduction in the activity of the enzyme lysine ketoglutarate reductase (LKR), which catalyzes the first step in lysine breakdown. A procedure to isolate plant LKR genes is provided. Chimeric genes for expression of antisense LKR RNA or for cosuppression of LKR in the seeds of plants are created. The chimeric gene is then linked to the chimeric DHDPS gene and both are introduced into plants via transformation simultaneously, or the genes are brought together by crossing plants transformed independently with each of the chimeric genes.

In the second approach, excess free lysine is incorporated into a form that is insensitive to breakdown, e.g., by incorporating it into a di-, tri- or oligopeptide, or a lysine-rich storage protein. The design of polypeptides which can be expressed in vivo to serve as lysine-rich seed storage proteins is provided. Genes encoding the lysine-rich synthetic storage proteins (SSP) are synthesized and chimeric genes wherein the SSP genes are linked to suitable regulatory sequences for expression in the seeds of plants are created. The SSP chimeric gene is then linked to the chimeric DHDPS gene and both are introduced into plants via transformation simultaneously, or the genes are brought together by crossing plants transformed independently with each of the chimeric genes.

A method for transforming plants, preferably corn, rapeseed and soybean plants is taught herein wherein the resulting seeds of the plants have at least ten percent, preferably ten percent to 400 percent greater lysine than the seeds of untransformed plants. Provided as examples herein are transformed rapeseed plants with seed lysine levels increased by 100% over untransformed plants, soybean plants with seed lysine levels increased by 400% over untransformed plants, and transformed corn plants with seed lysine levels increased by 130% over untransformed plants.

In the context of this disclosure, a number of terms are utilized. As used herein; the term "nucleic acid" refers to a large molecule which can be single-stranded or double-stranded, composed of monomers (nucleotides) containing a sugar, phosphate and either a purine or pyrimidine. A "nucleic acid fragment" is a fraction of a given nucleic acid molecule. In higher plants, deoxyribonucleic acid (DNA) is the genetic material while ribonucleic acid (RNA) is involved in the transfer of the information in DNA into proteins. A "genome" is the entire body of genetic material contained in each cell of an organism. The term "nucleotide sequence" refers to a polymer of DNA or RNA which can be single- or double-stranded, optionally containing synthetic, non-natural or altered nucleotide bases capable of incorporation into DNA or RNA polymers.

"Gene" refers to a nucleic acid fragment that expresses a specific protein, including regulatory sequences preceding (5' non-coding) and following (3' non-coding) the coding region. "Native" gene refers to the gene as found in nature with its own regulatory sequences. "Chimeric" gene refers to a gene comprising heterogeneous regulatory and coding sequences. "Endogenous" gene refers to the native gene normally found in its natural location in the genome. A "foreign" gene refers to a gene not normally found in the host organism but that is introduced by gene transfer.

"Coding sequence" refers to a DNA sequence that codes for a specific protein and excludes the non-coding sequences.

"Initiation codon" and "termination codon" refer to a unit of three adjacent nucleotides in a coding sequence that specifies initiation and chain termination, respectively, of protein synthesis (mRNA translation). "Open reading frame" refers to the amino acid sequence encoded between translation initiation and termination codons of a coding sequence.

As used herein, suitable "regulatory sequences" refer to nucleotide sequences located upstream (5'), within, and/or downstream (3') to a coding sequence, which control the transcription and/or expression of the coding sequences, potentially in conjunction with the protein biosynthetic apparatus of the cell. These regulatory sequences include promoters, translation leader sequences, transcription termination sequences, and polyadenylation sequences.

"Promoter" refers to a DNA sequence in a gene, usually upstream (5') to its coding sequence, which controls the expression of the coding sequence by providing the recognition for RNA polymerase and other factors required for proper transcription. A promoter may also contain DNA sequences that are involved in the binding of protein factors which control the effectiveness of transcription initiation in response to physiological or developmental conditions. It may also contain enhancer elements.

An "enhancer" is a DNA sequence which can stimulate promoter activity. It may be an innate element of the promoter or a heterologous element inserted to enhance the level and/or tissue-specificity of a promoter. "Constitutive promoters" refers to those that direct gene expression in all tissues and at all times.
"Organ-specific" or "development-specific" promoters as referred to herein are those that direct gene expression almost exclusively in specific organs, such as leaves or seeds, or at specific development stages in an organ, such as in early or late embryogenesis, respectively.

The term "operably linked" refers to nucleic acid sequences on a single nucleic acid molecule which are associated so that the function of one is affected by the other. For example, a promoter is operably linked with a structure gene when it is capable of affecting the expression of that structural gene (i.e., that the structural gene is under the transcriptional control of the promoter).

The term "expression", as used herein, is intended to mean the production of the protein product encoded by a gene. More particularly, "expression" refers to the transcription and stable accumulation of the sense (mRNA) or tha antisense RNA derived from the nucleic acid fragment(s) of the invention that, in conjuction with the protein apparatus of the cell, results in altered levels of protein product. "Antisense inhibition" refers to the production of antisense RNA transcripts capable of preventing the expression of the target protein. "Overexpression" refers to the production of a gene product in transgenic organisms that exceeds levels of production in normal or non-transformed organisms. "Cosuppression" refers to the expression of a foreign gene which has substantial homology to an endogenous gene resulting in the suppression of expression of both the foreign and the endogenous gene. "Altered levels" refers to the production of gene product(s) in transgenic organisms in amounts or proportions that differ from that of normal or non-transformed organisms.

The "3' non-coding sequences" refers to the DNA sequence portion of a gene that contains a polyadenylation signal and any other regulatory signal capable of affecting mRNA processing or gene expression. The polyadenylation signal is usually characterized by affecting the addition of polyadenylic acid tracts to the 3' end of the mRNA precursor.

The "translation leader sequence" refers to that DNA sequence portion of a gene between the promoter and coding sequence that is transcribed into RNA and is present in the fully processed mRNA upstream (5') of the translation start codon. The translation leader sequence may affect processing of the primary transcript to mRNA, mRNA stability or translation efficiency.

"Mature" protein refers to a post-translationally processed polypeptide without its targeting signal. "Precursor" protein refers to the primary product of translation of mRNA. A "chloroplast targeting signal" is an amino acid sequence which is translated in conjunction with a protein and directs it to the chloroplast. "Chloroplast transit sequence" refers to a nucleotide sequence that encodes a chloroplast targeting signal.

"Transformation" herein refers to the transfer of a foreign gene into the genome of a host organism and its genetically stable inheritance. Examples of methods of plant transformation include Agrobacterium-mediated transformation and particle-accelerated or "gene gun" transformation technology.

"Amino acids" herein refer to the naturally occuring L amino acids (Alanine, Arginine, Aspartic acid, Asparagine, Cystine, Glutamic acid, Glutamine, Glycine, Histidine, Isoleucine, Leucine, Lysine, Methionine, Proline, Phenylalanine, Serine, Threonine, Tryptophan, Tyrosine, and Valine). "Essential amino acids" are those amino acids which cannot be synthesized by animals. A "polypeptide" or "protein" as used herein refers to a molecule composed of monomers (amino acids) linearly linked by amide bonds (also known as peptide bonds).

"Synthetic protein" herein refers to a protein consisting of amino acid sequences that are not known to occur in nature. The amino acid sequence may be derived from a consensus of naturally occuring proteins or may be entirely novel.

"Primary sequence" refers to the connectivity order of amino acids in a polypeptide chain without regard to the conformation of the molecule. Primary sequences are written from the amino terminus to the carboxy terminus of the polypeptide chain by convention.

"Secondary structure" herein refers to physicochemically favored regular backbone arrangements of a polypeptide chain without regard to variations in side chain identities or conformations. "Alpha helices" as used herein refer to right-handed helices with approximately 3.6 residues residues per turn of the helix. An "amphipathic helix" refers herein to a polypeptide in a helical conformation where one side of the helix is predominantly hydrophobic and the other side is predominantly hydrophilic.

"Coiled-coil" herein refers to an aggregate of two parallel right-handed alpha helices which are wound around each other to form a left-handed superhelix.

"Salt bridges" as discussed here refer to acid-base pairs of charged amino acid side chains so arranged in space that an attractive electrostatic interaction is maintained between two parts of a polypeptide chain or between one chain and another.

"Host cell" means the cell that is transformed with the introduced genetic material.

### Isolation of DHDPS genes

The E. coli dapA gene (ecodapA) was obtained as a bacteriophage lambda clone from an ordered library of 3400 overlapping segments of E. coli DNA constructed by Kohara, Akiyame and Isono [Kohara et al. (1987) Cell 50:595-508]. Details of the isolation and modification of ecodapA are presented in Example 1. The ecodapA gene encodes a DHDPS enzyme that is at least 20-fold less sensitive to inhibition by lysine than a typical plant enzyme, e.g., wheat DHDPS. For purposes of the present invention, 20-fold less sensitive to inhibition by lysine is termed lysine-insensitive.

The Corynebacterium dapA gene (cordapA) was isolated from genomic DNA from ATCC strain 13032 using polymerase chain reaction (PCR). The nucleotide sequence of the Corynebacterium dapA gene has been published [Bonnassie et al. (1990) Nucleic Acids Res. 18:6421]. From the sequence it was possible to design oligonucleotide primers for polymerase chain reaction (PCR) that would allow amplification of a DNA fragment containing the gene, and at the same time add unique restriction endonuclease sites at the start codon and just past the stop codon of the gene to facilitate further constructions involving the gene. The details of the isolation of the Corynebacterium dapA (cordapA) gene are presented in Example 1. The cordapA gene encodes a preferred lysine-insensitive DHDPS enzyme that is unaffected by the presence of 70mM lysine in the enzyme reaction mix.

The isolation of other genes encoding DHDPS has been described in the literature. A cDNA encoding DHDPS from wheat [Kaneko et al. (1990) J. Biol. Chem. 265:17451-17455], and a cDNA encoding DHDPS from corn [Frisch et al. (1991) Mol. Gen. Genet. 228:287-293] are two examples of plant DHDPS genes that have been isolated and sequenced. The plant genes encode wild type lysine-sensitive DHDPS enzymes. However, Negrutui et al. [(1984) Theor. Appl. Genet. 68:11-20], obtained two AEC-resistant tobacco mutants in which DHDPS activity was less sensitive to lysine inhibition than the wild type enzyme. This indicates that these tobacco mutants contain DHDPS genes encoding lysine-resistant enzyme. These genes could be readily isolated from the tobacco mutants using the methods already described for isolating the wheat or corn genes or, alternatively, by using the wheat or corn genes as heterologous hybridization probes.

Still other genes encoding DHDPS can be isolated by using either the E. coli dapA gene, the cordapA gene, or either of the plant DHDPS genes as DNA hybridization probes. Alternatively, other genes encoding DHDPS could be isolated by functional complementation of an E. coli dapA mutant, as was done to isolate the cordapA gene [Yeh et al. (1988) Mol. Gen. Genet. 212:105-111] and the corn DHDPS gene.

### Construction of Chimeric Genes for Expression of dapA Coding Region in Plants

The expression of foreign genes in plants is well-established [De Blaere et al. (1987) Meth. Enzymol. 143:277-291]. Proper level of expression of dapA mRNA may require the use of different chimeric genes utilizing different promoters. Such chimeric genes can be transferred into host plants either together in a single expression vector or sequentially using more than one vector. A preferred class of heterologous hosts for the expression of the coding sequence of the dapA genes are eukaryotic hosts, particularly the cells of higher plants. Particularly preferred among the higher plants and the seeds derived from them are rapeseed (Brassica napus, B. campestris) and soybean (Glycine max).

The origin of promoter chosen to drive the expression of the coding sequence is not critical as long as it has sufficient transcriptional activity to accomplish the invention by expressing translatable mRNA for dapA genes in the desired host tissue. Preferred promoters are those that allow expression of the protein specifically in seeds. This may be especially useful, since seeds are the primary source of vegetable amino acids and also since seed-specific expression will avoid any potential deleterious effect in non-seed organs. Examples of seed-specific promoters include, but are not limited to, the promoters of seed storage proteins. The seed storage proteins are strictly regulated, being expressed almost exclusively in seeds in a highly organ-specific and stage-specific manner [Higgins et al.(1984) Ann. Rev. Plant Physiol. 35:191-221; Goldberg et al. (1989) Cell 56:149-160; Thompson et al. (1989) BioEssays 10:108-113]. Moreover, different seed storage proteins may be expressed at different stages of seed development.

There are currently numerous examples for seed-specific expression of seed storage protein genes in transgenic dicotyledonous plants. These include genes from dicotyledonous plants for bean β-phaseolin [Sengupta-Goplalan et al. (1985) Proc. Natl. Acad. Sci. USA 82:3320-3324; Hoffman et al. (1988) Plant Mol. Biol. 11:717-729], bean lectin [Voelker et al. (1987) EMBO J. 6: 3571-3577], soybean lectin [Okamuro et al. (1986) Proc. Natl. Acad. Sci. USA 83:8240-8244], soybean kunitz trypsin inhibitor [Perez-Grau et al. (1989) Plant Cell 1:095-1109], soybean β-conglycinin [Beachy et al. (1985) EMBO J. 4:3047-3053; Barker et al. (1988) Proc. Natl. Acad. Sci. USA 85:458-462; Chen et al. (1988) EMBO J. 7:297-302; Chen et al. (1989) Dev. Genet. 10:112-122; Naito et al. (1988) Plant Mol. Biol. 11:109-123], pea vicilin [Higgins et al. (1988) Plant Mol. Biol. 11:683-695], pea convicilin [Newbigin et al. (1990) Planta 180:461], pea legumin [Shirsat et al. (1989) Mol. Gen. Genetics 215:326]; rapeseed napin [Radke et al. (1988) Theor. Appl. Genet. 75:685-694] as well as genes from monocotyledonous plants such as for maize 15 kD zein [Hoffman et al. (1987) EMBO J. 6:3213-3221; Schernthaner et al. (1988) EMBO J. 7:1249-1253; Williamson et al. (1988) Plant Physiol. 88:1002-1007], barley β-hordein [Marris et al. (1988) Plant Mol. Biol. 10:359-366] and wheat glutenin [Colot et al. (1987) EMBO J. 6:3559-3564]. Moreover, promoters of seed-specific genes, operably linked to heterologous coding sequences in chimeric gene constructs, also maintain their temporal and spatial expression pattern in transgenic plants. Such examples include Arabidopsis thaliana 2S seed storage protein gene promoter to express enkephalin peptides in Arabidopsis and B. napus seeds [Vandekerckhove et al. (1989) Bio/Technology 7:929-932], bean lectin and bean β-phaseolin promoters to express luciferase [Riggs et al. (1989) Plant Sci. 63:47-57], and wheat glutenin promoters to express chloramphenicol acetyl transferase [Colot et al. (1987) EMBO J. 6:3559-3564].

Of particular use in the expression of the nucleic acid fragment of the invention will be the promoters from several extensively-characterized seed storage protein genes such as those for bean β-phaseolin [Sengupta-Goplalan et al. (1985) Proc. Natl. Acad. Sci. USA 82:3320-3324; Hoffman et al. (1988) Plant Mol. Biol. 11:717-729], soybean Kunitz trypsin inhibitor [Jofuku et al. (1989) Plant Cell 1:1079-1093; Perez-Grau et al. (1989) Plant Cell 1:1095-1109], soybean β-conglycinin [Harada et al. (1989) Plant Cell 1:415-425], and rapeseed napin [Radke et al. (1988) Theor. Appl. Genet. 75:685-694]. Promoters of genes for bean β-phaseolin and soybean β-conglycinin storage protein will be particularly useful in expressing the dapA mRNA in the cotyledons at mid- to late-stages of seed development.

Also of particular use in the expression of the nucleic acid fragments of the invention will be the heterologous promoters from several extensively characterized corn seed storage protein genes such as endosperm-specific promoters from the 10 kD zein [Kirihara et al. (1988) Gene 71:359-370], the 27 kD zein [Prat et al. (1987) Gene 52:51-49; Gallardo et al. (1988) Plant Sci. 54:211-281; Reina et al. (1990) Nucleic Acids Res. 18:6426-6426], and the 19 kD zein [Marks et al. (1985) J. Biol. Chem. 260:16451-16459]. The relative transcriptional activities of these promoters in corn have been reported [Kodrzyck et al. (1989) Plant Cell 1:105-114] providing a basis for choosing a promoter for use in chimeric gene constructs for corn. For expression in corn embryos, the strong embryo-specific promoter from the globulin 1 (GLB1) gene [Kriz (1989) Biochemical Genetics 27:239-251, Wallace et al. (1991) Plant Physiol. 95:973-975] can be used.

It is envisioned that the introduction of enhancers or enhancer-like elements into other promoter constructs will also provide increased levels of primary transcription for dapA genes to accomplish the invention. These would include viral enhancers such as that found in the 35S promoter [Odell et al. (1988) Plant Mol. Biol. 10:263-272], enhancers from the opine genes [Fromm et al. (1989) Plant Cell 1:977-984], or enhancers from any other source that result in increased transcription when placed into a promoter operably linked to the nucleic acid fragment of the invention.

Of particular importance is the DNA sequence element isolated from the gene for the α'-subunit of β-conglycinin that can confer 40-fold seed-specific enhancement to a constitutive promoter [Chen et al. (1988) EMBO J. 7:297-302; Chen et al. (1989) Dev. Genet. 10:112-122]. One skilled in the art can readily isolate this element and insert it within the promoter region of any gene in order to obtain seed-specific enhanced expression with the promoter in transgenic plants. Insertion of such an element in any seed-specific gene that is expressed at different times than the β-conglycinin gene will result in expression in transgenic plants for a longer period during seed development.

Any 3' non-coding region capable of providing a polyadenylation signal and other regulatory sequences that may be required for the proper expression of the dapA coding regions can be used to accomplish the invention. This would include the 3' end from any storage protein such as the 3' end of the bean phaseolin gene, the 3' end of the soybean β-conglycinin gene, the 3' end from viral genes such as the 3' end of the 35S or the 19S cauliflower mosaic virus transcripts, the 3' end from the opine synthesis genes, the 3' ends of ribulose 1,5-bisphosphate carboxylase or chlorophyll a/b binding protein, or 3' end sequences from any source such that the sequence employed provides the necessary regulatory information within its nucleic acid sequence to result in the proper expression of the promoter/coding region combination to which it is operably linked. There are numerous examples in the art that teach the usefulness of different 3' non-coding regions [for example, see Ingelbrecht et al. (1989) Plant Cell 1:671-680].

DNA sequences coding for intracellular localization sequences may be added to the dapA coding sequence if required for the proper expression of the proteins to accomplish the invention. Plant amino acid biosynthetic enzymes are known to be localized in the chloroplasts and therefore are synthesized with a chloroplast targeting signal. Bacterial proteins such as Corynebacterium DHDPS have no such signal. A chloroplast transit sequence could, therefore, be fused to the dapA coding sequence. Preferred chloroplast transit sequences are those of the small subunit of ribulose 1,5-bisphosphate carboxylase, e.g. from soybean [Berry-Lowe et al. (1982) J. Mol. Appl. Genet. 1:483-498] for use in dicotyledonous plants and from corn [Lebrun et al. (1987) Nucleic Acids Res. 15:4360] for use in monocotyledonous plants.

### Introduction of dapA Chimeric Genes into Plants

Various methods of introducing a DNA sequence (i.e., of transforming) into eukaryotic cells of higher plants are available (see EPO publications 0 295 959 A2 and 0 138 341 A1). Such methods include those based on transformation vectors based on the Ti and Ri plasmids of Agrobacterium spp. It is particularly preferred to use the binary type of these vectors. Ti-derived vectors transform a wide variety of higher plants, including monocotyledonous and dicotyledonous plants, such as soybean, cotton and rape [Pacciotti et al. (1985) Bio/Technology 3:241; Byrne et al. (1987) Plant Cell, Tissue and Organ Culture 8:3; Sukhapinda et al. (1987) Plant Mol. Biol. 8:209-216; Lorz et al. (1985) Mol. Gen. Genet. 199:178; Potrykus (1985) Mol. Gen. Genet. 199:183].

For introduction into plants the chimeric genes of the invention can be inserted into binary vectors as described in Examples 6-12. The vectors are part of a binary Ti plasmid vector system [Bevan, (1984) Nucl. Acids. Res. 12:8711-87201 of Agrobacterium tumefaciens.

Other transformation methods are available to those skilled in the art, such as direct uptake of foreign DNA constructs [see EPO publication 0 295 959 A2], techniques of electroporation [see Fromm et al. (1986) Nature (London) 319:791] or high-velocity ballistic bombardment with metal particles coated with the nucleic acid constructs [see Kline et al. (1987) Nature (London) 327:70, and see U.S. Pat. No. 4,945,050]. Once transformed, the cells can be regenerated by those skilled in the art.

Of particular relevance are the recently described methods to transform foreign genes into commercially important crops, such as rapeseed [see De Block et al. (1989) Plant Physiol. 91:694-701], sunflower [Everett et al. (1987) Bio/Technology 5:1201], soybean [McCabe et al. (1988) Bio/Technology 6:923; Hinchee et al. (1988) Bio/Technology 6:915; Chee et al. (1989) Plant Physiol. 91:1212-1218; Christou et al. (1989) Proc. Natl. Acad. Sci USA 86:7500-7504; EPO Publication 0 301 749 A2], and corn [Gordon-Kamm et al. (1990) Plant Cell 2:603-618; Fromm et al. (1990) Biotechnology 8:833-839].

For introduction into plants by high-velocity ballistic bombardment, the chimeric genes of the invention can be inserted into suitable vectors as described in Example 6.

### Expression of dapA Chimeric Genes in Rapeseed, Soybean and Corn Plants

To analyze for expression of the chimeric dapA gene in seeds and for the consequences of expression on the amino acid content in the seeds, a seed meal can be prepared as described in Examples 5 or 6 or by any other suitable method. The seed meal can be partially or completely defatted, via hexane extraction for example, if desired. Protein extracts can be prepared from the meal and analyzed for DHDPS enzyme activity. Alternatively the presence of the DHDPS protein can be tested for immunologically by methods well-known to those skilled in the art. Nearly all of the transformants expressed the foreign DHDPS protein (see Examples 5, 6 and 13). To measure free amino acid composition of the seeds, free amino acids can be extracted from the meal and analyzed by methods known to those skilled in the art (see Examples 5 and 6 for suitable procedures).

Rapeseed transformants expressing DHDPS protein showed a greater than 100-fold increase in free lysine level in their seeds. There was a good correlation between transformants expressing higher levels of DHDPS protein and those having higher levels of free lysine. Among the transformants, there has been no greater accumulation of free lysine due to expression of a lysine insensitive AK enzyme along with a lysine-insensitive DHDPS compared to expression of a lysine-insensitive DHDPS alone. Thus, in rapeseed, expression of a lysine-insensitive DHDPS in seeds is necessary and sufficient to cause a large increase in free lysine. A high level of α-aminoadipic acid, indicative of lysine catabolism, was observed in all of the transformed lines with increased levels of free lysine.

To measure the total amino acid composition of mature rapeseed seeds, defatted meal was analyzed as described in Example 5. Relative amino acid levels in the seeds were compared as percentages of lysine to total amino acids. The highest expressing lines showed a nearly 2-fold increase in the lysine level in the seeds, so that lysine makes up about 12% of the total seed amino acids.

Twenty-one of twenty-three soybean transformants expressed the DHDPS protein. Analysis of single seeds of these transformants showed excellent correlation between expression of the GUS transformation marker gene and DHDPS in individual seeds. Therefore, the GUS and DHDPS genes are integrated at the same site in the soybean genome.

There was excellent correlation between transformants expressing Corynebacteria DHDPS protein and those having higher levels of free lysine. From 20-fold to 120-fold increases in free lysine level was observed in seeds expressing Corynebacteria DHDPS.

Analyses of free lysine levels in individual seeds from transformants in which the transgenes segregated as a single locus revealed that the increase in free lysine level was significantly higher in about one-fourth of the seeds. Since one-fourth of the seeds are expected to be homozygous for the transgene, it is likely that the higher lysine seeds are the homozygotes. Furthermore, this indicates that the level of increase in free lysine is dependent upon the copy number of the DHDPS gene. Therefore, lysine levels could be further increased by making hybrids of two different transformants, and obtaining progeny that are homozygous at both transgene loci, thus increasing the copy number of the DHDPS gene from two to four.

A high level of saccharopine, indicative of lysine catabolism, was observed in seeds that contained high levels of lysine. Thus, prevention of lysine catabolism by inactivation of lysine ketoglutarate reductase should further increase the accumulation of free lysine in the seeds. Alternatively, incorporation of lysine into a peptide or lysine-rich protein would prevent catabolism and lead to an increase in the accumulation of lysine in the seeds.

Total lysine levels were significantly increased in seeds expressing Corynebacteria DHDPS protein. Seeds with a 10-260% increase in the lysine level compared to the untransformed control were observed. Expression of DHDPS along with a lysine-insensitive aspartokinase enzyme resulted in lysine increases of more than 400%. Thus, these seeds contain much more lysine than any previous soybean seed.

Expression of the Corynebacterium DHDPS protein, driven by either the corn globulin 1 promoter for expression in the embryo or the corn glutelin 2 promoter for expression in the endosperm, was observed in the corn seeds. Free lysine levels in the seeds increased from about 1.4% of free amino acids in control seeds to 15-27% of free amino acids in seeds expressing Corynebacterium DHDPS from the globulin 1 promoter. A smaller increase in free lysine was observed in in seeds expressing Corynebacterium DHDPS from the glutelin 2 promoter. Thus to increase lysine, it may be better to express this enzyme in the embryo rather than the endosperm. A high level of saccharopine, indicative of lysine catabolism, was observed in seeds that contained high levels of lysine. The increased accumulation of free lysine in seeds expressing Corynebacterium DHDPS from the globulin 1 promoter was sufficient to result in substantial increases (35%-130%) in the total lysine content of the seeds.

### Isolation of a Plant Lysine Ketoglutarate Reductase Gene

To accumulate higher levels of free lysine it may be desirable to prevent lysine catabolism. Evidence indicates that lysine is catabolized in plants via the saccharopine pathway. The first enzymatic evidence for the existence of this pathway was the detection of lysine ketoglutarate reductase (LKR) activity in immature endosperm of developing maize seeds [Arruda et al. (1982) Plant Physiol. 69:988-989]. LKR catalyzes the first step in lysine catabolism, the condensation of L-lysine with α-ketoglutarate into saccharopine using NADPH as a cofactor. LKR activity increases sharply from the onset of endosperm development in corn, reaches a peak level at about 20 days after pollination, and then declines [Arruda et al. (1983) Phytochemistry 22:2687-2689]. In order to prevent the catabolism of lysine it would be desirable to reduce or eliminate LKR expression or activity. This could be accomplished by cloning the LKR gene, preparing a chimeric gene for cosuppression of LKR or preparing a chimeric gene to express antisense RNA for LKR, and introducing the chimeric gene into plants via transformation.

Several methods to clone a plant LKR gene are available to one skilled in the art. The protein can be purified from corn endosperm, as described in Brochetto-Braga et al. [(1992) Plant Physiol. 98:1139-1147] and used to raise antibodies. The antibodies can then be used to screen an cDNA expression library for LKR clones. Alternatively the purified protein can be used to determine amino acid sequence at the amino-terminal of the protein or from protease derived internal peptide fragments. Degenerate oligonucleotide probes can be prepared based upon the amino acid sequence and used to screen a plant cDNA or genomic DNA library via hybridization. Another method makes use of an E. coli strain that is unable to grow in a synthetic medium containing 20 µg/mL of L-lysine. Expression of LKR full-length cDNA in this strain will reverse the growth inhibition by reducing the lysine concentration. Construction of a suitable E. coli strain and its use to select clones from a plant cDNA library that lead to lysine-resistant growth is described in Example 7.

In order to block expression of the LKR gene in transformed plants, a chimeric gene designed for cosuppression of LKR can be constructed by linking the LKR gene or gene fragment to any of the plant promoter sequences described above (U.S. Patent No. 5,231,020). Alternatively, a chimeric gene designed to express antisense RNA for all or part of the LKR gene can be constructed by linking the LKR gene or gene fragment in reverse orientation to any of the plant promoter sequences described above (Eur. Patent Applic. No. 84112647.7). Either the cosuppression or antisense chimeric gene could be introduced into plants via transformation. Transformants wherein expression of the endogenous LKR gene is reduced or eliminated are selected.

Preferred promoters for the chimeric genes would be seed-specific promoters. For soybean, rapeseed and other dicotyledonous plants, strong seed-specific promoters from a bean phaseolin gene, a soybean β-conglycinin gene, glycinin gene, Kunitz trypsin inhibitor gene, or rapeseed napin gene would be preferred. For corn and other monocotyledonous plants, a strong endosperm-specific promoter, e.g., the 10 kD or 27 kD zein promoter, would be preferred.

Transformed plants containing any of the chimeric LKR genes can be obtained by the methods described above. In order to obtain transformed plants that express a chimeric gene for cosuppression of LKR or antisense LKR, as well as a chimeric gene encoding lysine-insensitive DHDPS, the cosuppression or antisense LKR gene could be linked to the chimeric gene encoding lysine-insensitive DHDPS and the two genes could be introduced into plants via transformation. Alternatively, the chimeric gene for cosuppression of LKR or antisense LKR could be introduced into previously transformed plants that express lysine-insensitive DHDPS, or the cosuppression or antisense LKR gene could be introduced into normal plants and the transformants obtained could be crossed with plants that express lysine-insensitive DHDPS.

### Design of Lysine-Rich Polypeptides

It may be desirable to convert the high levels of lysine produced into a form that is insensitive to breakdown, e.g., by incorporating it into a di-, tri- or oligopeptide, or a lysine-rich storage protein. No natural lysine-rich proteins are known.

One aspect of this invention is the design of polypeptides which can be expressed in vivo to serve as lysine-rich seed storage proteins. Polypeptides are linear polymers of amino acids where the α-carboxyl group of one amino acid is covalently bound to the α-amino group of the next amino acid in the chain. Noncovalent interactions among the residues in the chain and with the surrounding solvent determine the final conformation of the molecule. Those skilled in the art must consider electrostatic forces, hydrogen bonds, Van der Waals forces, hydrophobic interactions, and conformational preferences of individual amino acid residues in the design of a stable folded polypeptide chain [see for example: Creighton, (1984) Proteins, Structures and Molecular Properties, W. H. Freeman and Company, New York, pp. 133-197, or Schulz et al., (1979) Principles of Protein Structure, Springer Verlag, New York, pp. 27-45]. The number of interactions and their complexity suggest that the design process may be aided by the use of natural protein models where possible.

The synthetic storage proteins (SSPs) embodied in this invention are chosen to be polypeptides with the potential to be enriched in lysine relative to average levels of proteins in plant seeds. Lysine is a charged amino acid at physiological pH and is therefore found most often on the surface of protein molecules [Chotia, (1976) Journal of Molecular Biology 105:1-14]. To maximize lysine content, Applicants chose a molecular shape with a high surface-to-volume ratio for the synthetic storage proteins embodied in this invention. The alternatives were either to stretch the common globular shape of most proteins to form a rod-like extended structure or to flatten the globular shape to a disk-like structure. Applicants chose the former configuration as there are several natural models for long rod-like proteins in the class of fibrous proteins [Creighton, (1984) Proteins, Structures and Molecular Properties, W.H. Freeman and Company, New York, p. 191].

Coiled-coils constitute a well-studied subset of the class of fibrous proteins [see Cohen et al., (1986) Trends Biochem. Sci. 11:295-298]. Natural examples are found in α-keratins, paramyosin, light meromyosin and tropomyosin. These protein molecules consist of two parallel alpha helices twisted about each other in a left-handed supercoil. The repeat distance of this supercoil is 140 Å (compared to a repeat distance of 5.4 Å for one turn of the individual helices). The supercoil causes a slight skew (10°) between the axes of the two individual alpha helices.

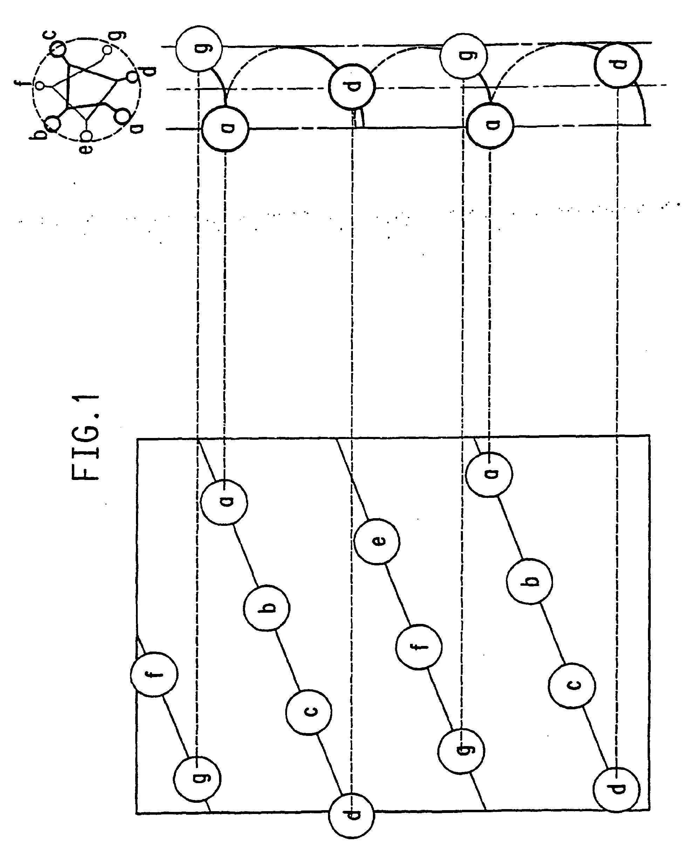

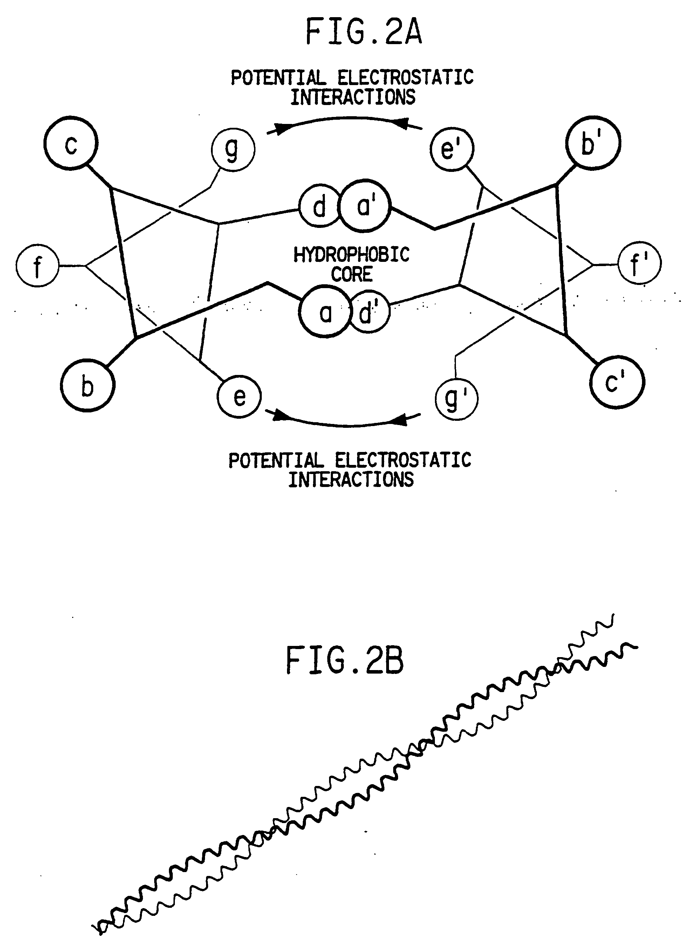

In a coiled coil there are 3.5 residues per turn of the individual helices resulting in an exact 7 residue periodicity with respect to the superhelix axis (see Figure 1). Every seventh amino acid in the polypeptide chain therefore occupies an equivalent position with respect to the helix axis. Applicants refer to the seven positions in this heptad unit of the invention as (d e f g a b c) as shown in Figures 1 and 2a. This conforms to the conventions used in the coiled-coil literature.

The a and d amino acids of the heptad follow a 4,3 repeat pattern in the primary sequence and fall on one side of an individual alpha helix (See Figure 1). If the amino acids on one side of an alpha helix are all non-polar, that face of the helix is hydrophobic and will associate with other hydrophobic surfaces as, for example, the non-polar face of another similar helix. A coiled-coil structure results when two helices dimerize such that their hydrophobic faces are aligned with each other (See Figure 2a).

The amino acids on the external faces of the component alpha helices (b, c, e, f, g) are usually polar in natural coiled-coils in accordance with the expected pattern of exposed and buried residue types in globular proteins [Schulz, et al., (1979) Principles of Protein Structure. Springer Verlag, New York, p. 12; Talbot, et al, (1982) Acc. Chem. Res. 15:224-230; Hodges et al., (1981) Journal of Biological Chemistry 256:1214-1224]. Charged amino acids ace sometimes found forming salt bridges between positions e and g' or positions g and e' on the opposing chain (see Figure 2a).

Thus, two amphipathic helices like the one shown in Figure 1 are held together by a combination of hydrophobic interactions between the a, a', d, and d' residues and by salt bridges between e and g' and/or g and e' residues. The packing of the hydrophobic residues in the supercoil maintains the chains "in register". For short polypeptides comprising only a few turns of the component alpha helical chains, the 10° skew between the helix axes can be ignored and the two chains treated as parallel (as shown in Figure 2a).

A number of synthetic coiled-coils have been reported in the literature (Lau et al., (1984) Journal of Biological Chemistry 259:13253-13261; Hodges et al., (1988) Peptide Research 1:19-30; DeGrado et al., (1989) Science 243:622-628; O'Neil et al., (1990) Science 250:646-651]. Although these polypeptides vary in size, Lau et al. found that 29 amino acids were sufficient for dimerization to form the coiled-coil structure [Lau et al., (1984) Journal of Biological Chemistry 259:13253-13261]. Applicants constructed the polypeptides in this invention as 28-residue and larger chains for reasons of conformational stability.

The polypeptides of this invention are designed to dimerize with a coiled-coil motif in aqueous environments. Applicants have used a combination of hydrophobic interactions and electrostatic interactions to stabilize the coiled-coil conformation. Most nonpolar residues are restricted to the a and d positions which creates a hydrophobic stripe parallel to the axis of the helix. This is the dimerization face. Applicants avoided large, bulky amino acids along this face to minimize steric interference with dimerization and to facilitate formation of the stable coiled-coil structure.

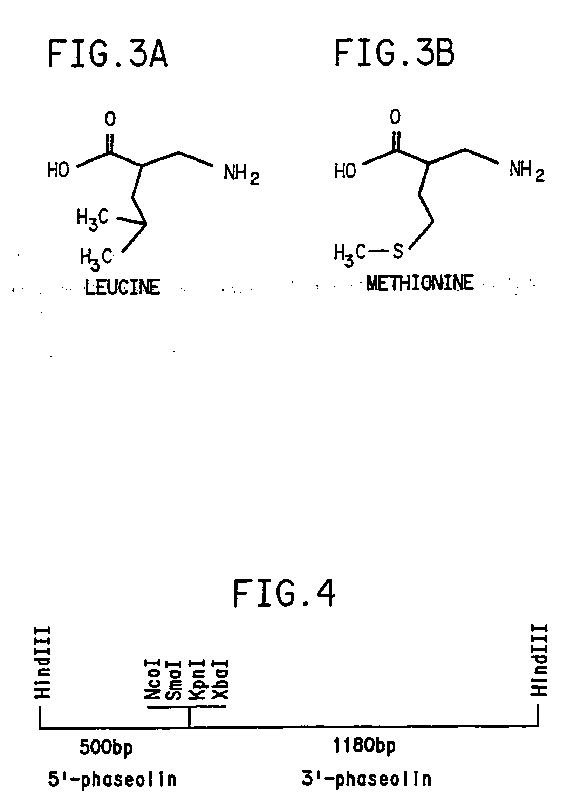

Despite recent reports in the literature suggesting that methionine at positions a and d is destabilizing to coiled-coils in the leucine zipper subgroup [Landschulz et al., (1989) Science 243:1681-1688 and Hu et al., (1990) Science 250:1400-1403], Applicants chose to substitute methionine residues for leucine on the hydrophobic face of the SSP polypeptides. Methionine and leucine are similar in molecular shape (Figure 3). Applicants demonstrated that any destabilization of the coiled-coil that may be caused by methionine in the hydrophobic core appears to be compensated in sequences where the formation of salt bridges (e-g' and g-e') occurs at all possible positions in the helix (i.e., twice per heptad).

To the extent that it is compatible with the goal of creating a polypeptide enriched in lysine, Applicants minimized the unbalanced charges in the polypeptide. This may help to prevent undesirable interactions between the synthetic storage proteins and other plant proteins when the polypeptides are expressed in vivo.

The polypeptides of this invention are designed to spontaneously fold into a defined, conformationally stable structure, the alpha helical coiled-coil, with minimal restrictions on the primary sequence. This allows synthetic storage proteins to be custom-tailored for specific end-user requirements. Any amino acid can be incorporated at a frequency of up to one in every seven residues using the b, c, and f positions in the heptad repeat unit. Applicants note that up to 43% of an essential amino acid from the group isoleucine, leucine, lysine, methionine, threonine, and valine can be incorporated and that up to 14% of the essential amino acids from the group phenylalanine, tryptophan, and tyrosine can be incorporated into the synthetic storage proteins of this invention.

In the SSPs only Met, Leu, Ile, Val or Thr are located in the hydrophobic core. Furthermore, the e, g, e', and g' positions in the SSPs are restricted such that an attractive electrostatic interaction always occurs at these positions between the two polypeptide chains in an SSP dimer. This makes the SSP polypeptides more stable as dimers.

Thus, the novel synthetic storage proteins described in this invention represent a particular subset of possible coiled-coil polypeptides. Not all polypeptides which adopt an amphipathic alpha helical conformation in aqueous solution are suitable for the applications described here.

The following rules derived from Applicants' work define the SSP polypeptides that Applicants use in their invention:

The synthetic polypeptide comprises n heptad units (d e f g a b c), each heptad being either the same or different, wherein:
- n: is at least 4;
- a and d: are independently selected from the group consisting of Met, Leu, Val, Ile and Thr;
- e and g: are independently selected from the group consisting of the acid/base pairs Glu/Lys, Lys/Glu, Arg/Glu, Arg/Asp, Lys/Asp, Glu/Arg, Asp//Arg and Asp/Lys; and
- b, c and f: are independently any amino acids except Gly or Pro and at least two amino acids of b, c and f in each heptad are selected from the group consisting of Glu, Lys, Asp, Arg, His, Thr, Ser, Asn, Gln, Cys and Ala.

### Chimeric Genes Encoding Lysine-Rich Polypeptides

DNA sequences which encode the polypeptides described above can be designed based upon the genetic code. Where multiple codons exist for particular amino acids, codons should be chosen from those preferable for translation in plants. Oligonucleotides corresponding to these DNA sequences can be synthesized using an ABI DNA synthesizer, annealed with oligonucleotides corresponding to the complementary strand and inserted into a plasmid vector by methods known to those skilled in the art. The encoded polypeptide sequences can be lengthened by inserting additional annealed oligonucleotides at restriction endonuclease sites engineered into the synthetic gene. Some representative strategies for constructing genes encoding lysine-rich polypeptides of the invention, as well as DNA and amino acid sequences of preferred embodiments are provided in Example 8.

A chimeric gene designed to express RNA for a synthetic storage protein gene encoding a lysine-rich polypeptide can be constructed by linking the gene to any of the plant promoter sequences described above. Preferred promoters would be seed-specific promoters. For soybean, rapeseed and other dicotyledonous plants strong seed-specific promoters from a bean phaseolin gene, a soybean β-conglycinin gene, glycinin gene, Kunitz trypsin inhibitor gene, or rapeseed napin gene would be preferred. For corn or other monocotyledonous plants, a strong endosperm-specific promoter, e.g., the 10 kD or 27 kD zein promoter, or a strong embyro-specific promoter, e.g., the corn globulin 1 promoter, would be preferred.

In order to obtain plants that express a chimeric gene for a synthetic storage protein gene encoding a lysine-rich polypeptide, plants can be transformed by any of the methods described above. In order to obtain plants that express both a chimeric SSP gene and a chimeric gene encoding lysine-insensitive DHDPS, the SSP gene could be linked to the chimeric gene encoding lysine-insensitive DHDPS and the two genes could be introduced into plants via transformation. Alternatively, the chimeric SSP gene could be introduced into previously transformed plants that express lysine-insensitive DHDPS, or the SSP gene could be introduced into normal plants and the transformants obtained could be crossed with plants that express lysine-insensitive DHDPS.

Results from genetic crosses of transformed plants containing lysine biosynthesis genes with transformed plants containing lysine-rich protein genes (see Example 10) demonstrate that the total lysine levels in seeds can be increased by the coordinate expression of these genes. This result was especially striking because the gene copy number of all of the transgenes was reduced in the hybrid. It is expected that the lysine level would be further increased if the biosynthesis genes and the lysine-rich protein genes were all homozygous.

### EXAMPLES

The present invention is further defined in the following Examples, in which all parts and percentages are by weight and degrees are Celsius, unless otherwise stated.

### EXAMPLE 1

### Isolation of the E. coli and Corynebacterium glutamicum dapA genes

The E. coli dapA gene (ecodapA) has been cloned, restriction endonuclease mapped and sequenced previously [Richaud et al. (1986) J. Bacteriol. 166:297-300]. For the present invention the dapA gene was obtained on a bacteriophage lambda clone from an ordered library of 3400 overlapping segments of cloned E. coli DNA constructed by Kohara, Akiyama and Isono [Kohara et al. (1987) Cell 50:595-508]. From the knowledge of the map position of dapA at 53 min on the E. coli genetic map [Bachman (1983) Microbiol. Rev. 47:180-230], the restriction endonuclease map of the cloned gene [Richaud et al. (1986) J. Bacteriol. 166:297-300], and the restriction endonuclease map of the cloned DNA fragments in the E. coli library [Kohara et al. (1987) Cell 50:595-508], it was possible to choose lambda phages 4C11 and 5A8 [Kohara et al. (1987) Cell 50:595-508] as likely candidates for carrying the dapA gene. The phages were grown in liquid culture from single plaques as described [see Current Protocols in Molecular Biology (1987) Ausubel et al. eds., John Wiley & Sons New York] using LE392 as host [see Sambrook et al. (1989) Molecular Cloning: a Laboratory Manual, Cold Spring Harbor Laboratory Press]. Phage DNA was prepared by phenol extraction as described [see Current Protocols in Molecular Biology (1987) Ausubel et al. eds., John Wiley & Sons, New York]. Both phages contained an approximately 2.8 kb Pst I DNA fragment expected for the dapA gene [Richaud et al. (1986) J. Bacteriol. 166:297-300]. The fragment was isolated from the digest of phage 5A8 and inserted into Pst I digested vector pBR322 yielding plasmid pBT427.

The Corynebacterium dapA gene (cordapA) was isolated from genomic DNA from ATCC strain 13032 using polymerase chain reaction (PCR). The nucleotide sequence of the Corynebacterium dapA gene has been published [Bonnassie et al. (1990) Nucleic Acids Res. 18:6421]. From the sequence it was possible to design oligonucleotide primers for PCR that would allow amplification of a DNA fragment containing the gene, and at the same time add unique restriction endonuclease sites at the start codon (Nco I) and just past the stop codon (EcoR I) of the gene. The oligonucleotide primers used were:
SEQ ID NO:1:
   CCCGGGCCAT GGCTACAGGT TTAACAGCTA AGACCGGAGT AGAGCACT
SEQ ID NO:2:
   GATATCGAAT TCTCATTATA GAACTCCAGC TTTTTTC

PCR was performed using a Perkin-Elmer Cetus kit according to the instructions of the vendor on a thermocycler manufactured by the same company. The reaction product, when run on an agarose gel and stained with ethidium bromide, showed a strong DNA band of the size expected for the Corynebacterium dapA gene, about 900 bp. The PCR-generated fragment was digested with restriction endonucleases Nco I and EcoR I and inserted into expression vector pBT430 (see Example 2) digested with the same enzymes. In addition to introducing an Nco I site at the translation start codon, the PCR primers also resulted in a change of the second codon from AGC coding for serine to GCT coding for alanine. Several clones that expressed active, lysine-insensitive DHDPS (see Example 2) were isolated, indicating that the second codon amino acid substitution did not affect activity; one clone was designated FS766.

The Nco I to EcoR I fragment carrying the PCR-generated Corynebacterium dapA gene was subcloned into the phagemid vector pGEM-9Zf(-) from Promega, single-stranded DNA was prepared and sequenced. This sequence is shown in SEQ ID NO:3.

Aside from the differences in the second codon already mentioned, the sequence matched the published sequence except at two positions, nucleotides 798 and 799. In the published sequence these are TC, while in the gene shown in SEQ ID NO:3 they are CT. This change results in an amino acid substitution of leucine for serine. The reason for this difference is not known. It may be due to an error in the published sequence, the difference in strains used to isolate the gene, or a PCR-generated error. The latter seems unlikely since the same change was observed in at least 3 independently isolated PCR-generated dapA genes. The difference has no apparent effect on DHDPS enzyme activity (see Example 2).

### EXAMPLE 2

### High level expression of the E. coli and Corynebacterium glutamicum dapA genes in E. coli

An Nco I (CCATGG) site was inserted at the translation initiation codon of the E. coli dapA gene using oligonucleotide-directed mutagenesis. The 2.8 kb Pst I DNA fragment carrying the dapA gene in plasmid pBT427 (see Example 1) was inserted into the Pst I site of phagemid vector pTZ18R (Pharmacia) yielding pBT431. The orientation of the dapA gene was such that the coding strand would be present on the single-stranded phagemid DNA. Oligonucleotide-directed mutagenesis was carried out using a Muta-Gene kit from Bio-Rad according to the manufacturer's protocol with the mutagenic primer shown below:
SEQ ID NO:4:
   CTTCCCGTGA CCATGGGCCA TC
Putative mutants were screened for the presence of an Nco I site and a plasmid, designated pBT437, was shown to have the the proper sequence in the vicinity of the mutation by DNA sequencing. The addition of an Nco I site at the translation start codon also resulted in a change of the second codon from TTC coding for phenylalanine to GTC coding for valine.

To achieve high level expression of the dapA genes in E. coli the bacterial expression vector pBT430. This expression vector is a derivative of pET-3a [Rosenberg et al. (1987) Gene 56:125-135] which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

The E. coli dapA gene was cut out of plasmid pBT437 as an 1150 bp Nco I-Hind III fragment and inserted into the expression vector pBT430 digested with the same enzymes, yielding plasmid pBT442. For expression of the Corynebacterium dapA gene, the 917 bp Nco I to EcoR I fragment of SEQ ID NO:3 inserted in pBT430 (pFS766, see Example 1) was used.

For high level expression each of the plasmids was transformed into E. coli strain BL21(DE3) [Studier et al. (1986) J. Mol. Biol. 189:113-130]. Cultures were grown in LB medium containing ampicillin (100 mg/L) at 25°C. At an optical density at 600 nm of approximately 1, IPTG (isopropylthio-β-galactoside, the inducer) was added to a final concentration of 0.4 mM and incubation was continued for 3 h at 25°C. The cells were collected by centrifugation and resuspended in 1/20th (or 1/100th) the original culture volume in 50 mM NaCl; 50 mM Tris-Cl, pH 7.5; 1 mM EDTA, and frozen at -20°C. Frozen aliquots of 1 mL were thawed at 37°C and sonicated, in an ice-water bath, to lyse the cells. The lysate was centrifuged at 4°C for 5 min at 15,000 rpm. The supernatant was removed and the pellet was resuspended in 1 mL of the above buffer.

The supernatant and pellet fractions of uninduced and IPTG-induced cultures of BL21(DE3)/pBT442 or BL21(DE3)/pFS766 were analyzed by SDS polyacrylamide gel electrophoresis. The major protein visible by Coomassie blue staining in the supernatant and pellet fractions of both induced cultures had a molecular weight of 32-34 kd, the expected size for DHDPS. Even in the uninduced cultures this protein was the most prominent protein produced.

In the BL21(DE3)/pBT442 IPTG-induced culture about 80% of the DHDPS protein was in the supernatant and DHDPS represented 10-20% of the total protein in the extract. In the BL21(DE3)/pFS766 IPTG-induced culture more than 50% of the DHDPS protein was in the pellet fraction. The pellet fractions in both cases were 90-95% pure DHDPS, with no other single protein present in significant amounts. Thus, these fractions were pure enough for use in the generation of antibodies. The pellet fractions containing 2-4 milligrams of either E. coli DHDPS or Corynebacterium DHDPS were solubilized in 50 mM NaCl; 50 mM Tris-Cl, pH 7.5; 1 mM EDTA, 0.2 mM dithiothreitol, 0.2% SDS and sent to Hazelton Research Facility (310 Swampridge Road, Denver, PA 17517) to have rabbit antibodies raised against the proteins.

DHDPS enzyme activity was assayed as follows: Assay mix (for 10 X 1.0 mL assay tubes or 90 X 0.25 mL for microtiter dish); made fresh, just before use:

| | |
|---|---|
| 2.5mL | H₂O |
| 0.5mL | 1.0M Tris-HCl pH8.0 |
| 0.5mL | 0.1M Na Pyruvate |
| 0.5mL | o-Aminobenzaldehyde (10mg/mL in ethanol) |
| 25µL | 1.0M DL-Aspartic-β-semialdehyde (ASA) in 1.0N HCl |

| | Assay (1.0mL): | MicroAssay (0.25mL): |
|---|---|---|
| DHDPS assay mix | 0.40mL | 0.10mL |
| enzyme extract + H₂O; | 0.10mL | .025mL |
| 10mM L-lysine | 5µL or 20µL | 1µL or 5µL |

| | | |
|---|---|---|
| Incubate at 30°C for desired time. | | Stop by addition of: |
| 1.0N HCl | 0.50mL | 0.125mL |

Color allowed to develop for 30-60 min. Precipitate spun down in eppendorf centrifuge. OD₅₄₀ vs 0 min read as blank. For MicroAssay, aliquot 0.2 mL into microtiter well and read at OD₅₃₀.

The specific activity of E. coli DHDPS in the supernatant fraction of induced extracts was about 50 OD₅₄₀ units per minute per milligram protein in a 1.0 mL assay. E. coli DHDPS was sensitive to the presence of L-lysine in the assay. Fifty percent inhibition was found at a concentration of about 0.5 mM. For Corynebacterium DHDPS, the activity was measured in the supernatant fraction of uninduced extracts, rather than induced extracts. Enzyme activity was about 4 OD₅₃₀ units per minute per milligram protein in a 0.25 mL assay. In contrast to E. coli DHDPS, Corynebacterium DHDPS was not inhibited at all by L-lysine, even at a concentration of 70 mM.

### EXAMPLE 3

### Isolation of the E. coli lysC Gene and mutations in lysC resulting in lysine-insensitive AKIII

The E. coli lysC gene has been cloned, restriction endonuclease mapped and sequenced previously [Cassan et al. (1986) J. Biol. Chem. 261:1052-1057]. For the present invention the lysC gene was obtained on a bacteriophage lambda clone from an ordered library of 3400 overlapping segments of cloned E. coli DNA constructed by Kohara, Akiyama and Isono [Kohara et al. (1987) Cell 50:595-508]. This library provides a physical map of the whole E. coli chromosome and ties the physical map to the genetic map. From the knowledge of the map position of lysC at 90 min. on the E. coli genetic map [Theze et al. (1974) J. Bacteriol. 117:133-143], the restriction endonuclease map of the cloned gene [Cassan et al. (1986) J. Biol. Chem. 261:1052-1057], and the restriction endonuclease map of the cloned DNA fragments in the E. coli library [Kohara et al. (1987) Cell 50:595-508], it was possible to choose lambda phages 4E5 and 7A4 [Kohara et al. (1987) Cell 50:595-508] as likely candidates for carrying the lysC gene. The phages were grown in liquid culture from single plaques as described [see Current Protocols in Molecular Biology (1987) Ausubel et al. eds. John Wiley & Sons New York] using LE392 as host [see Sambrook et al. (1989) Molecular Cloning: a Laboratory Manual, Cold Spring Harbor Laboratory Press]. Phage DNA was prepared by phenol extraction as described [see Current Protocols in Molecular Biology (1987) Ausubel et al. eds. John Wiley & Sons, New York].

From the sequence of the gene several restriction endonuclease fragments diagnostic for the lysC gene were predicted, including an 1860 bp EcoR I-Nhe I fragment, a 2140 bp EcoR I-Xmn I fragment and a 1600 bp EcoR I-BamH I fragment. Each of these fragments was detected in both of the phage DNAs confirming that these carried the lysC gene. The EcoR I-Nhe I fragment was isolated and subcloned in plasmid pBR322 digested with the same enzymes, yielding an ampicillin-resistant, tetracycline-sensitive E. coli transformant. The plasmid was designated pBT436.

To establish that the cloned lysC gene was functional, pBT436 was transformed into E. coli strain Gif106M1 (E. coli Genetic Stock Center strain CGSC-5074) which has mutations in each of the three E. coli AK genes [Theze et al. (1974) J. Bacteriol. 117:133-143]. This strain lacks all AK activity and therefore requires diaminopimelate (a precursor to lysine which is also essential for cell wall biosynthesis), threonine and methionine. In the transformed strain all these nutritional requirements were relieved demonstrating that the cloned lysC gene encoded functional AKIII.

Addition of lysine (or diaminopimelate which is readily converted to lysine in vivo) at a concentration of approximately 0.2 mM to the growth medium inhibits the growth of Gif106M1 transformed with pBT436. M9 media [see Sambrook et al. (1989) Molecular Cloning: a Laboratory Manual, Cold Spring Harbor Laboratory Press] supplemented with the arginine and isoleucine, required for Gif106M1 growth, and ampicillin, to maintain selection for the pBT436 plasmid, was used. This inhibition is reversed by addition of threonine plus methionine to the growth media. These results indicated that AKIII could be inhibited by exogenously added lysine leading to starvation for the other amino acids derived from aspartate. This property of pBT436-transformed Gif106M1 was used to select for mutations in lysC that encoded lysine-insensitive AKIII.

Single colonies of Gif106M1 transformed with pBT436 were picked and resuspended in 200 µL of a mixture of 100 µL 1% lysine plus 100 µL of M9 media. The entire cell suspension containing 10⁷-10⁸ cells was spread on a petri dish containing M9 media supplemented with the arginine, isoleucine, and ampicillin. Sixteen petri dishes were thus prepared. From 1 to 20 colonies appeared on 11 of the 16 petri dishes. One or two (if available) colonies were picked and retested for lysine resistance and from this nine lysine-resistant clones were obtained. Plasmid DNA was prepared from eight of these and re-transformed into Gif106M1 to determine whether the lysine resistance determinant was plasmid-borne. Six of the eight plasmid DNAs yielded lysine-resistant colonies. Three of these six carried lysC genes encoding AKIII that was uninhibited by 15mM lysine, whereas wild type AKIII is 50% inhibited by 0.3-0.4 mM lysine and >90% inhibited by 1 mM lysine (see Example 2 for details).

To determine the molecular basis for lysine-resistance the sequences of the wild type lysC gene and three mutant genes were determined. A method for "Using mini-prep plasmid DNA for sequencing double stranded templates with sequenase^{™}" [Kraft et al. (1988) BioTechniques 6:544-545] was used. Oligonucleotide primers, based on the published lysC sequence and spaced approximately every 200 bp, were synthesized to facilitate the sequencing. The sequence of the wild type lysC gene cloned in pBT436 (SEQ ID NO:5) differed from the published lysC sequence in the coding region at 5 positions. Four of these nucleotide differences were at the third position in a codon and would not result in a change in the amino acid sequence of the AKIII protein. One of the differences would result in a cysteine to glycine substitution at amino acid 58 of AKIII. These differences are probably due to the different strains from which the lysC genes were cloned.

The sequences of the three mutant lysC genes that encoded lysine-insensitive AK each differed from the wild type sequence by a single nucleotide, resulting in a single amino acid substitution in the protein. Mutant M2 had an A substituted for a G at nucleotide 954 of SEQ ID NO:5 resulting in an isoleucine for methionine substitution at amino acid 318 and mutants M3 and M4 had identical T for C substitutions at nucleotide 1055 of SEQ ID NO:5 resulting in an isoleucine for threonine substitution at amino acid 352. Thus, either of these single amino acid substitutions is sufficient to render the AKIII enzyme insensitive to lysine inhibition.

An Nco I (CCATGG) site was inserted at the translation initiation codon of the lysC gene using the following oligonucleotides:
SEQ ID NO:6:
   GATCCATGGC TGAAATTGTT GTCTCCAAAT TTGGCG
SEQ ID NO:7:
   GTACCGCCAA ATTTGGAGAC AACAATTTCA GCCATG
When annealled these oligonucleotides have BamH I and Asp 718 "sticky" ends. The plasmid pBT436 was digested with BamH I, which cuts upstream of the lysC coding sequence and Asp 718 which cuts 31 nucleotides downstream of the initiation codon. The annealled oligonucleotides were ligated to the plasmid vector and E. coli transformants were obtained. Plasmid DNA was prepared and screened for insertion of the oligonucleotides based on the presence of an Nco I site. A plasmid containing the site was sequenced to assure that the insertion was correct, and was designated pBT457. In addition to creating an Nco I site at the initiation codon of lysC, this oligonucleotide insertion changed the second codon from TCT, coding for serine, to GCT, coding for alanine. This amino acid substitution has no apparent effect on the AKIII enzyme activity.

The lysC gene was cut out of plasmid pBT457 as a 1560 bp Nco I-EcoR I fragment and inserted into the expression vector pBT430 digested with the same enzymes, yielding plasmid pBT461. For expression of the mutant lysC-M4 gene pBT461 was digested with Kpn I-EcoR I, which removes the wild type lysC gene from about 30 nucleotides downstream from the translation start codon, and inserting the analogous Kpn I-EcoR I fragments from the mutant genes yielding plasmid pBT492.

### EXAMPLE 4

### Construction of Chimeric dapA Genes for Expression in the Seeds of Plants

A seed-specific expression cassette (Figure 4) is composed of the promoter and transcription terminator from the gene encoding the β subunit of the seed storage protein phaseolin from the bean Phaseolus vulgaris [Doyle et al. (1986) J. Biol. Chem. 261:9228-9238]. The phaseolin cassette includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon), Sma I, Kpn I and Xba I. The entire cassette is flanked by Hind III sites.

Plant amino acid biosynthetic enzymes are known to be localized in the chloroplasts and therefore are synthesized with a chloroplast targeting signal. Bacterial proteins such as DHDPS and AKIII have no such signal. A chloroplast transit sequence (cts) was therefore fused to the dapA and lysC-M4 coding sequence in the chimeric genes. The cts used was based on the the cts of the small subunit of ribulose 1,5-bisphosphate carboxylase from soybean [Berry-Lowe et al. (1982) J. Mol. Appl. Genet. 1:483-498]. The oligonucleotides SEQ ID NOS:8-11 were synthesized and used as described below.

Three chimeric genes were created:
No. 1) phaseolin 5' region/cts/lysC-M4/phaseolin 3' region
No. 2) phaseolin 5' region/cts/ecodapA/phaseolin 3' region
No. 3) phaseolin 5' region/cts/cordapA/phaseolin 3' region

Oligonucleotides SEQ ID NO:8 and SEQ ID NO:9, which encode the carboxy terminal part of the chloroplast targeting signal, were annealed, resulting in Nco I compatible ends, purified via polyacrylamide gel electrophoresis, and inserted into Nco I digested pBT461. The insertion of the correct sequence in the correct orientation was verified by DNA sequencing yielding pBT496. Oligonucleotides SEQ ID NO:10 and SEQ ID NO:11, which encode the amino terminal part of the chloroplast targeting signal, were annealed, resulting in Nco I compatible ends, purified via polyacrylamide gel electrophoresis, and inserted into Nco I digested pBT496. The insertion of the correct sequence in the correct orientation was verified by DNA sequencing yielding pBT521. Thus the cts was fused to the lysC gene.

To fuse the cts to the lysC-M4 gene, pBT521 was digested with Sal I, and an approximately 900 bp DNA fragment that included the cts and the amino terminal coding region of lysC was isolated. This fragment was inserted into Sal I digested pBT492, effectively replacing the amino terminal coding region of lysC-M4 with the fused cts and the amino terminal coding region of lysC. Since the mutation that resulted in lysine-insensitivity was not in the replaced fragment, the new plasmid, pBT523, carried the cts fused to lysC-M4.

The 1600 bp Nco I-Hpa I fragment containing the cts fused to lysC-M4 plus about 90 bp of 3' non-coding sequence was isolated and inserted into the seed-specific expression cassette digested with Nco I and Sma I (chimeric gene No. 1), yielding plasmid pBT544.

Before insertion into the expression cassette, the ecodapA gene was modified to insert a restriction endonuclease site, Kpn I, just after the translation stop codon. The oligonucleotides SEQ ID NOS:12-13 were synthesized for this purpose:
SEQ ID NO:12:
   CCGGTTTGCT GTAATAGGTA CCA
SEQ ID NO:13:
   AGCTTGGTAC CTATTACAGC AAACCGGCAT G

Oligonucleotides SEQ ID NO:12 and SEQ ID NO:13 were annealed, resulting in an Sph I compatible end on one end and a Hind III compatible end on the other and inserted into Sph I plus Hind III digested pBT437. The insertion of the correct sequence was verified by DNA sequencing yielding pBT443.

An 880 bp Nco I-Kpn I fragment from pBT443 containing the entire ecodapA coding region was isolated from an agarose gel following electrophoresis and inserted into the seed-specific expression cassette digested with Nco I and Kpn I, yielding plasmid pBT494. Oligonucleotides SEQ ID NO:8-11 were used as described above to add a cts to the ecodapA coding region in the seed-specific expression cassette, yielding chimeric gene No. 2 in pBT520.

An 870 bp Nco I-EcoR I fragment from pFS766 containing the entire cordapA coding region was isolated from an agarose gel following electrophoresis and inserted into the leaf expression cassette digested with Nco I and EcoR I, yielding plasmid pFS789 To attach the cts to the cordapA gene a DNA fragment containing the entire cts was prepared using PCR. The template DNA was pBT544 and the oligonucleotide primers used were:
SEQ ID NO:14:
   GCTTCCTCAA TGATCTCCTC CCCAGCT
SEQ ID NO:15:
   CATTGTACTC TTCCACCGTT GCTAGCAA

PCR was performed using a Perkin-Elmer Cetus kit according to the instructions of the vendor on a thermocycler manufactured by the same company. The PCR-generated 160 bp fragment was treated with T4 DNA polymerase in the presence of the 4 deoxyribonucleotide triphosphates to obtain a blunt-ended fragment. The cts fragment was inserted into the Nco I containing the start codon of the cordapA gene which had been digested and treated with the Klenow fragment of DNA polymerase to fill in the 5' overhangs. The inserted fragment and the vector/insert junctions were determined to be correct by DNA sequencing.

A 1030 bp Nco I-Kpn I fragment containing the cts attached to the cordapA coding region was isolated from an agarose gel following electrophoresis and inserted into the phaseolin seed expression cassette digested with Nco I and Kpn I, yielding plasmid pFS889 containing chimeric gene No. 3.

### EXAMPLE 5

### Transformation of Rapeseed with the Phaseolin Promoter/cts/cordapA and Phaseolin Promoter/cts/lysC-M4 Chimeric Genes

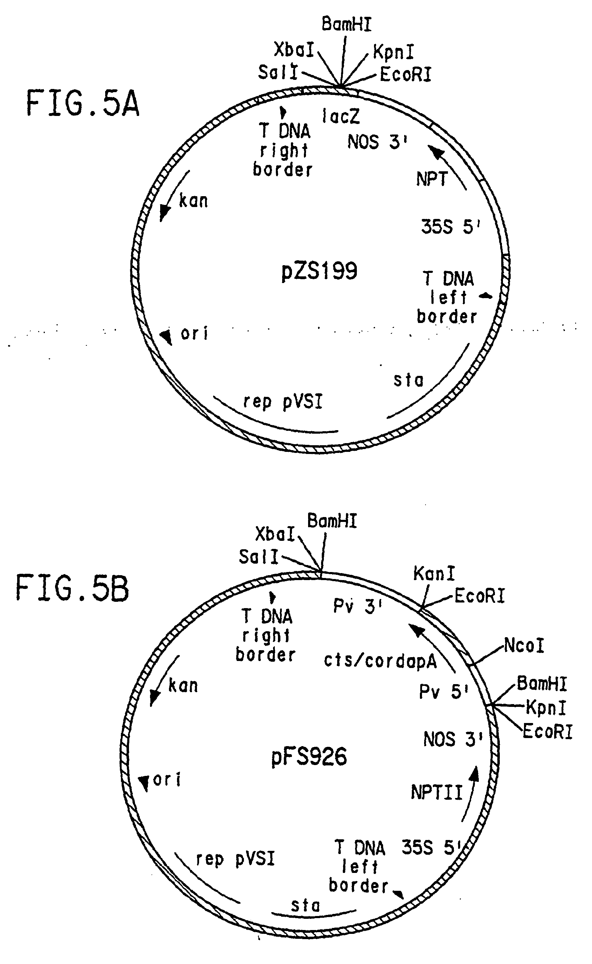

The chimeric gene cassettes, phaseolin 5' region/cts/cordapA/phaseolin 3' region, phaseolin 5' region/cts/lysC-M4/phaseolin 3', and phaseolin 5' region/cts/cordapA/phaseolin 3' region plus phaseolin 5' region/cts/lysC-M4/phaseolin 3' (Example 4) were inserted into the binary vector pZS199 (Figure 5A). In pZS199 the 35S promoter from Cauliflower Mosaic Virus drives expression of the NPT II.

The phaseolin 5' region/cts/cordapA/phaseolin 3' region chimeric gene cassette was modified using oligonucleotide adaptors to convert the Hind III sites at each end to BamH I sites. The gene cassette was then isolated as a 2.7 kb BamH I fragment and inserted into BamH I digested pZS199, yielding plasmid pFS926 (Figure 5B). This binary vector has the chimeric gene, phaseolin 5' region/cts/cordapA/phaseolin 3' region inserted in the same orientation as the 35S/NPT II/nos 3' marker gene.

To insert the phaseolin 5' region/cts/lysC-M4/phaseolin 3' region, the gene cassette was isolated as a 3.3 kb EcoR I to Spe I fragment and inserted into EcoR I plus Xba I digested pZS199, yielding plasmid pBT593. This binary vector has the chimeric gene, phaseolin 5' region/cts/lysC-M4/phaseolin 3' region inserted in the same orientation as the 35S/NPT II/nos 3' marker gene.

To combine the two cassettes, the EcoR I site of pBT593 was converted to a BamH I site using oligonucleotide adaptors, the resulting vector was cut with BamH I and the phaseolin 5' region/cts/cordapA/ phaseolin 3' region gene cassette was isolated as a 2.7 kb BamH I fragment and inserted, yielding pBT597. This binary vector has both chimeric genes, phaseolin 5' region/cts/cordapA/phaseolin 3' region and phaseolin 5' region/cts/lysC-M4/phaseolin 3' region inserted in the same orientation as the 35S/NPT II/nos 3' marker gene.

Brassica napus cultivar "Westar" was transformed by co-cultivation of seedling pieces with disarmed Agrobacterium tumefaciens strain LBA4404 carrying the the appropriate binary vector.

B. napus seeds were sterilized by stirring in 10% Chlorox, 0.1% SDS for thirty min, and then rinsed thoroughly with sterile distilled water. The seeds were germinated on sterile medium containing 30 mM CaCl2 and 1.5% agar, and grown for six d in the dark at 24°C.

Liquid cultures of Agrobacterium for plant transformation were grown overnight at 28°C in Minimal A medium containing 100 mg/L kanamycin. The bacterial cells were pelleted by centrifugation and resuspended at a concentration of 10⁸ cells/mL in liquid Murashige and Skoog Minimal Organic medium containing 100 uM acetosyringone.

B. napus seedling hypocotyls were cut into 5 mm segments which were immediately placed into the bacterial suspension. After 30 min, the hypocotyl pieces were removed from the bacterial suspension and placed onto BC-35 callus medium containing 100 uM acetosyringone. The plant tissue and Agrobacteria were co-cultivated for three d at 24°C in dim light.

The co-cultivation was terminated by transferring the hypocotyl pieces to BC-35 callus medium containing 200 mg/L carbenicillin to kill the Agrobacteria, and 25 mg/L kanamycin to select for transformed plant cell growth. The seedling pieces were incubated on this medium for three weeks at 24°C under continuous light.

After three weeks, the segments were transferred to BS-48 regeneration medium containing 200 mg/L carbenicillin and 25 mg/L kanamycin. Plant tissue was subcultured every two weeks onto fresh selective regeneration medium, under the same culture conditions described for the callus medium. Putatively transformed calli grew rapidly on regeneration medium; as calli reached a diameter of about 2 mm, they were removed from the hypocotyl pieces and placed on the same medium lacking kanamycin

Shoots began to appear within several weeks after transfer to BS-48 regeneration medium. As soon as the shoots formed discernable stems, they were excised from the calli, transferred to MSV-1A elongation medium, and moved to a 16:8-h photoperiod at 24°C.

Once shoots had elongated several internodes, they were cut above the agar surface and the cut ends were dipped in Rootone. Treated shoots were planted directly into wet Metro-Mix 350 soiless potting medium. The pots were covered with plastic bags which were removed when the plants were clearly growing, after about ten d. Results of the transformation are shown in Table 1. Transformed plants were obtained with each of the binary vectors.

### Minimal A Bacterial Growth Medium

Dissolve in distilled water:
10.5 g potassium phosphate, dibasic
4.5 g potassium phosphate, monobasic
1.0 g ammonium sulfate
0.5 g sodium citrate, dihydrate
Make up to 979 mL with distilled water
Autoclave
Add 20 mL filter-sterilized 10% sucrose
Add 1 mL filter-sterilized 1 M MgSO4

### Brassica Callus Medium BC-35

Per liter:
Murashige and Skoog Minimal Organic Medium
(MS salts, 100 mg/L i-inositol, 0.4 mg/L thiamine; GIBCO #510-3118)
30 g sucrose
18 g mannitol
0.5 mg/L 2,4-D
0.3 mg/L kinetin
0.6% agarose
pH 5.8

### Brassica Regeneration Medium BS-48

Murashige and Skoog Minimal Organic Medium
Gamborg B5 Vitamins (SIGMA #1019)
10 g glucose
250 mg xylose
600 mg MES
0.4% agarose
pH 5.7

Filter-sterilize and add after autoclaving:
2.0 mg/L zeatin
0.1 mg/L IAA

### Brassica Shoot Elongation Medium MSV-1A

Murashige and Skoog Minimal Organic Medium
Gamborg B5 Vitamins
10 g sucrose
0.6% agarose
pH 5.8

**TABLE 1 Canola transformants**

| BINARY VECTOR | NUMBER OF CUT ENDS | NUMBER OF KAN^{R} CALLI | NUMBER OF SHOOTING CALLI | NUMBER OF PLANTS |
|---|---|---|---|---|
| pZS199 | 120 | 41 | 5 | 2 |
| pFS926 | 600 | 278 | 52 | 28 |
| pBT593 | 600 | 70 | 10 | 3 |
| pBT597 | 600 | 223 | 40 | 23 |

Plants were grown under a 16:8-h photoperiod, with a daytime temperature of 23°C and a nightime temperature of 17°C. When the primary flowering stem began to elongate, it was covered with a mesh pollen-containment bag to prevent outcrossing. Self-pollination was facilitated by shaking the plants several times each day. Mature seeds derived from self-pollinations were harvested about three months after planting.

A partially defatted seed meal was prepared as follows: 40 milligrams of mature dry seed was ground with a mortar and pestle under liquid nitrogen to a fine powder. One milliliter of hexane was added and the mixture was shaken at room temperature for 15 min. The meal was pelleted in an eppendorf centrifuge, the hexane was removed and the hexane extraction was repeated. Then the meal was dried at 65° for 10 min until the hexane was completely evaporated leaving a dry powder. Total proteins were extracted from mature seeds as follows. Approximately 30-40 mg of seeds were put into a 1.5 mL disposable plastic microfuge tube and ground in 0.25 mL of 50 mM Tris-HCl pH 6.8, 2 mM EDTA, 1% SDS, 1% β-mercaptoethanol. The grinding was done using a motorized grinder with disposable plastic shafts designed to fit into the microfuge tube. The resultant suspensions were centrifuged for 5 min at room temperature in a microfuge to remove particulates. Three volumes of extract was mixed with 1 volume of 4 X SDS-gel sample buffer (0.17M Tris-HCl pH6.8, 6.7% SDS, 16.7% β-mercaptoethanol, 33% glycerol) and 5 µL from each extract were run per lane on an SDS polyacrylamide gel, with bacterially produced DHDPS or AKIII serving as a size standard and protein extracted from untransformed tobacco seeds serving as a negative control. The proteins were then electrophoretically blotted onto a nitrocellulose membrane. The membranes were exposed to the DHDPS or AKIII antibodies at a 1:5000 dilution of the rabbit serum using standard protocol provided by BioRad with their Immun-Blot Kit. Following rinsing to remove unbound primary antibody the membranes were exposed to the secondary antibody, donkey anti-rabbit Ig conjugated to horseradish peroxidase (Amersham) at a 1:3000 dilution. Following rinsing to remove unbound secondary antibody, the membranes were exposed to Amersham chemiluminescence reagent and X-ray film.

Eight of eight FS926 transformants and seven of seven BT597 transformants expressed the DHDPS protein. The single BT593 transformant and five of seven BT597 transformants expressed the AKIII-M4 protein (Table 2).

To measure free amino acid composition of the seeds, free amino acids were extracted from 40 milligrams of the defatted meal in 0.6 mL of methanol/chloroform/water mixed in ratio of 12v/5v/3v (MCW) at room temperature. The mixture was vortexed and then centrifuged in an eppendorf microcentrifuge for about 3 min. Approximately 0.6 mL of supernatant was decanted and an additional 0.2 mL of MCW was added to the pellet which was then vortexed and centrifuged as above. The second supernatant, about 0.2 mL, was added to the first. To this, 0.2 mL of chloroform was added followed by 0.3 mL of water. The mixture was vortexed and then centrifuged in an eppendorf microcentrifuge for about 3 min, the upper aqueous phase, approximately 1.0 mL, was removed, and was dried down in a Savant Speed Vac Concentrator. The samples were hydrolyzed in 6N hydrochloric acid, 0.4% β-mercaptoethanol under nitrogen for 24 h at 110-120°C; 1/4 of the sample was run on a Beckman Model 6300 amino acid analyzer using post-column ninhydrin detection. Relative free amino acid levels in the seeds were compared as ratios of lysine or threonine to leucine, thus using leucine as an internal standard.

There was a good correlation between transformants expressing higher levels of DHDPS protein and those having higher levels of free lysine. The highest expressing lines showed a greater than 100-fold increase in free lysine level in the seeds. There has been no greater accumulation of free lysine due to expression of AKIII-M4 along with Corynebacteria DHDPS compared to expression of Corynebacteria DHDPS alone. The transformant that expressed AKIII-M4 in the absence of Corynebacteria DHDPS showed a 5-fold increase in the level of free threonine in the seeds. A high level of α-aminoadipic acid, indicative of lysine catabolism, was observed in many of the transformed lines. Thus, prevention of lysine catabolism by inactivation of lysine ketoglutarate reductase should further increase the accumulation of free lysine in the seeds. Alternatively, incorporation of lysine into a peptide or lysine-rich protein would prevent catabolism and lead to an increase in the accumulation of lysine in the seeds.

To measure the total amino acid composition of mature seeds, 2 milligrams of the defatted meal were hydrolyzed in 6N hydrochloric acid, 0.4% β-mercaptoethanol under nitrogen for 24 h at 110-120°C: 1/100 of the sample was run on a Beckman Model 6300 amino acid analyzer using post-column ninhydrin detection. Relative amino acid levels in the seeds were compared as percentages of lysine, threonine or α-aminoadipic acid to total amino acids. There was a good correlation between transformants expressing DHDPS protein and those having high levels of lysine. Seeds with a 5-100% increase in the lysine level, compared to the untransformed control, were observed. In the seeds with the highest levels, lysine makes up 11-13% of the total seed amino acids, considerably higher than any previously known rapeseed seed.

**TABLE 2 FS926 Transformants: phaseolin 5' region/cts/cordapA/phaseolin 3' BT593 Transformants: phaseolin 5' region/cts/lysC-M4/phaseolin 3' BT597 Transformants: phaseolin 5' region/cts/lysC-M4/phaseolin 3' phaseolin 5' region/cts/cordapA/phaseolin 3'**

| | FREE | AMINO | ACIDS | WESTERN CORYNE. | WESTERN E. COLI | % TOTAL AMINO ACIDS | | |
|---|---|---|---|---|---|---|---|---|
| LINE | K/L | T/L | AA/L | DHDPS | AKIII-M4 | K | T | AA |
| WESTAR | 0.8 | 2.0 | 0 | - | - | 6.5 | 5.6 | 0 |
| ZS199 | 1.3 | 3.2 | 0 | - | - | 6.3 | 5.4 | 0 |
| FS926-3 | 140 | 2.0 | 16 | ++++ | - | 12 | 5.1 | 1.0 |
| FS926-9 | 110 | 1.7 | 12 | ++++ | - | 11 | 5.0 | 0.8 |
| FS926-11 | 7.9 | 2.0 | 5.2 | ++ | - | 7.7 | 5.2 | 0 |
| FS926-6 | 14 | 1.8 | 4.6 | +++ | - | 8.2 | 5.9 | 0 |
| FS926-22 | 3.1 | 1.3 | 0.3 | + | - | 6.9 | 5.7 | 0 |
| FS926-27 | 4.2 | 1.9 | 1.1 | ++ | - | 7.1 | 5.6 | 0 |
| FS926-29 | 38 | 1.8 | 4.7 | ++++ | - | 12 | 5.2 | 1.6 |
| FS926-68 | 4.2 | 1.8 | 0.9 | ++ | - | 8.3 | 5.5 | 0 |
| BT593-42 | 1.4 | 11 | 0 | - | ++ | 6.3 | 6.0 | 0 |
| BT597-14 | 6.0 | 2.6 | 4.3 | ++ | +/- | 7.0 | 5.3 | 0 |
| BT597-145 | 1.3 | 2.9 | 0 | + | - | | | |
| BT597-4 | 38 | 3.7 | 4.5 | ++++ | ++++ | 13 | 5.6 | 1.6 |
| BT597-68 | 4.7 | 2.7 | 1.5 | ++ | + | 6.9 | 5.8 | 0 |
| BT597-100 | 9.1 | 1.9 | 1.7 | +++ | ++ | 6.6 | 5.7 | 0 |
| BT597-148 | 7.6 | 2.3 | 0.9 | +++ | + | 7.3 | 5.7 | 0 |
| BT597-169 | 5.6 | 2.6 | 1.7 | +++ | +++ | 6.6 | 5.7 | 0 |
| AA is α-amino adipic acid | | | | | | | | |

### EXAMPLE 6

### Transformation of Soybean with the Phaseolin Promoter/cts/cordapA and Phaseolin Promoter/cts/lysC-M4 Chimeric Genes

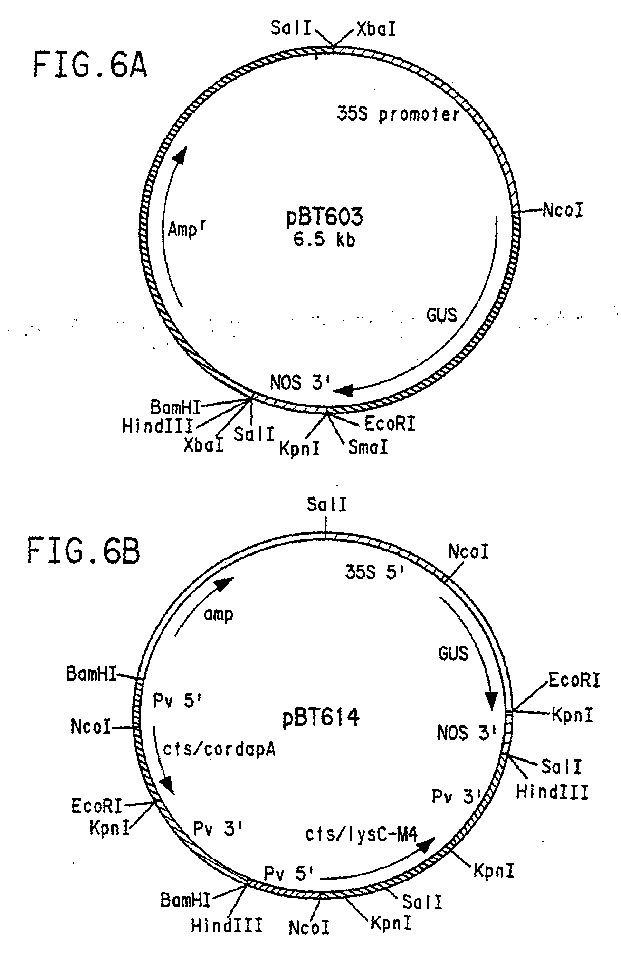

The chimeric gene cassettes, phaseolin 5' region/cts/cordapA/phaseolin 3' region plus phaseolin 5' region/cts/lysC-M4/phaseolin 3', (Example 4) were inserted into the soybean transformation vector pBT603 (Figure 6A). This vector has a soybean transformation marker gene consisting of the 35S promoter from Cauliflower Mosaic Virus driving expression of the E. coli β-glucuronidase (GUS) gene [Jefferson et al. (1986) Proc. Natl. Acad. Sci. USA 83:8447-8451] with the Nos 3' region in a modified pGEM9Z plasmid.

To insert the phaseolin 5' region/cts/lysC-M4/ phaseolin 3' region, the gene cassette was isolated as a 3.3 kb Hind III fragment and inserted into Hind III digested pBT603, yielding plasmid pBT609. This vector has the chimeric gene, phaseolin 5' region/ cts/lysC-M4/phaseolin 3' region inserted in the opposite orientation from the 35S/GUS/Nos 3' marker gene.

The phaseolin 5' region/cts/cordapA/phaseolin 3'region chimeric gene cassette was modified using oligonucleotide adaptors to convert the Hind III sites at each end to BamH I sites. The gene cassette was then isolated as a 2.7 kb BamH I fragment and inserted into BamH I digested pBT609, yielding plasmid pBT614 (Figure 6B). This vector has both chimeric genes, phaseolin 5' region/cts/cordapA/phaseolin 3' region plus phaseolin 5' region/cts/lysC-M4/phaseolin 3' inserted in the same orientation, and both are in the opposite orientation from the 35S/GUS/Nos 3' marker gene.

Plasmid pBT614 was introduced into soybean via transformation by Agracetus Company (Middleton, WI), according to the procedure described in United States Patent No. 5,015,580. Seeds from five transformed lines were obtained and analyzed.

It was expected that the transgenes would be segregating in the R1 seeds of the transformed plants. To identify seeds that carried the transformation marker gene, a small chip of the seed was cut off with a razor and put into a well in a a disposable plastic microtiter plate. A GUS assay mix consisting of 100 mM NaH₂PO₄, 10 mM EDTA, 0.5 mM K₄Fe(CN)₆, 0.1% Triton X-100, 0.5 mg/mL 5-Bromo-4-chloro-3-indolyl β-D-glucuronic acid was prepared and 0.15 mL was added to each microtiter well. The microtiter plate was incubated at 37° for 45 minutes. The development of blue color indicated the expression of GUS in the seed.

Four of five transformed lines showed approximately 3:1 segregation for GUS expression (Table 3). This indicates that the GUS gene was inserted at a single site in the soybean genome. The other transformant showed 9:1 segregation, suggesting that the GUS gene was inserted at two sites.

A meal was prepared from a fragment of individual seeds by grinding into a fine powder. Total proteins were extracted from the meal by adding 1 mg to 0.1 mL of 43 mM Tris-HCl pH 6.8, 1.7% SDS, 4.2% β-mercaptoethanol, 8% glycerol, vortexing the suspension, boiling for 2-3 minutes and vortexing again. The resultant suspensions were centrifuged for 5 min at room temperature in a microfuge to remove particulates and 10 µL from each extract were run per lane on an SDS polyacrylamide gel, with bacterially produced DHDPS or AKIII serving as a size standard. The proteins were then electrophoretically blotted onto a nitrocellulose membrane. The membranes were exposed to the DHDPS or AKIII antibodies, at a 1:5000 or 1:1000 dilution, respectively, of the rabbit serum using standard protocol provided by BioRad with their Immun-Blot Kit. Following rinsing to remove unbound primary antibody the membranes were exposed to the secondary antibody, donkey anti-rabbit Ig conjugated to horseradish peroxidase (Amersham) at a 1:3000 dilution. Following rinsing to remove unbound secondary antibody, the membranes were exposed to Amersham chemiluminescence reagent and X-ray film.

Four of five transformants expressed the DHDPS protein. In the four transformants that expressed DHDPS, there was excellent correlation between expression of GUS and DHDPS in individual seeds (Table 3). Therefore, the GUS and DHDPS genes are integrated at the same site in the soybean genome. Two of five transformants expressed the AKIII protein, and again there was excellent correlation between expression of AKIII, GUS and DHDPS in individual seeds (Table 3). Thus, in these two transformants the GUS, AKIII and DHDPS genes are integrated at the same site in the soybean genome. One transformant expressed only GUS in its seeds.

To measure free amino acid composition of the seeds, free amino acids were extracted from 8-10 milligrams of the meal in 1.0 mL of methanol/chloroform/water mixed in ratio of 12v/5v/3v (MCW) at room temperature. The mixture was vortexed and then centrifuged in an eppendorf microcentrifuge for about 3 min; approximately 0.8 mL of supernatant was decanted. To this supernatant, 0.2 mL of chloroform was added followed by 0.3 mL of water. The mixture was vortexed and then centrifuged in an eppendorf microcentrifuge for about 3 min, the upper aqueous phase, approximately 1.0 mL, was removed, and was dried down in a Savant Speed Vac Concentrator. The samples were hydrolyzed in 6N hydrochloric acid, 0.4% β-mercaptoethanol under nitrogen for 24 h at 110-120°C; 1/10 of the sample was run on a Beckman Model 6300 amino acid analyzer using post-column ninhydrin detection. Relative free amino acid levels in the seeds were compared as ratios of lysine to leucine, thus using leucine as an internal standard.

There was excellent correlation between transformants expressing Corynebacteria DHDPS protein and those having higher levels of free lysine. From 20 fold to 120-fold increases in free lysine level was observed in seeds expressing Corynebacteria DHDPS. A high level of saccharopine, indicative of lysine catabolism, was observed in seeds the contained high levels of lysine.

To measure the total amino acid composition of mature seeds, 1-1.4 milligrams of the seed meal was hydrolyzed in 6N hydrochloric acid, 0.4% β-mercaptoethanol under nitrogen for 24 h at 110-120°C; 1/50 of the sample was run on a Beckman Model 6300 amino acid analyzer using post-column ninhydrin detection. Lysine (and other amino acid) levels in the seeds were compared as percentages of the total amino acids.

There was excellent correlation between seeds expressing Corynebacteria DHDPS protein and those having high levels of lysine. Seeds with a 5-35% increase in the lysine level, compared to the untransformed control, were observed. In these seeds lysine makes up 7.5-7.7% of the total seed amino acids, considerably higher than any previously known soybean seed.

**TABLE 3**

| LINE-SEED | GUS | Free LYS/LEU | DHDPS | AKIII | % LYS TOT |
|---|---|---|---|---|---|
| A2396-145-4 | - | 0.9 | - | - | 5.75 |
| A2396-145-8 | - | 1 | - | - | |
| A2396-145-5 | - | 0.8 | | | 5.85 |
| A2396-145-3 | - | 1 | | | |
| A2396-145-9 | + | 2 | | | |
| A2396-145-6 | + | 4.6 | | | |
| A2396-145-1 | + | 8.7 | | | |
| A2396-145-10 | + | 18.4 | | | 7.54 |
| A2396-145-7 | + | 21.7 | + | - | 6.68 |
| A2396-145-2 | + | 45.5 | + | - | 7.19 |
| | | | | | |
| A5403-175-9 | - | 1.3 | | | |
| A5403-175-4 | - | 1.2 | - | - | 6.01 |
| A5403-175-3 | - | 1 | - | - | 6.02 |
| A5403-175-7 | + | 1.5 | | | |
| A5403-175-5 | + | 1.8 | | | |
| A5403-175-1 | + | 6.2 | | | |
| A5403-175-2 | + | 6.5 | | | 6.3 |
| A5403-175-6 | + | 14.4 | | | |
| A5403-175-8 | + | 47.8 | + | - | 7.67 |
| A5403-175-10 | + | 124.3 | + | - | 7.49 |
| | | | | | |
| A5403-181-9 | + | 1.4 | | | |
| A5403-181-10 | + | 1.4 | - | - | 5.68 |
| A5403-181-8 | + | 0.9 | | | |
| A5403-181-6 | + | 1.5 | | | |
| A5403-181-4 | - | 0.7 | - | - | 5.85 |
| A5403-181-5 | + | 1.1 | | | |
| A5403-181-2 | - | 1.8 | - | - | 5.59 |
| A5403-181-3 | + | 2.7 | - | - | 5.5 |
| A5403-181-7 | + | 1.9 | | | |
| A5403-181-1 | - | 2.3 | | | |
| | | | | | |
| A5403-183-9 | - | 0.8 | | | |
| A5403-183-6 | - | 0.7 | - | - | 6.03 |
| A5403-183-8 | - | 1.3 | | | |
| A5403-183-4 | - | 1.3 | - | - | 6.04 |
| A5403-183-5 | + | 0.9 | | | |
| A5403-183-3 | + | 3.1 | | | |
| A5403-183-1 | + | 3.3 | | | |
| A5403-183-7 | + | 9.9 | | | |
| A5403-183-10 | + | 22.3 | + | + | 6.74 |
| A5403-183-2 | + | 23.1 | + | + | 7.3 |
| | | | | | |
| A5403-196-8 | - | 0.9 | - | - | 5.92 |
| A5403-196-6 | + | 8.3 | | | |
| A5403-196-1 | + | 16.1 | + | + | 6.83 |
| A5403-196-7 | + | 27.9 | | | |
| A5403-196-3 | + | 52.8 | | | |
| A5403-196-5 | + | 26 | | | |
| A5403-196-2 | + | 16.2 | + | + | |
| A5403-196-10 | + | 29 | + | + | 7.53 |
| A5403-196-4 | + | 58.2 | + | + | 7.57 |
| A5403-196-9 | + | 47.1 | | | |
| wild type control | - | 0.9 | - | - | 5.63 |

Eighteen additional transformed soybean lines were obtained. Single seeds from the lines were analyzed for GUS activity as described above, and all lines exhibited GUS-positive seeds. Meal was prepared from single seeds, or in some cases a pool of several seeds, and assayed for expression of DHDPS and AKIII proteins via western blot. Seventeen of the eighteen lines expressed DHDPS, and fifteen of the eighteen expressed AKIII. Again there was excellent correlation between seeds expressing GUS, DHDPS and AKIII, indicating that the genes are linked in the transformed lines.

The amino acid composition of the seeds from these lines was determined as described above. Again seeds expressing Corynebacteria DHDPS protein showed increased levels of lysine. Expression of DHDPS alone resulted in 5% to 40% increases in total seed lysine. Expression of DHDPS along with AKIII-M4 results in lysine increases of more than 400%. A summary of all the different transformed lines is shown in Table 3A

**TABLE 3A**

| LINE-SEED | GUS + to - | DHDPS | AKIII | % LYS TOT |
|---|---|---|---|---|
| A2396-145 | 6 to 4 | + | - | 7.5 |
| A2396-233 | 3 to 1 | + | + | 25 |
| A2396-234 | 15 to 1 | + | + | 16 |
| A2396-248 | 4 to 10 | + | - | 6.3 |
| A2396-263 | 14 to 2 | - | - | |
| A2396-240 | 7 to 1 | + | + | 11 |
| A2396-267 | 2 to 53 | + | + | 8.9 |
| | | | | |
| A2242-273 | 11 to 5 | + | + | 13 |
| A2242-315 | 6 to 2 | + | + | 16 |
| A2242-316 | 1 to 15 | + | + | 12 |
| | | | | |
| A5403-175 | 7 to 3 | + | + | 7.6 |
| A5403-181 | 7 to 3 | - | - | 5.7 |
| A5403-183 | 6 to 4 | + | + | 6 |
| A5403-185 | 9 to 11 | + | + | 7.6 P |
| A5403-196 | 9 to 1 | + | + | 7.6 |
| A5403-203 | 6 to 36 | + | + | 6.1 P |
| A5403-204 | 17 to 3 | + | + | 8.8 P |
| A5403-212 | 13 to 5 | + | + | 9.4 P |
| A5403-214 | 21 to 16 | + | + | 32 |
| A5403-216 | 14 to 4 | + | - | 8.2 P |
| A5403-218 | 13 to 9 | + | + | 9.8 P |
| A5403-222 | 12 to 27 | + | + | 15 |
| A5403-225 | 14 to 12 | + | + | 13 |

P indicates seeds were pooled before meal extraction and assay

### EXAMPLE 7

### Isolation of a Plant Lysine Ketoglutarate Reductase Gene

Lysine Ketoglutarate Reductase (LKR) enzyme activity has been observed in immature endosperm of developing maize seeds [Arruda et al. (1982) Plant Physiol. 69:988-989]. LKR activity increases sharply from the onset of endosperm development, reaches a peak level at about 20 d after pollination, and then declines [Arruda et al. (1983) Phytochemistry 22:2687-2689].

In order to clone the corn LKR gene, RNA was isolated from developing seeds 19 d after pollination. This RNA was sent to Clontech Laboratories, Inc., (Palo Alto, CA) for the custom synthesis of a cDNA library in the vector Lambda Zap II. The conversion of the Lambda Zap II library into a phagemid library, then into a plasmid library was accomplished following the protocol provided by Clontech. Once converted into a plasmid library the ampicillin-resistant clones obtained carry the cDNA insert in the vector pBluescript SK(-). Expression of the cDNA is under control of the lacZ promoter on the vector.

Two phagemid libraries were generated using the mixtures of the Lambda Zap II phage and the filamentous helper phage of 100 µL to 1 µL. Two additional libraries were generated using mixtures of 100 µL Lambda Zap II to 10 µL helper phage and 20 µL Lambda Zap II to 10 µL helper phage. The titers of the phagemid preparations were similar regardless of the mixture used and were about 2 x 10³ ampicillin-resistant-transfectants per mL with E. coli strain XL1-Blue as the host and about 1 x 10³ with DE126 (see below) as host.

To select clones that carried the LKR gene a specially designed E. coli host, DE126 was constructed. Construction of DE126 occurred in several stages.
(1) A generalized transducing stock of coliphage Plvir was produced by infection of a culture of TST1 [F⁻, araD139, delta (argF-lac) 205, flb5301, ptsF25, relA1, rpsL150, malE52::Tn10, deoC1, λ⁻] (E. coli Genetic Stock Center #6137) using a standard method (for Methods see J. Miller, Experiments in Molecular Genetics).
(2) This phage stock was used as a donor in a transductional cross (for Method see J. Miller, Experiments in Molecular Genetics) with strain GIF106M1 [F⁻, arg-, ilvA296, lysC1001, thrA1101, metL1000, λ⁻, rpsL9, malT1, xyl-7, mtl-2, thi1(?), supE44(?)] (E. coli Genetic Stock Center #5074) as the recipient. Recombinants were selected on rich medium [L supplemented with DAP] containing the antibiotic tetracycline. The transposon Tn10, conferring tetracycline resistance, is inserted in the malE gene of strain TST1. Tetracycline-resistant transductants derived from this cross are likely to contain up to 2 min of the E. coli chromosome in the vicinity of malE. The genes malE and lysC are separated by less than 0.5 minutes, well within cotransduction distance.
(3) 200 tetracycline-resistant transductants were thoroughly phenotyped; appropriate fermentation and nutritional traits were scored. The recipient strain GIF106M1 is completely devoid of aspartokinase isozymes due to mutations in thrA, metL and lysC, and therefore requires the presence of threonine, methionine, lysine and meso-diaminopimelic acid (DAP) for growth. Transductants that had inherited lysC⁺ with malE::Tn10 from TST1 would be expected to grow on a minimal medium that contains vitamin B1, L-arginine, L-isoleucine and L-valine in addition to glucose which serves as a carbon and energy source. Moreover strains having the genetic constitution of lysC⁺, metL⁻ and thrA⁻ will only express the lysine sensitive aspartokinase. Hence addition of lysine to the minimal medium should prevent the growth of the lysC⁺ recombinant by leading to starvation for threonine, methionine and DAP. Of the 200 tetracycline resistant transductants examined, 49 grew on the minimal medium devoid of threonine, methionine and DAP. Moreover, all 49 were inhibited by the addition of L-lysine to the minimal medium. One of these transductants was designated DE125. DE125 has the phenotype of tetracycline resistance, growth requirements for arginine, isoleucine and valine, and sensitivity to lysine. The genotype of this strain is F- malE52::Tn10 arg- ilvA296 thrA1101 metL1000 lambda-rpsL9 malT1 xyl-7 mtl-2 thil(?) supE44(?).
(4) This step involves production of a male derivative of strain DE125. Strain DE125 was mated with the male strain AB1528 [F'16/delta(gpt-proA)62, lacY1 or lacZ4, glnV44, galK2 rac⁻(?), hisG4, rfbd1, mgl-51, kdgK51(?), ilvC7, argE3, thi-1] (E. coli Genetic Stock Center #1528) by the method of conjugation. F'16 carries the ilvGMEDAYC gene cluster. The two strains were cross streaked on rich medium permissive for the growth of each strain. After incubation, the plate was replica plated to a synthetic medium containing tetracycline, arginine, vitamin B1 and glucose. DE125 cannot grow on this medium because it cannot synthesize isoleucine. Growth of AB1528 is prevented by the inclusion of the antibiotic tetracycline and the omission of proline and histidine from the synthetic medium. A patch of cells grew on this selective medium. These recombinant cells underwent single colony isolation on the same medium. The phenotype of one clone was determined to be Ilv⁺, Arg⁻, TetR, Lysine-sensitive, male specific phage (MS2)-sensitive, consistent with the simple transfer of F'16 from AB1528 to DE125. This clone was designated DE126 and has the genotype F'16/malE52::Tn10, arg⁻, ilvA296, thrA1101, metL100, lysC⁺, λ⁻, rpsL9, malT1, xyl-7, mtl-2, thi-1?, supE44?. It is inhibited by 20 µg/mL of L-lysine in a synthetic medium.

To select for clones from the corn cDNA library that carried the LKR gene, 100 µL of the phagemid library was mixed with 100 µL of an overnight culture of DE126 grown in L broth and the cells were plated on synthetic media containing vitamin B1, L-arginine, glucose as a carbon and energy source, 100 µg/mL ampicillin and L-lysine at 20, 30 or 40 µg/mL. Four plates at each of the three different lysine concentrations were prepared. The amount of phagemid and DE126 cells was expected to yield about 1 x 10⁵ ampicillin-resistant transfectants per plate. Ten to thirty lysine-resistant colonies grew per plate (about 1 lysine-resistant per 5000 ampicillin-resistant colonies).

Plasmid DNA was isolated from 10 independent clones and retransformed into DE126. Seven of the ten DNAs yielded lysine-resistant clones demonstrating that the lysine-resistance trait was carried on the plasmid. Several of the cloned DNAs were sequenced and biochemically characterized. The inserted DNA fragments were found to be derived from the E. coli genome, rather than a corn cDNA indicating that the cDNA library provided by Clontech was contaminated. A new cDNA library will therefore be prepared and screened as described above.

### EXAMPLE 8

### Construction of Synthetic Genes in Expression Vector pSK5

To facilitate the construction and expression of the synthetic genes described below, it was necessary to construct a plasmid vector with the following attributes:
1. No Ear I restriction endonuclease sites such that insertion of sequences would produce a unique site.
2. Containing a tetracycline resistance gene to avoid loss of plasmid during growth and expression of toxic proteins.
3. Containing approximately 290 bp from plasmid pBT430 including the T7 promoter and terminator segment for expression of inserted sequences in E. coli.
4. Containing unique EcoR I and Nco I restriction endonuclease recognition sites in proper location behind the T7 promoter to allow insertion of the oligonucleotide sequences.

To obtain attributes 1 and 2 Applicants used plasmid pSK1 which was a spontaneous mutant of pBR322 where the ampicillin gene and the Ear I site near that gene had been deleted. Plasmid pSK1 retained the tetracycline resistance gene, the unique EcoR I restriction sites at base 1 and a single Ear I site at base 2353. To remove the Ear I site at base 2353 of pSK1 a polymerase chain reaction (PCR) was performed using pSK1 as the template. Approximately 10 femtomoles of pSK1 were mixed with 1 µg each of oligonucleotides SM70 and SM71 which had been synthesized on an ABI1306B DNA synthesizer using the manufacturer's procedures.
SM70 5'-CTGACTCGCTGCGCTCGGTC 3' SEQ ID NO:16
SM71 5'-TATTTTCTCCTTACGCATCTGTGC-3' SEQ ID NO:17

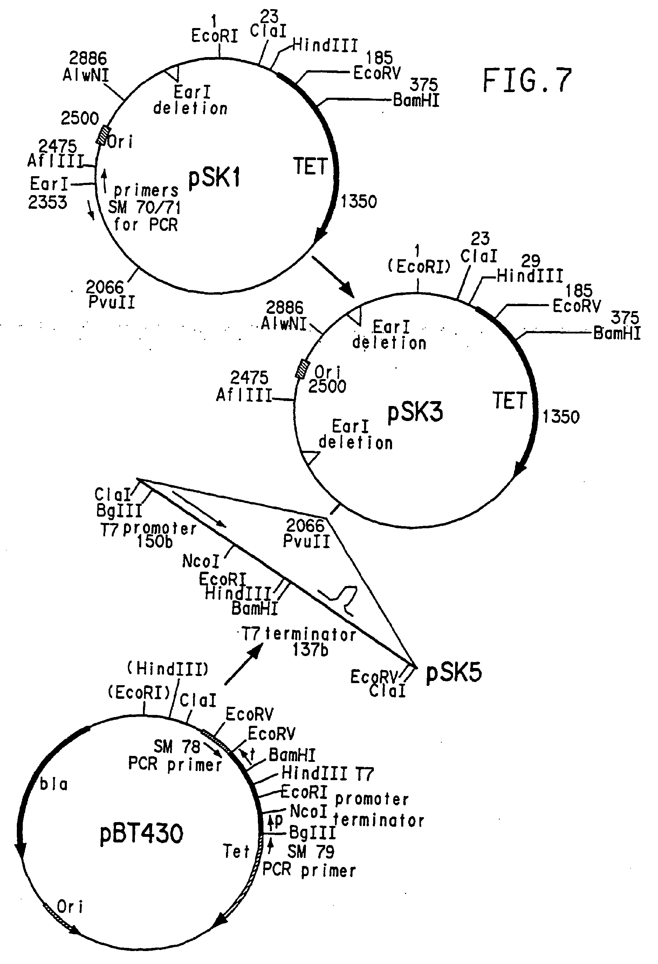

The priming sites of these oligonucleotides on the pSK1 template are depicted in Figure 7. The PCR was performed using a Perkin-Elmer Cetus kit (Emeryville, CA) according to the instructions of the vendor on a thermocycler manufactured by the same company. The 25 cycles were 1 min at 95°, 2 min at 42° and 12 min at 72°. The oligonucleotides were designed to prime replication of the entire pSK1 plasmid excluding a 30 b fragment around the Ear I site (see Figure 7). Ten microliters of the 100 µL reaction product were run on a 1% agarose gel and stained with ethidium bromide to reveal a band of about 3.0 kb corresponding to the predicted size of the replicated plasmid.

The remainder of the PCR reaction mix (90 µL) was mixed with 20 µL of 2.5 mM deoxynucleotide triphosphates (dATP, dTTP, dGTP, and dCTP), 30 units of Klenow enzyme added and the mixture incubated at 37° for 30 min followed by 65° for 10 min. The Klenow enzyme was used to fill in ragged ends generated by the PCR. The DNA was ethanol precipitated, washed with 70% ethanol, dried under vacuum and resuspended in water. The DNA was then treated with T4 DNA kinase in the presence of 1 mM ATP in kinase buffer. This mixture was incubated for 30 mins at 37° followed by 10 min at 65°. To 10 µL of the kinased preparation, 2 µL of 5X ligation buffer and 10 units of T4 DNA ligase were added. The ligation was carried out at 15° for 16 h. Following ligation, the DNA was divided in half and one half digested with Ear I enzyme. The Klenow, kinase, ligation and restriction endonuclease reactions were performed as described in Sambrook et al., [Molecular Cloning, A Laboratory Manual, 2nd ed. (1989) Cold Spring Harbor Laboratory Press]. Klenow, kinase, ligase and most restriction endonucleases were purchased from BRL. Some restriction endonucleases were purchased from NEN Biolabs (Beverly, MA) or Boehringer Mannheim (Indianapolis, IN). Both the ligated DNA samples were transformed separately into competent JM103 [supE thi del (lac-proAB) F' [traD36 porAB, lacIq lacZ del M15] restriction minus] cells using the CaCl₂ method as described in Sambrook et al., [Molecular Cloning, A Laboratory Manual, 2nd ed. (1989) Cold Spring Harbor Laboratory Press] and plated onto media containing 12.5 ug/mL tetracycline. With or without Ear I digestion the same number of transformants were recovered suggesting that the Ear I site had been removed from these constructs. Clones were screened by preparing DNA by the alkaline lysis miniprep procedure as described in Sambrook et al., [Molecular Cloning, A Laboratory Manual, 2nd ed. (1989) Cold Spring Harbor Laboratory Press] followed by restriction endonuclease digest analysis. A single clone was chosen which was tetracycline-resistant and did not contain any Ear I sites. This vector was designated pSK2. The remaining EcoR I site of pSK2 was destroyed by digesting the plasmid with EcoR I to completion, filling in the ends with Klenow and ligating. A clone which did not contain an EcoR I site was designated pSK3.

To obtain attributes 3 and 4 above, the bacteriophage T7 RNA polymerase promoter/terminator segment from plasmid pBT430 (see Example 2) was amplified by PCR. Oligonucleotide primers SM78 (SEQ ID NO:18) and SM79 (SEQ ID NO:19) were designed to prime a 300b fragment from pBT430 spanning the T7 promoter/terminator sequences (see Figure 7).
SM78 5'-TTCATCGATAGGCGACCACACCCGTCC-3' SEQ ID NO:18
SM79 5'-AATATCGATGCCACGATGCGTCCGGCG-3' SEQ ID NO:19

The PCR reaction was carried out as described previously using pBT430 as the template and a 300 bp fragment was generated. The ends of the fragment were filled in using Klenow enzyme and kinased as described above. DNA from plasmid pSK3 was digested to completion with PvuII enzyme and then treated with calf intestinal alkaline phophatase (Boehringer Mannheim) to remove the 5' phosphate. The procedure was as described in Sambrook et al., [Molecular Cloning, A Laboratory Manual, 2nd ed. (1989) Cold Spring Harbor Laboratory Press]. The cut and phosphatased pSK3 DNA was purified by ethanol precipitation and a portion used in a ligation reaction with the PCR generated fragment containing the T7 promoter sequence. The ligation mix was transformed into JM103 [supE thi del (lac-proAB) F' [traD36 porAB, lacIq lacZ del M15] restriction minus] and tetracycline-resistant colonies were screened. Plasmid DNA was prepared via the alkaline lysis mini prep method and restriction endonuclease analysis was performed to detect insertion and orientation of the PCR product. Two clones were chosen for sequence analysis: Plasmid pSK5 had the fragment in the orientation shown in Figure 7. Sequence analysis performed on alkaline denatured double-stranded DNA using Sequenase® T7 DNA polymerase (US Biochemical Corp) and manufacturer's suggested protocol revealed that pSK5 had no PCR replication errors within the T7 promoter/terminator sequence.

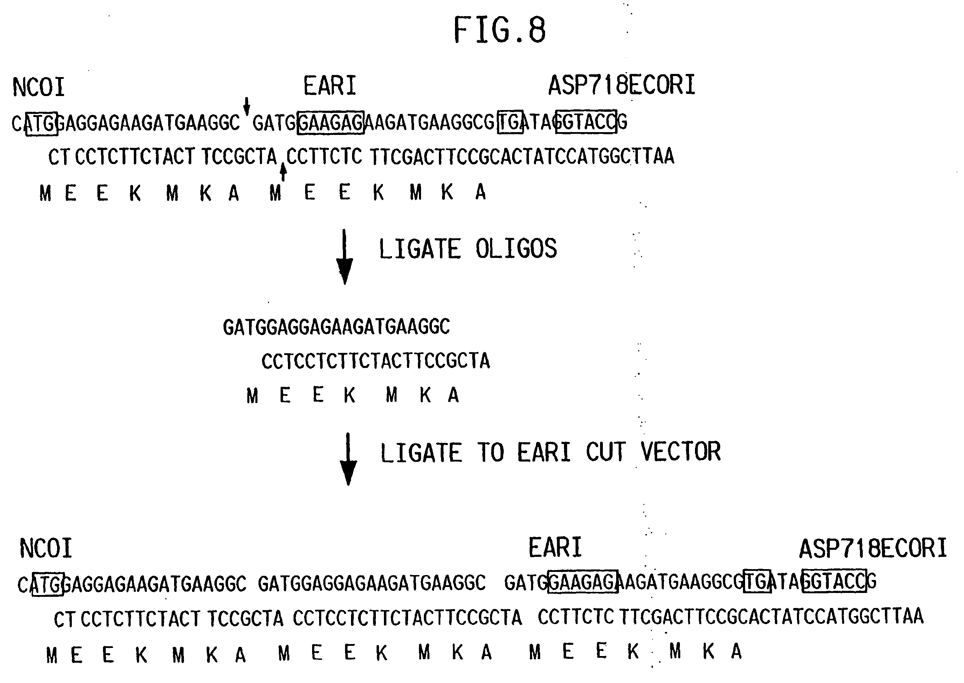

The strategy for the construction of repeated synthetic gene sequences based on the Ear I site is depicted in Figure 8. The first step was the insertion of an oligonucleotide sequence encoding a base gene of 14 amino acids. This oligonucleotide insert contained a unique Ear I restriction site for subsequent insertion of oligonucleotides encoding one or more heptad repeats and added an unique Asp 718 restriction site for use in transfer of gene sequences to plant vectors. The overhanging ends of the oligonucleotide set allowed insertion into the unique Nco I and EcoR I sites of vector pSK5.

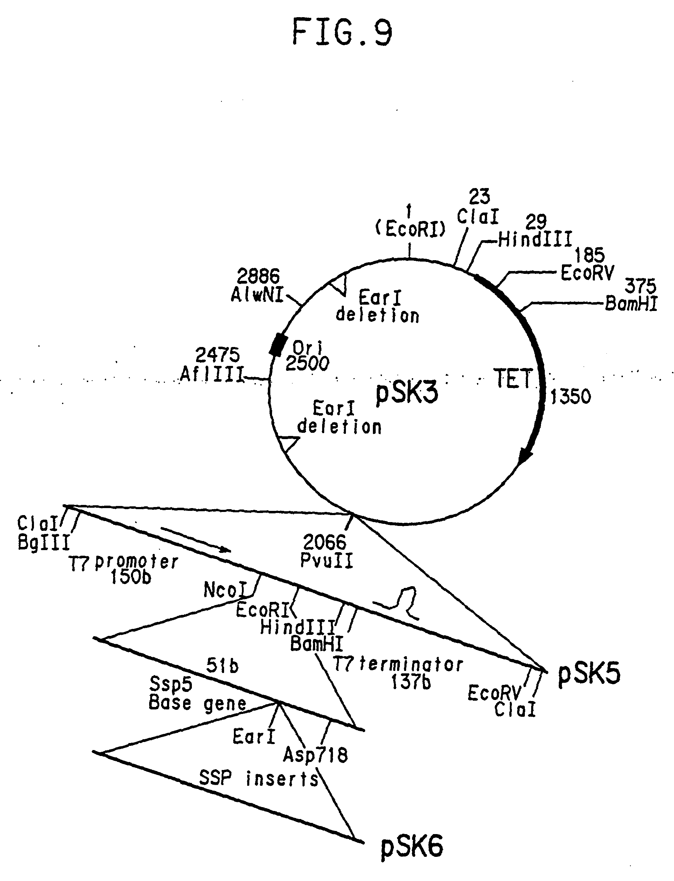

DNA from plasmid pSK5 was digested to completion with Nco I and EcoR I restriction endonucleases and purified by agarose gel electrophoresis. Purified DNA (0.1 ug) was mixed with 1 µg of each oligonucleotide SM80 (SEQ ID NO:14) and SM81 (SEQ ID NO:13) and ligated. The ligation mixture was transformed into E. coli strain JM103 [supE thi del (lac-proAB) F' [traD36 porAB, lacIq lacZ del M15] restriction minus] and tetracycline resistant transformants screened by rapid plasmid DNA preps followed by restriction digest analysis. A clone was chosen which had one each of Ear I, Nco I, Asp 718 and EcoR I sites indicating proper insertion of the oligonucleotides. This clone was designated pSK6 (Figure 9). Sequencing of the region of DNA following the T7 promoter confirmed insertion of oligonucleotides of the expected sequence.

Repetitive heptad coding sequences were added to the base gene construct of described above by generating oligonucleotide pairs which could be directly ligated into the unique Ear I site of the base gene. Oligonucleotides SM84 (SEQ ID NO:23) and SM85 (SEQ ID NO:24) code for repeats of the SSP5 heptad. Oligonucleotides SM82 (SEQ ID NO:25) and SM83 (SEQ ID NO:26) code for repeats of the SSP7 heptad.

Oligonucleotide sets were ligated and purified to obtain DNA fragments encoding multiple heptad repeats for insertion into the expression vector. Oligonucleotides from each set totalling about 2 µg were kinased, and ligated for 2 h at room temperature. The ligated multimers of the oligonucleotide sets were separated on an 18% non-denaturing 20 X 20 X 0.015 cm polyacrylamide gel (Acrylamide: bis-acrylamide = 19:1). Multimeric forms which separated on the gel as 168 bp (8n) or larger were purified by cutting a small piece of polyacrylamide containing the band into fine pieces, adding 1.0 mL of 0.5 M ammonium acetate, 1 mM EDTA (pH 7.5) and rotating the tube at 37° overnight. The polyacrylamide was spun down by centrifugation, 1 µg of tRNA was added to the supernatant,the DNA fragments were precipitated with 2 volumes of ethanol at -70°, washed with 70% ethanol, dried, and resuspended in 10 µL of water.

Ten micrograms of pSK6 DNA were digested to completion with Ear I enzyme and treated with calf intestinal alkaline phosphatase. The cut and phosphatased vector DNA was isolated following electrophoresis in a low melting point agarose gel by cutting out the banded DNA, liquifying the agarose at 55°, and purifying over NACS PREPAC^{™} columns (BRL) following manufacturer's suggested procedures. Approximately 0.1 µg of purified Ear I digested and phosphatase treated pSK6 DNA was mixed with 5 µL of the gel purified multimeric oligonucleotide sets and ligated. The ligated mixture was transformed into E. coli strain JM103 [supE thi del (lac-proAB) F' [traD36 porAB, lacIq lacZ del M15] restriction minus] and tetracycline-resistant colonies selected. Clones were screened by restriction digests of rapid plasmid prep DNA to determine the length of the inserted DNA. Restriction endonuclease analyses were usually carried out by digesting the plasmid DNAs with Asp 718 and Bgl II, followed by separation of fragments on 18% non-denaturing polyacrylamide gels. Visualization of fragments with ethidium bromide, showed that a 150 bp fragment was generated when only the base gene segment was present. Inserts of the oligonucleotide fragments increased this size by multiples of 21 bases. From this screening several clones were chosen for DNA sequence analysis and expression of coded sequences in E. coli. The first and last SSP5 heptads flanking the sequence of each construct are from the base gene described above. Inserts are designated by underlining (Table 4).

**Table 4**

| Clone # | SEQ ID NO: | Sequence by Heptad Amino Acid Repeat (SSP) | SEQ ID NO: |
|---|---|---|---|
| C15 | 29 | 5.7.7.7.7.7.5 | 30 |
| C20 | 31 | 5.7.7.7.7.7.5 | 32 |
| C30 | 33 | 5.7.7.7.7.5 | 34 |
| D16 | 35 | 5.5.5.5 | 36 |
| D20 | 37 | 5.5.5.5.5 | 38 |
| D33 | 39 | 5.5.5.5 | 40 |

Because the gel purification of the oligomeric forms of the oligonucleotides did not give the expected enrichment of longer (i.e., >8n) inserts, Applicants used a different procedure for a subsequent round of insertion constructions. For this series of constructs four more sets of oligonucleotides were generated which code for SSP 8,9,10 and 11 amino acid sequences respectively:

The following HPLC procedure was used to purify multimeric forms of the oligonucleotide sets after kinasing and ligating the oligonucleotides as described above. Chromatography was performed on a Hewlett Packard Liquid Chromatograph instrument, Model 1090M. Effluent absorbance was monitored at 260 nm. Ligated oligonucleotides were centrifuged at 12,000xg for 5 min and injected onto a 2.5 µ TSK DEAE-NPR ion exchange column (35 cm x 4.6 mm I.D.) fitted with a 0.5 µ in-line filter (Supelco). The oligonucleotides were separated on the basis of length using a gradient elution and a two buffer mobile phase [Buffer A: 25 mM Tris-Cl, pH 9.0, and Buffer B: Buffer A + 1 M NaCl]. Both Buffers A and B were passed through 0.2 µ filters before use. The following gradient program was used with a flow rate of 1 mL per min at 30°:

| Time | %A | %B |
|---|---|---|
| initial | 75 | 25 |
| 0.5 min | 55 | 45 |
| 5 min | 50 | 50 |
| 20 min | 38 | 62 |
| 23 min | 0 | 100 |
| 30 min | 0 | 100 |
| 31 min | 75 | 25 |

Fractions (500 µL) were collected between 3 min and 9 min. Fractions corresponding to lengths between 120 bp and 2000 bp were pooled as determined from control separations of restriction digests of plasmid DNAs.

The 4.5 mL of pooled fractions for each oligonucleotide set were precipitated by adding 10 µg of tRNA and 9.0 mL of ethanol, rinsed twice with 70% ethanol and resuspended in 50 µL of water. Ten microliters of the resuspended HPLC purified oligonucleotides were added to 0.1 µg of the Ear I cut, phosphatased pSK6 DNA described above and ligated overnight at 15°. All six oligonucleotide sets described above which had been kinased and self-ligated but not purified by gel or HPLC were also used in separate ligation reactions with the pSK6 vector. The ligation mixtures were transformed into E. coli strain DH5a [supE44 del lacU169 (phi 80 lacZ del M15) hsdR17 recA1 endA1 gyr196 thi1 relA1] and tetracycline-resistant colonies selected. Applicants chose to use the DH5α [supE44 del lacU169 (phi 80 lacZ del M15) hsdR17 recA1 endA1 gyr196 thi1 relA1] strain for all subsequent work because this strain has a very high transformation rate and is recA-. The recA-phenotype eliminates concerns that these repetitive DNA structures may be substrates for homologous recombination leading to deletion of multimeric sequences.

Clones were screened as described above. Several clones were chosen to represent insertions of each of the six oligonucleotide sets. The first and last SSP5 heptads flanking the sequence represent the base gene sequence. Insert sequences are underlined. Clone numbers including the letter "H" designate HPLC-purified oligonucleotides (Table 5).

**Table 5**

| Clone # | SEQ ID NO: | Sequence by Heptad Amino Acid Repeat (SSP) | SEQ ID NO: |
|---|---|---|---|
| 82-4 | 53 | 7.7.7.7.7.7.5 | 54 |
| 84-H3 | 55 | 5.5.5.5 | 56 |
| 86-H23 | 57 | 5.8.8.5 | 58 |
| 88-2 | 59 | 5.9.9.9.5 | 60 |
| 90-H8 | 61 | 5.10.10.10.5 | 62 |
| 92-2 | 63 | 5.11.11.5 | 64 |

The loss of the first base gene repeat in clone 82-4 may have resulted from homologous recombination between the base gene repeats 5.5 before the vector pSK6 was transferred to the recA- strain. The HPLC procedure did not enhance insertion of longer multimeric forms of the oligonucleotide sets into the base gene but did serve as an efficient purification of the ligated oligonucleotides.

Oligonucleotides were designed which coded for mixtures of the SSP sequences and which varied codon usage as much as possible. This was done to reduce the possibility of deletion of repetitive inserts by recombination once the synthetic genes were transformed into plants and to extend the length of the constructed gene segments. These oligonucleotides encode four repeats of heptad coding units (28 amino acid residues) and can be inserted at the unique Ear I site in any of the previously constructed clones. SM96 and SM97 code for SSP(5)₄, SM98 and SM99 code for SSP(7)₄ and SM100 plus SM101 code for SSP8.9.8.9.

DNA from clones 82-4 and 84-H3 were digested to completion with Ear I enzyme, treated with phosphatase and gel purified. About 0.2 µg of this DNA were mixed with 1.0 µg of each of the oligonucleotide sets SM96 and SM97, SM98 and SM99 or SM100 and SM101 which had been previously kinased. The DNA and oligonucleotides were ligated overnight and then the ligation mixes transformed into E. coli strain DH5α. Tetracycline-resistant colonies were screened as described above for the presence of the oligonucleotide inserts. Clones were chosen for sequence analysis based on their restriction endonuclease digestion patterns (Table 6).

**Table 6**

| Clone # | SEQ ID NO: | Sequence by Heptad Amino Acid Repeat (SSP) | SEQ ID NO: |
|---|---|---|---|
| 2-9 | 74 | 7.7.7.7.7.7.8.9.8.9.5 | 75 |
| 3-5 | 78 | 7.7.7.7.7.7.5.5 | 79 |
| 5-1 | 76 | 5.5.5.7.7.7.7.5 | 77 |
| Inserted oligonucleotide segments are underlined | | | |

Clone 2-9 was derived from oligonucleotides SM100 (SEQ ID NO:71) and SM101 (SEQ ID NO:72) ligated into the Ear I site of clone 82-4 (see above). Clone 3-5 (SEQ ID NO:78) was derived from the insertion of the first 22 bases of the oligonucleotide set SM96.(SEQ ID NO:65) and SM97 (SEQ ID NO:66) into the Ear I site of clone 82-4 (SEQ ID NO:53). This partial insertion may reflect improper annealing of these highly repetitive oligos. Clone 5-1 (SEQ ID NO:76) was derived from oligonucleotides SM98 (SEQ ID NO:68) and SM99 (SEQ ID NO:69) ligated into the Ear I site of clone 84-H3 (SEQ ID NO:55) (see section).

### Strategy II.

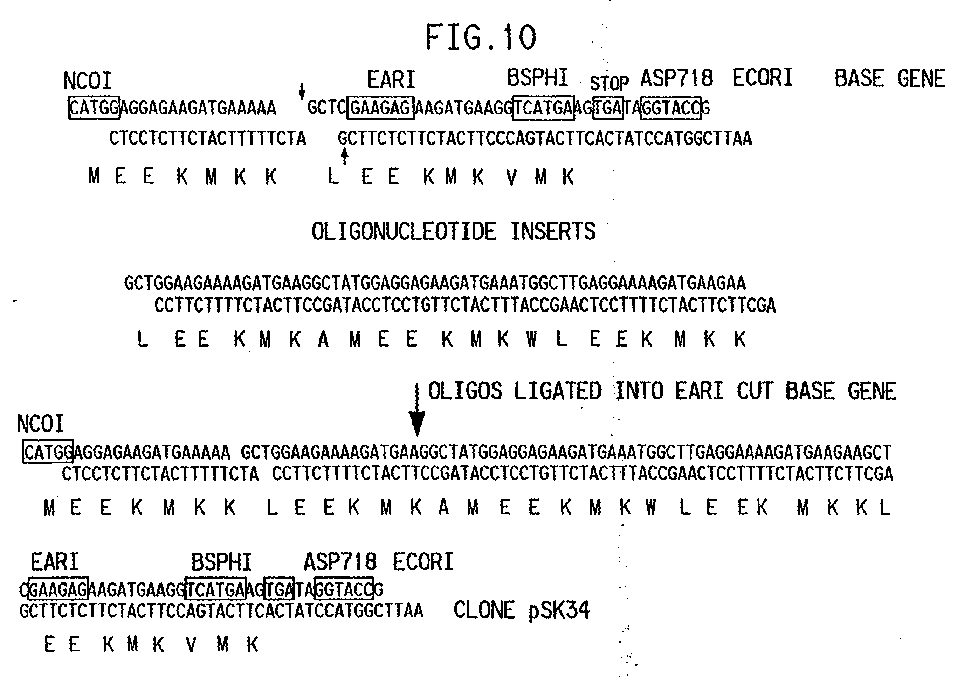

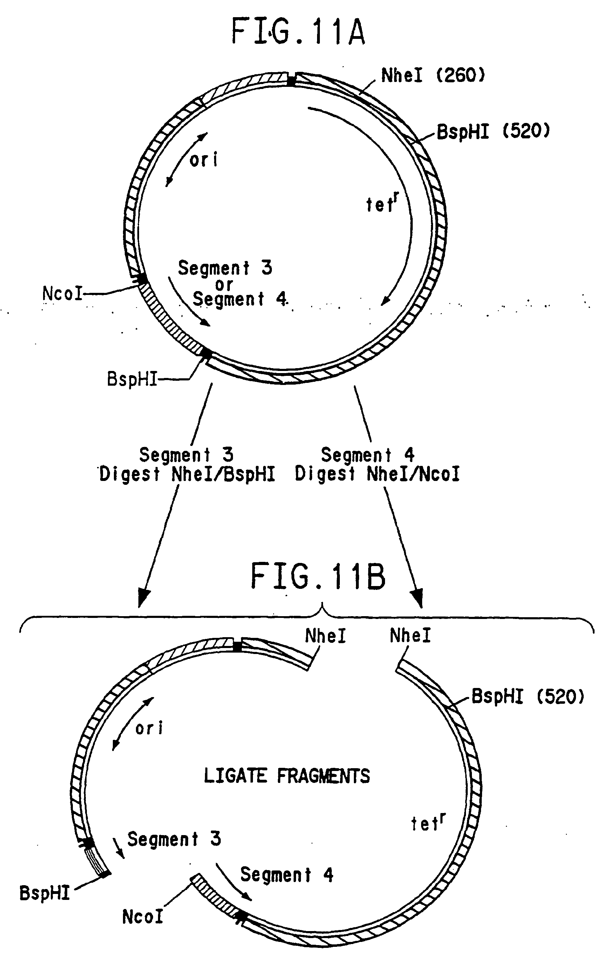

A second strategy for construction of synthetic gene sequences was implemented to allow more flexibility in both DNA and amino acid sequence. This strategy is depicted in Figure 10 and Figure 11. The first step was the insertion of an oligonucleotide sequence encoding a base gene of 16 amino acids into the original vector pSK5. This oligonucleotide insert contained an unique Ear I site as in the previous base gene construct for use in subsequent insertion of oligonucleotides encoding one or more heptad repeats. The base gene also included a BspH I site at the 3' terminus. The overhanging ends of this cleavage site are designed to allow "in frame" protein fusions using Nco I overhanging ends.
Therefore, gene segments can be multiplied using the duplication scheme described in Figure 11. The overhanging ends of the oligonucleotide set allowed insertion into the unique Nco I and EcoR I sites of vector pSK5. The oligonucleotide set was inserted into pSK5 vector as described in Strategy I above. The resultant plasmid was designated pSK34.

Oligonucleotide sets encoding 35 amino acid "segments" were ligated into the unique Ear I site of the pSK34 base gene using procedures as described above. In this case, the oligonucleotides were not gel or HPLC purified but simply annealed and used in the ligation reactions. The following oligonucleotide sets were used: Clones were screened for the presence of the inserted segments by restriction digestion followed by separation of fragments on 6% acrylamide gels. Correct insertion of oligonucleotides was confirmed by DNA sequence analyses. Clones containing segments 3, 4 and 5 respectively were designated pSKseg3, pSKseg4, and pSKseg5.

These "segment" clones were used in a duplication scheme as shown in Figure 11. Ten µg of plasmid pSKseg3 were digested to completion with Nhe I and BspH I and the 1503 bp fragment isolated from an agarose gel using the Whatmann paper technique. Ten µg of plasmid pSKseg4 were digested to completion with Nhe I and Nco I and the 2109 bp band gel isolated. Equal amounts of these fragments were ligated and recombinants selected on tetracycline. Clones were screened by restriction digestions and their sequences confirmed. The resultant plasmid was designated pSKseg34.

pSKseg34 and pSKseg5 plasmid DNAs were digested, fragments isolated and ligated in a similar manner as above to create a plasmid containing DNA sequences encoding segment 5 fused to segments 3 and 4. This construct was designated pSKseg534 and encodes the following amino acid sequence:

### EXAMPLE 9

### Construction of SSP Chimeric Genes for Expression in the Seeds of Plants

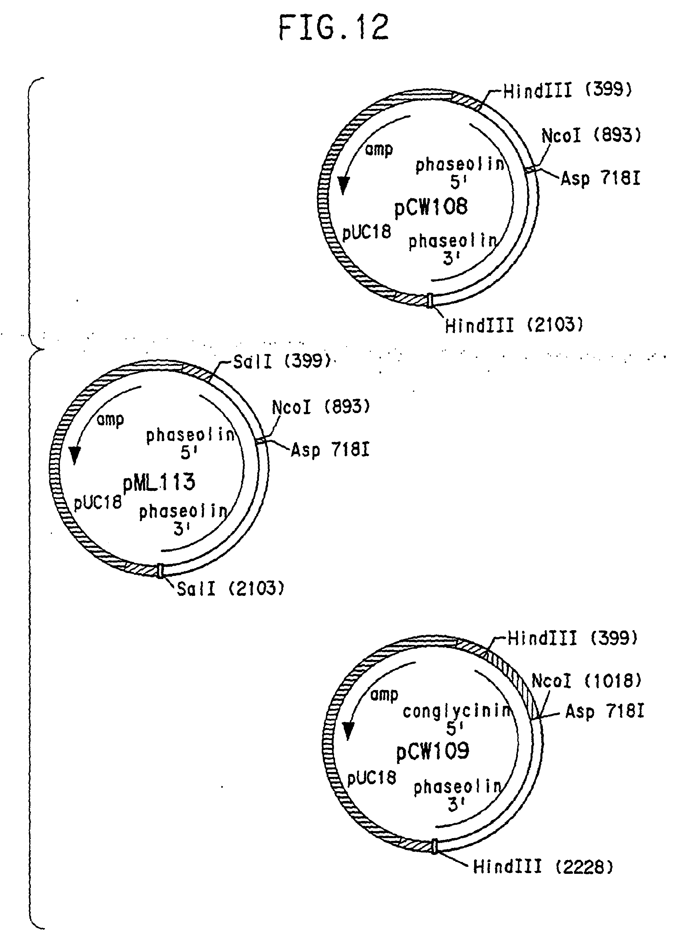

To express the synthetic gene products described in Example 8 in plant seeds, the sequences were transferred to the seed promoter vectors CW108, CW109 or ML113 (Figure 12). The vectors CW108 and ML113 contain the bean phaseolin promoter (from base +1 to base -494),and 1191 bases of the 3' sequences from bean phaseolin gene. CW109 contains the soybean β-conglycinin promoter (from base +1 to base -619) and the same 1191 bases of 3' sequences from the bean phaseolin gene. These vectors were designed to allow direct cloning of coding sequences into unique Nco I and Asp 718 sites. These vectors also provide sites (Hind III or Sal I) at the 5' and 3' ends to allow transfer of the promoter/coding region/ 3' sequences directly to appropriate binary vectors.

To insert the synthetic storage protein gene sequences, 10 µg of vector DNA were digested to completion with Asp 718 and Nco I restriction endonucleases. The linearized vector was purified via electrophoresis on a 1.0% agarose gel overnight electrophoresis at 15 volts. The fragment was collected by cutting the agarose in front of the band, inserting a 10 X 5 mm piece of Whatman 3MM paper into the agarose and electrophoresing the fragment into the paper [Errington, (1990) Nucleic Acids Research, 18:17]. The fragment and buffer were spun out of the paper by centrifugation and the DNA in the -100 µL was precipitated by adding 10 mg of tRNA, 10 µL of 3 M sodium acetate and 200 µL of ethanol. The precipitated DNA was washed twice with 70% ethanol and dried under vacuum. The fragment DNA was resuspended in 20 µL of water and a portion diluted 10-fold for use in ligation reactions.

Plasmid DNA (10 mg) from clones 3-5 and pSK534 was digested to completion with Asp 718 and Nco I restriction endonucleases. The digestion products were separated on an 18% polyacrylamide non-denaturing gel as described in Example 8. Gel slices containing the desired fragments were cut from the gel and purified by inserting the gel slices into a 1% agarose gel and electrophoresing for 20 min at 100 volts. DNA fragments were collected on 10 X 5 mm pieces of Whatman 3MM paper, the buffer and fragments spun out by centrifugation and the DNA precipitated with ethanol. The fragments were resuspended in 6 µL water. One microliter of the diluted vector fragment described above, 2 µL of 5X ligation buffer and 1 µL of T4 DNA ligase were added. The mixture was ligated overnight at 15°.

The ligation mixes were transformed into E. coli strain DH5a [supE44 del lacU169 (phi 80 lacZ del M15) hsdR17 recA1 endA1 gyr196 thi1 relA1] and ampicillin-resistant colonies selected. The clones were screened by restriction endonuclease digestion analyses of rapid plasmid DNAs and by DNA sequencing.

### EXAMPLE 10

### Tobacco Plants Containing the Chimeric Genes Phaseolin Promoter/cts/ecodapA, Phaseolin Promoter/cts/lysC-M4 and β-conglycinin promoter/SSP3-5

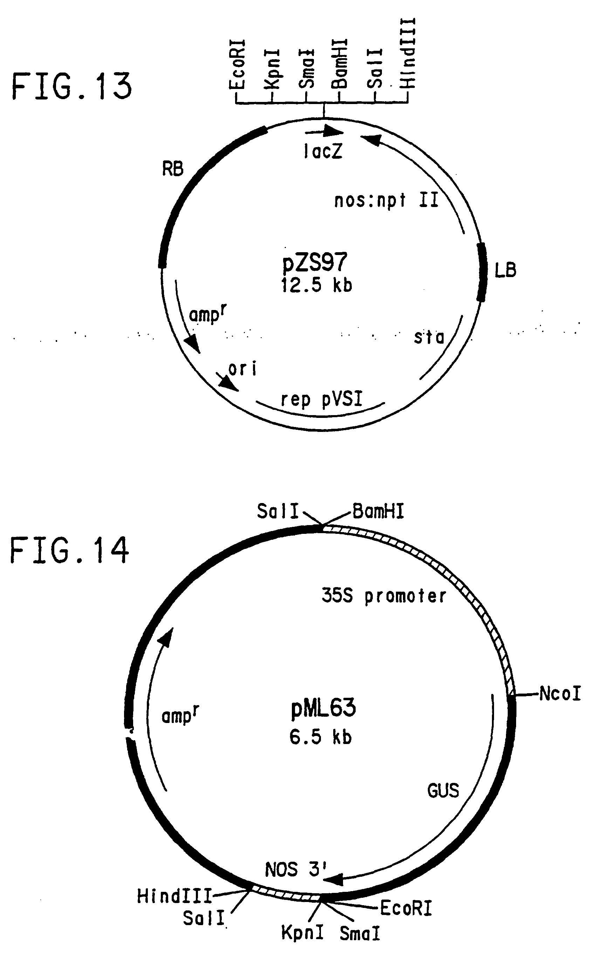

The binary vector pZS97 was used to transfer the chimeric SSP3-5 gene of Example 9 and the chimeric E. coli dapA and lysC-M4 genes of Example 4 to tobacco plants. Binary vector pZS97 (Figure 13) is part of a binary Ti plasmid vector system [Bevan, (1984) Nucl. Acids. Res. 12:8711-8720] of Agrobacterium tumefaciens. The vector contains: (1) the chimeric gene nopaline synthase::neomycin phosphotransferase (nos::NPTII) as a selectable marker for transformed plant cells [Bevan et al., (1983) Nature 304:184-186], (2) the left and right borders of the T-DNA of the Ti plasmid [Bevan, (1984) Nucl. Acids. Res. 12:8711-8720], (3) the E. coli lacZ a-complementing segment [Viering et al., (1982) Gene 19:259-267] with a unique Sal I site(pSK97K) or unique Hind III site (pZS97) in the polylinker region, (4) the bacterial replication origin from the Pseudomonas plasmid pVS1 [Itoh et al., (1984) Plasmid 11:206-220], and (5) the bacterial β-lactamase gene as a selectable marker for transformed A. tumefaciens.

Plasmid pZS97 DNA was digested to completion with Hind III enzyme and the digested plasmid was gel purified. The Hind III digested pZS97 DNA was mixed with the Hind III digested and gel isolated chimeric gene fragments, ligated, transformed as above and colonies selected on ampicillin.

Binary vectors containing the chimeric genes were transferred by tri-parental matings [Ruvkin et al., (1981) Nature 289:85-88] to Agrobacterium strain LBA4404/pAL4404 [Hockema et al., (1983), Nature 303:179-180] selecting for carbenicillin resistance. Cultures of Agrobacterium containing the binary vector were used to transform tobacco leaf disks [Horsch et al., (1985) Science 227:1229-1231]. Transgenic plants were regenerated in selective medium containing kanamycin.

Transformed tobacco plants containing the chimeric gene, β-conglycinin promoter/SSP3-5/phaseolin 3' region, were thus obtained. Two transformed lines, pSK44-3A and pSK44-9A, which carried a single site insertion of the SSP3-5 gene were identified based upon 3:1 segregation of the marker gene for kanamycin resistance. Progeny of the primary transformants, which were homozygous for the transgene, pSK44-3A-6 and pSK44-9A-5, were then identified based upon 4:0 segregation of the kanamycin resistance in seeds of these plants.

Similarly, transformed tobacco plants with the chimeric genes phaseolin 5' region/cts/lysC-M4/phaseolin 3' region and phaseolin 5' region/cts/ecodapA/phaseolin 3' region were obtained. A transformed line, BT570-45A, which carried a single site insertion of the DHDPS and AK genes was identified based upon 3:1 segregation of the marker gene for kanamycin resistance. Progeny from the primary transformant which were homozygous for the transgene, BT570-45A-3 and BT570-45A-4, were then identified based upon 4:0 segregation of the kanamycin resistance in seeds of these plants.

To generate plants carrying all three chimeric genes genetic crosses were performed using the homozygous parents. Plants were grown to maturity in greenhouse conditions. Flowers to be used as male and female were selected one day before opening and older flowers on the inflorescence removed. For crossing, female flowers were chosen at the point just before opening when the anthers were not dehiscent. The corolla was opened on one side and the anthers removed. Male flowers were chosen as flowers which had opened on the same day and had dehiscent anthers shedding mature pollen. The anthers were removed and used to pollinate the pistils of the anther-stripped female flowers. The pistils were then covered with plastic tubing to prevent further pollination. The seed pods were allowed to develop and dry for 4-6 weeks and harvested. Two to three separate pods were recovered from each cross. The following crosses were performed:

| Male | X | Female |
|---|---|---|
| BT570-45A-3 | | pSK44-3A-6 |
| BT570-45A-4 | | pSK44-3A-6 |
| pSK44-3A-6 | | BT570-45A-4 |
| BT570-45A-5 | | pSK44-9A-5 |
| pSk44-9A-5 | | BT570-45A-5 |

Dried seed pods were broken open and seeds collected and pooled from each cross. Thirty seeds were counted out for each cross and for controls seeds from selfed flowers of each parent were used. Duplicate seed samples were hydrolyzed and assayed for total amino acid content as described in Example 5. The amount of increase in lysine as a percent of total seeds amino acids over wild type seeds, which contain 2.56% lysine, is presented in Table 7 along the copy number of each gene in the endosperm of the seed.

**TABLE 7**

| male | X female | endosperm copy number AK & DHDPS genes | endosperm copy number SSP gene | lysine increase |
|---|---|---|---|---|
| BT570-45A | X BT570-45A | 1.5* | 0 | 0 |
| pSK44-9A | X pSK44-9A | 0 | 1.5* | 0.12 |
| pSK44-9A-5 | X pSK44-9A-5 | 0 | 3.0 | 0.29 |
| pSK44-9A-5 | X BT570-45A-5 | 2 | 1 | 0.6 |
| BT570-45A-5 | X pSK44-9A-5 | 1 | 2 | 0.29 |
| pSK44-3A | X pSK44-3A | 0 | 1.5* | 0.28 |
| pSK44-3A-6 | X pSK44-3A-6 | 0 | 3.0 | 0.5 |
| pSK44-3A-6 | X BT570-45A-4 | 2 | 1 | 0.62 |
| BT570-45A-3 | X pSK44-3A-6 | 1 | 2 | 0.27 |
| BT570-45A-4 | X pSK44-3A-6 | 1 | 2 | 0.29 |

| | | | | |
|---|---|---|---|---|
| * copy number is average in population of seeds | | | | |

The results of these crosses demonstrate that the total lysine levels in seeds can be increased 10-25 percent by the coordinate expression of the lysine biosynthesis genes and the high lysine protein SSP3-5. In seeds derived from hybrid plants, this synergism is strongest when the biosynthesis genes are derived from the female parent, possibly due to gene dosage in the endosperm. It is expected that the lysine level would be further increased if the biosynthesis genes and the lysine-rich protein genes were all homozygous.

### EXAMPLE 11

### Soybean Plants Containing the Chimeric Genes Phaseolin Promoter/cts/cordapA and Phaseolin Promoter/SSP3-5

Transformed soybean plants that express the chimeric gene, phaseolin promoter/cts/cordapA/ phaseolin 3' region have been described in Example 6. Transformed soybean plants that express the chimeric gene, phaseolin promoter/SSP3-5/phaseolin 3' region, were obtained by inserting the chimeric gene as an isolated Hind III fragment into an equivalent soybean transformation vector plasmid pML63 (Figure 14 Example 6) and carrying out transformation as described in Example 6.

Seeds from primary transformants were sampled by cutting small chips from the sides of the seeds away from the embryonic axis. The chips were assayed for GUS activity as described in Example 6 to determine which of the segregating seeds carried the transgenes. Half seeds were ground to meal and assayed for expression of SSP3-5 protein by Enzyme Linked ImmunoSorbent Assay (ELISA). Elisa was performed as follows:

A fusion protein of glutathione-S-transferase and the SSP3-5 gene product was generated through the use of the Pharmacia^{™} pGEX GST Gene Fusion System (Current Protocols in Molecular Biology, Vol. 2, pp 16.7.1-8, (1989) John Wiley and Sons). The fusion protein was purified by affinity chromatography on glutathione agarose (Sigma) or glutathione sepharose (Pharmacia) beads, concentrated using Centricon 10^{™} (Amicon) filters, and then subjected to SDS polyacrylamide electrophoresis (15% Acrylamide, 19:1 Acrylamide:Bis-acrylamide) for further purification. The gel was stained with Coomassie Blue for 30 min, destained in 50% Methanol, 10% Acetic Acid and the protein bands electroeluted using an Amicon^{™} Centiluter Microelectroeluter (Paul T. Matsudaira ed., A Practical Guide to Protein and Peptide Purification for Microsequencing, Academic Press, Inc. New York, 1989). A second gel prepared and run in the same manner was stained in a non acetic acid containing stain [9 parts 0.1% Coomassie Blue G250 (Bio-Rad) in 50% methanol and 1 part Serva Blue (Serva, Westbury, NY) in distilled water] for 1-2 h. The gel was briefly destained in 20% methanol, 3% glycerol for 0.5-1 h until the GST-SSP3-5 band was just barely visible. This band was excised from the gel and sent with the electroeluted material to Hazelton Laboratories for use as an antigen in immunizing a New Zealand Rabbit. A total of 1 mg of antigen was used (0.8 mg in gel, 0.2 mg in solution). Test bleeds were provided by Hazelton Laboratories every three weeks. The approximate titer was tested by western blotting of E. coli extracts from cells containing the SSP-3-5 gene under the control of the T7 promoter at different dilutions of protein and of serum.

IgG was isolated from the serum using a Protein A sepharose column. The IgG was coated onto microtiter plates at 5 µg per well. A separate portion of the IgG was biotinylated.

Aqueous extracts from transgenic plants were diluted and loaded into the wells usually starting with a sample containing 1 µg of total protein. The sample was diluted several more times to insure that at least one of the dilutions gave a result that was within the range of a standard curve generated on the same plate. The standard curve was generated using chemically synthesized SSP3-5 protein. The samples were incubated for one hour at 37° and the plates washed. The biotinylated IgG was then added to the wells. The plate was incubated at 37° for 1 hr and washed. Alkaline phosphatase conjugated to streptavidin was added to the wells, incubated at 37° for 1 hr and washed. A substrate consisting of 1 mg/ml p nitrophenylphosphate in 1M diethanolamine was added to the wells and the plates incubated at 37° for 1 hr. A 5% EDTA stop solution was added to the wells and the absorbance read at 405 nm minus 650 nm reading. Transgenic soybean seeds contained 0.5 to 2.0% of water extractable protein as SSP3-5.

The remaining half seeds positive for GUS and SSP3-5 protein were planted and grown to maturity in greenhouse conditions. To determine homozygotes for the GUS phenotype, seed from these R1 plants were screened for segregation of GUS activity as above. Plants homozygous for the phaseolin/SSP3-5 gene were crossed with homozygous transgenic soybeans expressing the Corynebacterium dapA gene product.

As an preferred alternative to bringing the chimeric SSP gene and chimeric cordapA geneA together via genetic crossing a single soybean tranformation vector carrying both genes was constructed. Plasmid pML63 carrying the chimeric gene phaseolin promoter/SSP3-5/phaseolin 3' region described above was cleaved with restriction enzyme BamH I and the BamH I fragment carrying the chimeric gene phaseolin promoter/cts/cordapA/ phaseolin 3' region (Example 5) was inserted. This vector can be transformed into soybean as described in Example 6.

### EXAMPLE 12

### Construction of Chimeric Genes for Expression of Corynebacterium DHDRS and SSP3-5 in the Embryo and Endosperm of Transformed Corn

The following chimeric genes were made for transformation into corn:
globulin 1 promoter/mcts/cordapA/NOS 3 region
glutelin 2 promoter/mcts/cordapA/NOS 3' region
globulin 1 promoter/SSP3-5/globulin 1 3' region
glutelin 2 promoter/SSP3-5/10 kD 3' region

The glutelin 2 promoter was cloned from corn genomic DNA using PCR with primers based on the published sequence [Reina et al. (1990) Nucleic Acids Res. 18:6426-6426]. The promoter fragment includes 1020 nucleotides upstream from the ATG translation start codon. An Nco I site was introduced via PCR at the ATG start site to allow for direct translational fusions. A BamH I site was introduced on the 5' end of the promoter. The 1.02 kb BamH I to Nco I promoter fragment was cloned into the BamH I to Nco I sites of the plant expression vector pML63 (see Example 11) replacing the 35S promoter to create vector pML90. This vector contains the glutelin 2 promoter linked to the GUS coding region and the NOS 3'.

The 10 kD zein 3' region was derived from a 10 kD zein gene clone generated by PCR from genomic DNA using oligonucleotide primers based on the published sequence [Kirihara et al. (1988) Gene 71:359-370]. The 3' region extends 940 nucleotides from the stop codon. Restriction endonuclease sites for Kpn I, Sma I and Xba I sites were added immediately following the TAG stop codon by oligonucleotide insertion to facilitate cloning. A Sma I to Hind III segment containing the 10 kD 3'region was isolated and ligated into Sma I and Hind III digested pML90 to replace the NOS 3' sequence with the 10 kD 3'region, thus creating plasmid pML103. pML103 contains the glutelin 2 promoter, an Nco I site at the ATG start codon of the GUS gene, Sma I and Xba I sites after the stop codon, and 940 nucleotides of the 10 kD zein 3' sequence.

The globulin 1 promoter and 3' sequences were isolated from a Clontech corn genomic DNA library using oligonucleotide probes based on the published sequence of the globulin 1 gene [Kriz et al. (1989) Plant Physiol. 91:636]. The cloned segment includes the promoter fragment extending 1078 nucleotides upstream from the ATG translation start codon, the entire globulin coding sequence including introns and the 3' sequence extending 803 bases from the translational stop. To allow replacement of the globulin 1 coding sequence with other coding sequences an Nco I site was introduced at the ATG start codon, and Kpn I and Xba I sites were introduced following the translational stop codon via PCR to create vector pCC50. There is a second Nco I site within the globulin 1 promoter fragment. The globulin 1 gene cassette is flanked by Hind III sites.

The plant amino acid biosynthetic enzymes are known to be localized in the chloroplasts and therefore are synthesized with a chloroplast targeting signal. Bacterial proteins such as DHDPS have no such signal. A chloroplast transit sequence (cts) was therefore fused to the cordapA coding sequence in the chimeric genes described below. For corn the cts used was based on the the cts of the small subunit of ribulose 1,5-bisphosphate carboxylase from corn [Lebrun et al. (1987) Nucleic Acids Res. 15:4360] and is designated acts to distinguish it from the soybean cts. The oligonucleotides SEQ ID NOS:93-98 were synthesized and used essentially as described in Example 4.

Oligonucleotides SEQ ID NO:93 and SEQ ID NO:94, which encode the carboxy terminal part of the corn chloroplast targeting signal, were annealed, resulting in Xba I and Nco I compatible ends, purified via polyacrylamide gel electrophoresis, and inserted into Xba I plus Nco I digested pBT492 (see Example 3). The insertion of the correct sequence was verified by DNA sequencing yielding pBT556. Oligonucleotides SEQ ID NO:95 and SEQ ID NO:96, which encode the middle part of the chloroplast targeting signal, were annealed, resulting in Bgl II and Xba I compatible ends, purified via polyacrylamide gel electrophoresis, and inserted into Bgl II and Xba I digested pBT556. The insertion of the correct sequence was verified by DNA sequencing yielding pBT557. Oligonucleotides SEQ ID NO:97 and SEQ ID NO:98, which encode the amino terminal part of the chloroplast targeting signal, were annealed, resulting in Nco I and Afl II compatible ends, purified via polyacrylamide gel electrophoresis, and inserted into Nco I and Afl II digested pBT557. The insertion of the correct sequence was verified by DNA sequencing yielding pBT558. Thus the mcts was fused to the lysC-M4 gene.

A DNA fragment containing the entire mcts was prepared using PCR. The template DNA was pBT558 and the oligonucleotide primers used were:
SEQ ID NO:99:
   GCGCCCACCG TGATGA
SEQ ID NO:100:
   CACCGGATTC TTCCGC

The mcts fragment was linked to the amino terminus of the DHDPS protein encoded by ecodapA gene by digesting with Nco I and treating with the Klenow fragment of DNA polymerase to fill in the 5' overhangs. The inserted fragment and the vector/insert junctions were determined to be correct by DNA sequencing, yielding pBT576.

To construct the chimeric gene:
globulin 1 promoter/mcts/cordapA/NOS 3 region an Nco I to Kpn I fragment containing the mcts/ecodapA coding sequence was isolated from plasmid pBT576 (see Example 6) and inserted into Nco I plus Kpn I digested pCC50 creating plasmid pBT662. Then the ecodapA coding sequence was replaced with the cordapA coding sequence as follows. An Afl II to Kpn I fragment containing the distal two thirds of the mcts fused to the cordapA coding sequence was inserted into Afl II to Kpn I digested pBT662 creating plasmid pBT677.

To construct the chimeric gene:
glutelin 2 promoter/mcts/cordapA/NOS 3' region an Nco I to Kpn I fragment containing the mcts/cordapA coding sequence was isolated from plasmid pBT677 and inserted into Nco I to Kpn I digested pML90, creating plasmid pBT679.

To construct the chimeric gene:
glutelin 2 promoter/SSP3-5/10 kD 3' region the plasmid pML103 (above) containing the glutelin 2 promoter and 10 kD zein 3' region was cleaved at the Nco I and Sma I sites. The SSP3-5 coding region (Example 9) was isolated as an Nco I to blunt end fragment by cleaving with Xba I followed by filling in the sticky end using Klenow fragment of DNA polymerase, then cleaving with Nco I. The 193 base pair Nco I to blunt end fragment was ligated into the Nco I and Sma I cut pML103 to create pLH104.

To construct the chimeric gene:
globulin 1 promoter/SSP3-5/globulin 1 3'region the 193 base pair Nco I and Xba I fragment containing the SSP3-5 coding region (Example 9) was inserted into plasmid pCC50 (above) which had been cleaved with Xba I to completion and then partially cut with Nco I to open the plasmid at the ATG start codon creating pLH105.

### EXAMPLE 13

### Corn Plants Containing Chimeric Genes for Expression of Corynebacterium DHDPS in the Embryo and Endosperm

Corn was transformed with the chimeric genes:
globulin 1 promoter/mcts/cordapA/NOS 3 region or
glutelin 2 promoter/mcts/cordapA/NOS 3' region Either one of two plasmid vectors containing selectable markers were used in the transformations. One plasmid, pDETRIC, contained the bar gene from Streptomyces hygroscopicus that confers resistance to the herbicide glufosinate [Thompson et al. (1987 The EMBO Journal 6:2519-2523]. The bacterial gene had its translation codon changed from GTG to ATG for proper translation initiation in plants [De Block et al. (1987) The EMBO Journal 6:2513-2518]. The bar gene was driven by the 35S promoter from Cauliflower Mosaic Virus and uses the termination and polyadenylation signal from the octopine synthase gene from Agrobacterium tumefaciens. Alternatively, the selectable marker used was 35S/Ac, a synthetic phosphinothricin-N-acetyltransferase (pat) gene under the control of the 35S promoter and 3' terminator/polyadenylation signal from Cauliflower Mosiac Virus [Eckes et.al., (1989) J Cell Biochem Suppl 13 D].

Embryogenic callus cultures were initiated from immature embryos (about 1.0 to 1.5 mm) dissected from kernels of a corn line bred for giving a "type II callus" tissue culture response. The embryos were dissected 10 to 12 d after pollination and were placed with the axis-side down and in contact with agarose-solidified N6 medium [Chu et al. (1974) Sci Sin 18:659-668] supplemented with 0.5 mg/L 2,4-D (N6-0.5). The embryos were kept in the dark at 27°C. Friable embryogenic callus consisting of undifferentiated masses of cells with somatic proembryos and somatic embryos borne on suspensor structures proliferated from the scutellum of the immature embryos. Clonal embryogenic calli isolated from individual embryos were identified and sub-cultured on N6-0.5 medium every 2 to 3 weeks.

The particle bombardment method was used to transfer genes to the callus culture cells. A Biolistic, PDS-1000/He (BioRAD Laboratories, Hercules, CA) was used for these experiments.

Circular plasmid DNA or DNA which had been linearized by restriction endonuclease digestion was precipitated onto the surface of gold particles. DNA from two or three different plasmids, one containing the selectable marker for corn transformation, and one or two containing the chimeric genes for increased lysine accumulation in seeds were co-precipitated. To accomplish this 1.5 µg of each DNA (in water at a concentration of about 1 mg/mL) was added to 25 mL of gold particles (average diameter of 1.5 µm) suspended in water (60 mg of gold per mL). Calcium chloride (25 mL of a 2.5 M solution) and spermidine (10 mL of a 1.0 M solution) were then added to the gold-DNA suspension as the tube was vortexing. The gold particles were centrifuged in a microfuge for 10 sec and the supernatant removed. The gold particles were then resuspended in 200 mL of absolute ethanol, were centrifuged again and the supernatant removed. Finally, the gold particles were resuspended in 25 mL of absolute ethanol and sonicated twice for one sec. Five µL of the DNA-coated gold particles were then loaded on each macro carrier disk and the ethanol was allowed to evaporate away leaving the DNA-covered gold particles dried onto the disk.

Embryogenic callus (from the callus line designated #LH132.5.X) was arranged in a circular area of about 6 cm in diameter in the center of a 100 X 20 mm petri dish containing N6-0.5 medium supplemented with 0.25M sorbitol and 0.25M mannitol. The tissue was placed on this medium for 2 h prior to bombardment as a pretreatment and remained on the medium during the bombardment procedure. At the end of the 2 h pretreatment period, the petri dish containing the tissue was placed in the chamber of the PDS-1000/He. The air in the chamber was then evacuated to a vacuum of 28 inch of Hg. The macrocarrier was accelerated with a helium shock wave using a rupture membrane that bursts when the He pressure in the shock tube reaches 1100 psi. The tissue was placed approximately 8 cm from the stopping screen. Four plates of tissue were bombarded with the DNA-coated gold particles. Immediately following bombardment, the callus tissue was transferred to N6-0.5 medium without supplemental sorbitol or mannitol.

Within 24 h after bombardment the tissue was transferred to selective medium, N6-0.5 medium that contained 2 mg/L glufosinate and lacked casein or proline. Tissue that continued to grow slowly on this medium was transferred to fresh N6-0.5 medium supplemented with glufosinate every 2 weeks. After 6-12 weeks clones of actively growing callus were identified. Callus was then transferred to medium that promotes plant regeneration.

Plants regenerated from transformed callus were analyzed for the presence of the intact transgenes via Southern blot or PCR. The plants were selfed or outcrossed to an elite line to generate R1 or F1 seeds, respectively. Single R1 seeds or six to eight F1 seeds were pooled and assayed for expression of the Corynebacterium DHDPS protein by western blot analysis. The free amino acid composition and total amino acid composition of the seeds were determined as described in previous examples.

Expression of the Corynebacterium DHDPS protein, driven by either the globulin or glutelin promoter, was observed in the corn seeds (Table 8). Free lysine levels in the seeds increased from about 1.4% of free amino acids in control seeds to 15-27% in seeds expressing Corynebacterium DHDPS from the globulin 1 promoter. The higher DHDPS expression and higher lysine level in the selfed seed probably results from the fact that half of the pooled seeds in the outcrossed lines are expected to lack the transgene due to segregation. A smaller increase in free lysine was observed in in seeds expressing Corynebacterium DHDPS from the glutelin 2 promoter. Thus to increase lysine, it may be better to express this enzyme in the embryo rather than the endosperm. A high level of saccharopine, indicative of lysine catabolism, was observed in seeds the contained high levels of lysine.

Lysine normally represents about 2.3% of the seed amino acid content. It is therefore apparent from Table 8 that substantial increases (35%-130%) in lysine as a percent of total seed amino acids was found in seeds expressing Corynebacterium DHDPS from the globulin 1 promoter.

**TABLE 8 1088.1.2 line: globulin 1 promoter/mcts/cordapA/NOS 3 region 1099.2.1 line: globulin 1 promoter/mcts/cordapA/NOS 3 region 1090.2.1 line: glutelin 2 promoter/mcts/cordapA/NOS 3' region**

| TRANSGENIC LINE | WESTERN CORYNE. DHDPS | % LYS of FREE SEED AMINO ACIDS | % LYS of TOTAL SEED AMINO ACIDS |
|---|---|---|---|
| 1088.1.2 x elite | + | 15 | 3.1 |
| 1099.2.1 selfed | ++ | 27 | 5.3 |
| 1090.2.1 x elite | + | 2.3 | 1.7 |

### EXAMPLE 14

### Transformation of Soybean with the Kunitz trypsin inhihitor 3 promoter/cts/cordapA Chimeric Gene

A seed-specific expression cassette composed of the promoter and transcription terminator from the the soybean Kunitz trypsin inhibitor 3 (KTI3) gene [Jofuku et al. (1989) Plant Cell 1:427-435] was created. The KTI3 cassette includes about 2000 nucleotides upstream (5') from the translation initiation codon and about 200 nucleotides downstream (3') from the translation stop codon of Kunitz trypsin inhibitor 3. Between the 5' and 3' regions restriction endonuclease sites Nco I (which includes the ATG translation initiation codon) and Kpn I were created to permit insertion of the Corynebacterium dapA gene. The entire cassette was flanked by BamH I and Sal I sites.

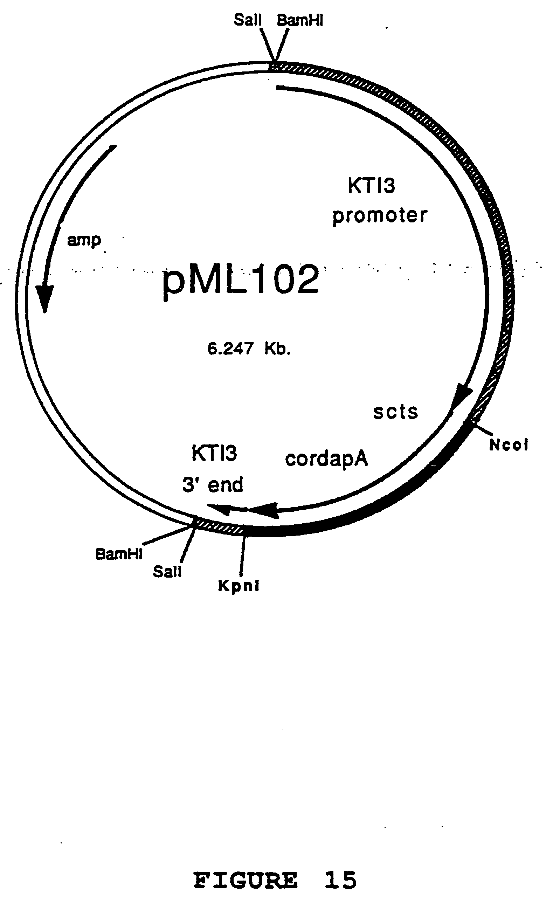

As described in Example 4 a chloroplast transit sequence (cts) was fused to the dapA coding sequence in the chimeric gene. The cts used was based on the the cts of the small subunit of ribulose 1,5-bisphosphate carboxylase from soybean [Berry-Lowe et al. (1982) J. Mol. Appl. Genet. 1:483-498]. A 1030 bp Nco I-Kpn I fragment containing the cts attached to the cordapA coding region was isolated from an agarose gel following electrophoresis and inserted into the KTI3 expression cassette yielding plasmid pML102 (Figure 15).

Plasmid pML102 was introduced into soybean by particle-mediated bombardment by Agracetus Company (Middleton, WI), according to the procedure described in United States Patent No. 5,015,580. To screen for transformed cells, plasmid pML102 was co-bombarded with another plasmid carrying a soybean transformation marker gene consisting of the 35S promoter from Cauliflower Mosaic Virus driving expression of the E. coli β-glucuronidase (GUS) gene [Jefferson et al. (1986) Proc. Natl. Acad. Sci. USA 83:8447-8451] with the Nos 3' region.

It was expected that the transgenes would be segregating in the R1 seeds of the transformed plants. To identify seeds that carried the transformation marker gene, a small chip of the seed was cut off with a razor and put into a well in a disposable plastic microtiter plate. A GUS assay mix consisting of 100 mM NaH₂PO₄, 10 mM EDTA, 0.5 mM K₄Fe(CN)₆, 0.1% Triton X-100, 0.5 mg/mL 5-Bromo-4-chloro-3-indolyl β-D-glucuronic acid was prepared and 0.15 mL was added to each microtiter well. The microtiter plate was incubated at 37° for 45 minutes. The development of blue color indicated the expression of GUS in the seed.

To measure the total amino acid composition of mature seeds, 1-1.4 milligrams of the seed meal was hydrolyzed in 6N hydrochloric acid, 0.4% β-mercaptoethanol under nitrogen for 24 h at 110-120°C; 1/50 of the sample was run on a Beckman Model 6300 amino acid analyzer using post-column ninhydrin detection. Lysine (and other amino acid) levels in the seeds were compared as percentages of the total amino acids. Wild type soybean seeds contain 5.7-6.0% lysine.

One hundred fifty individual seeds from sixteen independent transformed lines were analyzed (Table 9). Ten of the sixteen lines had seeds with a lysine content of 7% of the total seed amino acids or greater, a 16-22% increase over wild type seeds. Thus, more than 62% of the transformation events had co-integrated the plasmid carrying the cordapA gene along with the plasmid bearing the marker GUS gene. About 80% of the high lysine seeds were GUS positive, suggesting that the plasmid carrying the cordapA gene usually integrated at the same chromosomal site as the plasmid carrying the GUS gene. However, in some transformed lines, e.g. 260-05, there was little correlation between the GUS positive and high lysine phenotypes, indicating that the two plasmids integrated at unlinked sites. Both of these types of transformation events were expected based upon the procedure used for this transformation.

Seeds with a lysine content greater than 20% of the total seed amino acids were obtained. This represents nearly a three hundred percent increase in seed lysine content.

**TABLE 9**

| LINE # | SEED # | GUS | %LYS |
|---|---|---|---|
| 257-1 | G1 | + | 8.30 |
| | G2 | + | 7.99 |
| | G3 | + | 11.51 |
| | G4 | + | 8.52 |
| | G33 | + | 7.68 |
| | G34 | + | 9.93 |
| | G35 | - | 5.97 |
| | G36 | - | 5.71 |
| | G37 | + | 7.48 |
| | G38 | + | 9.42 |
| | G39 | + | 10.44 |
| | G40 | + | 8.63 |
| | G41 | + | 9.42 |
| | G42 | + | 8.53 |
| | G43 | + | 10.54 |
| | G44 | - | 5.83 |
| | G45 | + | 7.15 |
| | G46 | + | 7.85 |
| | G47 | + | 7.34 |
| 257-21 | G21 | + | 12.90 |
| | G22 | + | 11.52 |
| | G23 | + | 9.34 |
| | G24 | - | 5.82 |
| | G25 | - | 5.61 |
| | G26 | - | 5.70 |
| | G27 | - | 5.84 |
| | G28 | - | 14.27 |
| | G48 | + | 15.23 |
| | G49 | + | 18.79 |
| | G50 | + | 13.82 |
| | G51 | - | 5.94 |
| | G52 | + | 13.29 |
| | G53 | + | 14.61 |
| 257-41 | G54 | + | 6.28 |
| | G55 | + | 6.27 |
| | G56 | + | 6.32 |
| | G57 | + | 6.4 |
| | G180 | + | 5.75 |
| | G181 | + | 7.42 |
| 257-46 | G60 | + | 6.76 |
| | G61 | + | 6.73 |
| | G62 | - | 6.18 |
| | G63 | + | 6.13 |
| | G182 | + | 6.83 |
| | G183 | + | 6.23 |
| 257-49 | G78 | - | 6.40 |
| | G79 | + | 6.46 |
| | G184 | + | 6.37 |
| | G185 | + | 6.15 |
| | G186 | + | 6.41 |
| | G187 | + | 7.90 |
| 257-50 | G88 | - | 6.15 |
| | G89 | - | 6.12 |
| | G188 | + | 6.19 |
| | G189 | + | 6.07 |
| | G190 | + | 6.09 |
| | G191 | + | 6.30 |
| 257-51 | G228 | - | 5.81 |
| | G229 | - | 5.74 |
| | G230 | - | 5.59 |
| | G231 | - | 6.00 |
| | G232 | - | 5.89 |
| | G233 | + | 21.49 |
| | G234 | + | 20.30 |
| | G235 | + | 11.89 |
| | G236 | + | 12.40 |
| | G237 | + | 15.09 |
| | G238 | + | 12.79 |
| | G239 | + | 17.19 |
| 260-05 | G90 | - | 5.41 |
| | G91 | - | 7.65 |
| | G95 | - | 6.39 |
| | G96 | - | 5.80 |
| | G97 | - | 6.12 |
| | G98 | - | 5.90 |
| | G99 | - | 6.17 |
| | G160 | - | 8.04 |
| | G161 | - | 12.64 |
| | G162 | - | 6.91 |
| | G163 | - | 5.83 |
| | G164 | - | 8.28 |
| | G165 | - | 12.52 |
| | G166 | - | 5.68 |
| | G167 | - | 9.92 |
| | G168 | - | 5.89 |
| | G169 | - | 6.10 |
| | G170 | + | 6.49 |
| | G171 | + | 6.10 |
| | G172 | - | 12.83 |
| | G173 | - | 6.55 |
| | G174 | - | 6.62 |
| | G175 | + | 13.02 |
| | G176 | - | 10.13 |
| | G177 | - | 5.97 |
| | G178 | - | 11.37 |
| | G179 | - | 12.63 |
| 260-13 | G108 | + | 6.64 |
| | G109 | + | 7.92 |
| | G192 | + | 10.29 |
| | G193 | + | 7.37 |
| | G194 | + | 6.73 |
| | G195 | + | 10.35 |
| 260-16 | G29 | + | 11.64 |
| | G30 | + | 14.87 |
| | G31 | + | 15.02 |
| | G32 | - | 6.24 |
| 260-17 | G115 | + | 11.91 |
| | G116 | - | 6.21 |
| | G117 | - | 6.08 |
| | G118 | - | 6.28 |
| | G119 | - | 6.30 |
| | G196 | + | 7.76 |
| 260-23 | G129 | + | 5.93 |
| | G197 | + | 6.04 |
| | G198 | + | 5.99 |
| | G199 | + | 6.11 |
| | G200 | + | 6.35 |
| | G201 | + | 6.19 |
| 260-31 | G202 | + | 6.19 |
| | G203 | + | 6.19 |
| | G204 | + | 6.13 |
| | G205 | + | 6.40 |
| | G206 | + | 6.73 |
| | G207 | + | 6.23 |
| 260-33 | G217 | + | 6.80 |
| | G218 | - | 7.00 |
| | G219 | - | 6.80 |
| | G220 | - | 6.10 |
| | G221 | - | 6.83 |
| | G222 | - | 6.18 |
| | G223 | + | 5.92 |
| | G224 | + | 6.61 |
| | G226 | + | 6.17 |
| | G227 | + | 6.43 |
| | G240 | + | 6.25 |
| | G241 | + | 6.13 |
| 260-44 | G148 | + | 6.51 |
| | G149 | + | 6.21 |
| | G208 | + | 6.02 |
| | G209 | + | 6.17 |
| | G210 | + | 6.12 |
| | G211 | + | 6.09 |
| 260-46 | G158 | - | 6.00 |
| | G159 | + | 6.30 |
| | G212 | + | 6.40 |
| | G213 | + | 6.50 |
| | G214 | + | 6.40 |
| | G215 | + | 6.60 |

## Claims

1. A chimeric gene wherein a nucleic acid fragment encoding dihydrodipicolinic acid synthase which is insensitive to inhibition by lysine is operably linked to a monocot chloroplast transit sequence and to a monocot seed-specific regulatory sequence.

2. The chimeric gene of claim 1 wherein the monocot seed-specific regulatory sequence is a monocot embryo-specific promoter.

3. The chimeric gene of claim 2, wherein the nucleic acid fragment encoding dihydrodipicolinic acid synthase comprises the nucleotide sequence shown in SEQ ID NO:3 encoding dihydrodipicolinic acid synthase from Corynebacterium glutanicum and wherein the plant chloroplast transit sequence is derived from a gene encoding the small subunit of ribulose 1,5-bisphosphate carboxylase from Zea maize, and the seed-specific regulatory sequence is from the globulin 1 gene from Zea maize.

4. A nucleic acid fragment comprising;
(a) a first chimeric gene wherein a nucleic acid fragment encoding dihydrodipicolinic acid synthase which is insensitive to inhibition by lysine is operably linked to a plant chloroplast transit sequence and to a plant seed-specific regulatory sequence and
(b) a second chimeric gene wherein a nucleic acid fragment encoding a lysine-rich protein, wherein the weight percent lysine is at least 15%, is operably linked to a plant seed-specific regulatory sequence.

5. The nucleic acid fragment of claim 4 wherein the second chimeric gene comprises:
a nucleic acid fragment encoding a lysine-rich protein comprises a nucleic acid sequence encoding a protein comprising n heptad units (d e f g a b c), each heptad being either the same or different, wherein:
n is at least 4;
a and d are independently selected from the group consisting of Met, Leu, Val, Ile and Thr;
e and g are independently selected from the group consisting of the acid/base pairs Glu/Lys, Lys/Glu, Arg/Glu, Arg/Asp, Lys/Asp, Glu/Arg, Asp/Arg and Asp/Lys; and
b, c and f are independently any amino acids except Gly or Pro and at least two amino acids of b, c and f in each heptad are selected from the group consisting of Glu, Lys, Asp, Arg, His, Thr, Ser, Asn, Ala, Gln and Cys,
said nucleic acid fragment is operably linked to a plant seed-specific regulatory sequence.

6. The nucleic acid fragment of claim 4 wherein the a second chimeric gene comprises a nucleic acid fragment encoding a lysine-rich protein comprising a nucleic acid sequence encoding a protein having the amino acid sequence (MEEKLKA)₆(MEEKMKA)₂ which in operably linked to a plant seed-specific regulatory sequence.

7. A nucleic acid fragment comprising;
(a) a first chimeric gene wherein a nucleic acid fragment encoding dihydrodipicolinic acid synthase which is insensitive to inhibition by lysine is operably linked to a plant chloroplast transit sequence and to a plant seed-specific regulatory sequence and
(b) a second chimeric gene comprising a nucleic acid fragment encoding a lysine ketoglutarate reductase operably linked in the sense or antisense orientation to a plant seed-specific regulatory sequence.

8. A corn plant comprising in its genome the chimeric gene of any one of claims 1 to 3.

9. Seed obtained from the corn plant of claim 8 which comprise the chimeric gene of claim 1.

10. A method for obtaining a rapeseed, soybean or corn plant wherein the seeds of the plant accumulate lysine at a level from ten percent to four hundred percent higher then do seeds of an untransformed plant comprising:
(a) transforming rapeseed, soybean or corn plant cells with a chimeric gene comprising a nucleic acid fragment encoding dihydrodipicolinic acid synthase which is insensitive to inhibition by lysine operably linked to a plant chloroplast transit sequence and to a plant seed-specific regulatory sequence;
(b) regenerating fertile mature plants from the transformed plant cells obtained from step (a) under conditions suitable to obtain seeds;
(c) screening the progeny seed of step (b) for lysine content; and
(d) selecting those plants whose seeds contain increased levels of lysine ranging from ten percent to four hundred percent higher than seeds of an untransformed plant.

11. The method of claim 10 for obtaining a corn plant wherein the increase in lysine in the seeds is from ten percent to one hundred thirty percent higher than in seeds of an untransformed corn plant.

12. The method of claim 10, wherein the chimeric gene of step (a) comprises the nucleotide sequence shown in SEQ ID NO:3 encoding dihydrodipicolinic acid synthase from Corynebacterium glutamicum, and wherein the plant chloroplast transit sequence is derived from a gene encoding the small subunit of ribulose 1,5-bisphosphate carboxylase from Glycine max, and wherein the seed-specific regulatory sequence is from the gene encoding the subunit of the seed storage protein phaseolin from the bean Phaseolus vulgaris or the seed-specific regulatory sequence is from the Kunitz trypsin inhibitor 3 gene from Glycine max.

13. The method of claim 10 or claim 11, wherein the seed-specific regulatory sequence is a monocot embryo-specific promoter, and wherein the plant cell of claim 10(a) is from com.

14. The method of claim 10 or claim 11, wherein the plant cell of (a) is from corn and wherein the chimeric gene of step (a) comprises the nucleotide sequence shown in SEQ ID NO:3 encoding dihydrodipicolinic acid synthase from Corynebacterium glutamicum, and wherein the plant chloroplast transit sequence is derived from a gene encoding the small subunit of ribulose 1,5-bisphosphate carboxylase from Zea maize, and wherein the seed-specific regulatory sequence is from the globulin 1 gene from Zea maize.

15. A rapeseed, soybean or corn plant having in its genome the nucleic acid fragment of claim 4, 5, 6 or 7.

16. Rapeseed, soybean or corn seed containing the nucleic acid fragment of claim 4, 5, 6 or 7.

17. A corn plant transformed with the chimeric gene of any one of claims 1 to 3 or a rapeseed, soybean or corn plant transformed with the nucleic acid fragment of any one of claims 4 to 7 wherein the seeds obtained from the plant accumulate lysine at a level from ten percent to four hundred percent higher than do seeds of an untransformed plant.

18. Seeds, obtained from a corn plant, transformed with the chimeric gene of any one of claims 1 to 3, or obtained from a rapeseed, soybean or corn plant transformed with the nucleic acid fragment of any one of claims 4 to 7 containing lysine at a level from ten percent to four hundred percent higher than do seeds obtained from untransformed plants.

19. A corn plant cell transformed with a chimeric gene of any one of claims 1 to 3 or a rapeseed, soybean or corn plant cell transformed with a nucleic acid fragment of any one of claims 4 to 7 wherein seeds obtained from a plant regenerated from the transformed plant cell accumulates lysine at a level from ten percent to four hundred percent higher than do seeds obtained from a plant regenerated from an untransformed plant cell.

20. A seed meal derived from seeds of claim 18.

## Patentansprüche

1. Chimäres Gen, wobei ein Nukleinsäurefragment, das Dihydrodipicolinsäuresynthase kodiert, die gegen Inhibition durch Lysin unempfindlich ist, auf funktionsfähige Weise mit einer Monokotchloroplasttransitsequenz und einer monokoten samenspezifischen Regulationssequenz verknüpft ist.

2. Chimäres Gen nach Anspruch 1, wobei die monokote samenspezifische Regulationssequenz ein monokoter embryospezifischer Promotor ist.

3. Chimäres Gen nach Anspruch 2, wobei das Nukleinsäurefragment, das Dihydrodipicolinsäuresynthase kodiert, die Nukleotidsequenz umfasst, die in SEQ ID NO:3 gezeigt ist, die Dihydrodipicolinsäuresynthase aus Corynebacterium glutanicum kodiert, und wobei die Pflanzenchloroplasttransitsequenz von einem Gen deriviert ist, das die kleine Untereinheit von Ribulose-1,5-bisphosphatcarboxylase aus Zea-Mais kodiert, und die samenspezifische Regulationssequenz aus dem Globulin 1-Gen aus Zea-Mais stammt.

4. Nukleinsäurefragment umfassend:
(a) ein erstes chimäres Gen, wobei ein Nukleinsäurefragment, das Dihydrodipicolinsäuresynthase kodiert, die gegen Inhibition durch Lysin unempfindlich ist, auf funktionsfähige Weise mit einer Pflanzenchloroplasttransitsequenz und einer pflanzensamenspezifischen Regulationssequenz verknüpft und
(b) ein zweites chimäres Gen, wobei ein Nukleinsäurefragment, das ein an Lysin reiches Protein kodiert, wobei der Gewichtsprozentsatz von Lysin mindestens 15 % beträgt, funktionsfähig mit einer pflanzensamenspezifischen Regulationssequenz verknüpft ist.

5. Nukleinsäurefragment nach Anspruch 4, wobei das zweite chimäre Gen folgendes umfasst:
ein Nukleinsäurefragment, das ein lysinreiches Protein kodiert, umfasst eine Nukleinsäuresequenz, die ein Protein kodiert, das n Heptadeinheiten (d e f g a b c) umfasst, wobei jedes Heptad entweder gleich oder verschieden ist, wobei:
n mindestens 4 beträgt,
a und d unabhängig aus der Gruppe ausgewählt sind bestehend aus Met, Leu, Val, Ile und Thr,
e und g unabhängig aus der Gruppe ausgewählt sind bestehend aus den Säure/Basenpaaren Glu/Lys, Lys/Glu, Arg/Glu, Arg/Asp, Lys/Asp, Glu/Arg, Asp/Arg und Asp/Lys; und
b, c und f unabhängig irgendwelche Aminosäuren, mit Ausnahme von Gly oder Pro, sind und mindestens zwei Aminosäuren von b, c und f in jedem Heptad aus der Gruppe ausgewählt sind bestehend aus Glu, Lys, Asp, Arg, His, Thr, Ser, Asn, Ala Gin und Cys,
wobei das Nukleinsäurefragment funktionsfähig mit einer pflanzensamenspezifischen Regulationssequenz verknüpft ist.

6. Nukleinsäurefragment nach Anspruch 4, wobei das zweite chimäre Gen ein Nukleinsäurefragment umfasst, das ein lysinreiches Protein kodiert, das eine Nukleinsäuresequenz umfasst, die ein Protein kodiert, das die Aminosäuresequenz (MEEKLKA)₆(MEEKMKA)₂ aufweist, die funktionsfähig mit einer pflanzensamenspezifischen Regulationssequenz verknüpft ist.

7. Nukleinsäurefragment umfassend:
(a) ein erstes chimäres Gen, wobei ein Nukleinsäurefragment, das Dihydrodipicolinsäuresynthase kodiert, die gegen Inhibition durch Lysin unempfindlich ist, auf funktionsfähige Weise mit einer Pflanzenchloroplasttransitsequenz und einer pflanzensamenspezifischen Regulationssequenz verknüpft ist und
(b) ein zweites chimäres Gen umfassend ein Nukleinsäurefragment, das eine Lysinketoglutaratreductase kodiert, funktionsfähig in der Sinn- oder Gegensinnorientierung mit einer pflanzensamenspezifischen Regulationssequenz verknüpft ist.

8. Maispflanze umfassend in ihrem Genom das chimäre Gen nach irgendeinem der Ansprüche 1 bis 3.

9. Samen, der von der Maispflanze nach Anspruch 8 erhalten worden ist, der das chimäre Gen nach Anspruch 1 umfasst.

10. Methode zum Erhalten einer Rapssamen-, Sojabohnen- oder Maispflanze, wobei die Samen der Pflanze Lysin in einem zehn Prozent bis vierhundert Prozent höheren Niveau aufspeichern als die Samen einer untransformierten Pflanze, umfassend:
(a) das Transformieren von Rapssamen-, Sojabohnen- oder Maispflanzenzellen mit einem chimären Gen umfassend ein Nukleinsäurefragment, das Dihydrodipicolinsäuresynthase kodiert, die gegen Inhibition durch Lysin unempfindlich ist, und die funktionsfähig mit einer Pflanzenchloroplasttransitsequenz und einer pflanzensamenspezifischen Regulationssequenz verknüpft ist;
(b) das Regenerieren fruchtbarer reifer Pflanzen aus den aus Schritt (a) erhaltenen transformierten Pflanzenzellen unter Bedingungen, die zum Erhalten von Samen geeignet sind;
(c) das Screenen des Abkömmlingsamens aus Schritt (b) auf den Lysingehalt; und
(d) das Auswählen derjenigen Pflanzen, deren Samen erhöhte Niveaus an Lysin enthalten, die zehn Prozent bis vierhundert Prozent höher liegen als diejenigen der Samen einer untransformierten Pflanze.

11. Verfahren nach Anspruch 10 zum Erhalten einer Maispflanze, wobei die Lysinerhöhung in den Samen zehn Prozent bis einhundertdreissig Prozent höher ist als in Samen einer untransformierten Maispflanze.

12. Methode nach Anspruch 10, wobei das chimäre Gen aus Schritt (a) die Nukleotidsequenz umfasst, die in SEQ ID NO:3 gezeigt ist, die Dihydrodipicolinsäuresynthase aus Coynebacterium glutamicum kodiert, und wobei die Pflanzenchloroplasttransitsequenz aus einem Gen deriviert ist, das die kleine Untereinheit von Ribulose-1,5-Bisphosphatcarboxylase aus Glycine max kodiert, und wobei die samenspezifische Regulationssequenz aus dem Gen stammt, das die Untereinheit des Samenspeicherproteinphaseolins aus der Bohne Phaseolus vulgaris kodiert oder die samenspezifische Regulationssequenz aus dem Kunitz-Trypsininhibitor-3-Gen aus Glycine max stammt.

13. Verfahren nach Anspruch 10 oder 11, wobei die samenspezifische Regulationssequenz ein monokoter embryospezifischer Promotor ist und wobei die Pflanzenzelle nach Anspruch 10(a) aus Mais stammt.

14. Verfahren nach Anspruch 10 oder 11, wobei die Pflanzenzelle von (a) aus Mais stammt und wobei das chimäre Gen aus Schritt (a) die Nukleotidsequenz umfasst, die in SEQ ID NO:3 gezeigt ist, die Dihydrodipicolinsäuresynthase aus Corynebacterium glutamicum kodiert, und wobei die Pflanzenchloroplasttransitsequenz aus einem Gen deriviert ist, das die kleine Untereinheit von Ribulose-1,5-Bisphosphatcarboxylase aus Zea-Mais kodiert, und wobei die samenspezifische Regulationssequenz aus dem Globilin 1-Gen von Zea-Mais stammt.

15. Rapssamen-, Sojabohnen- oder Maispflanze, die in ihrem Genom das Nukleinsäurefragment nach Anspruch 4, 5, 6 oder 7 aufweist.

16. Rapssamen-, Sojabohnen- oder Maissamen, enthaltend das Nukleinsäurefragment nach Anspruch 4, 5, 6 oder 7.

17. Maispflanze, die mit dem chimären Gen nach irgendeinem der Ansprüche 1 bis 3 transformiert worden ist oder Rapssamen-, Sojabohnen- oder Maispflanze, die mit dem Nukleinsäurefragment nach irgendeinem der Ansprüche 4 bis 7 transformiert worden ist, wobei die aus der Pflanze erhaltenen Samen Lysin in einem zehn Prozent bis vierhundert Prozent höheren Niveau aufspeichern als die Samen einer untransformierten Pflanze.

18. Samen, die aus einer Maispflanze erhalten worden sind, die mit dem chimären Gen nach irgendeinem der Ansprüche 1 bis 3 transformiert worden ist, oder die aus einer Rapssamen-, Sojabohnen- oder Maispflanze erhalten worden sind, die mit dem Nukleinsäurefragment nach irgendeinem der Ansprüche 4 bis 7 transformiert worden ist, die Lysin in einem zehn Prozent bis vierhundert Prozent höheren Niveau enthalten als die Samen, die aus untransformierten Pflanzen erhalten worden sind.

19. Maissamenzelle, die mit einem chimären Gen nach irgendeinem der Ansprüche 1 bis 3 transformiert worden ist oder Rapssamen-, Sojabohnen- oder Maispflanzenzelle, die mit einem Nukleinsäurefragment nach irgendeinem der Ansprüche 4 bis 7 transformiert worden ist, wobei die aus einer Pflanze erhaltenen Samen, die aus der transformierten Pflanzenzelle regeneriert worden sind, Lysin in einem zehn Prozent bis vierhundert Prozent höheren Niveau aufspeichern als Samen, die aus einer Pflanze erhalten worden sind, die aus untransformierten Pflanzenzelle regeneriert worden ist.

20. Aus Samen nach Anspruch 18 deriviertes Samenmehl.

## Revendications

1. Gène chimère dans lequel un fragment d'acide nucléique codant une synthase d'acide dihydrodipicolinique qui est insensible à l'inhibition par la lysine, est lié de manière opérationnelle à une séquence transit de chloroplaste de monocotylédone et à une séquence régulatrice spécifique aux graines de monocotylédone.

2. Le gène chimère de la revendication 1, dans lequel la séquence régulatrice spécifique aux graines de monocotylédone est un promoteur spécifique aux embryons de monocotylédone.

3. Le gène chimère de la revendication 2, dans lequel le fragment d'acide nucléique codant une synthase d'acide dihydrodipicolinique comprend la séquence de nucléotides présentée dans la SEQ ID NO: 3 codant une synthase d'acide dihydrodipicolinique de Corynebacterium glutamicum, et dans lequel la séquence transit de chloroplaste végétal est dérivée d'un gène codant la petite sous-unité de ribulose 1,5-bisphosphate carboxylase de Zea maize, et la séquence régulatrice spécifique aux graines provient du gène de globuline-1 de Zea maize.

4. Fragment d'acide nucléique comprenant:
(a) un premier gène chimère dans lequel un fragment d'acide nucléique codant une synthase d'acide dihydrodipicolinique qui est insensible à l'inhibition par la lysine, est lié de manière opérationnelle à une séquence transit de chloroplaste végétal et à une séquence régulatrice spécifique à des graines végétales et
(b) un deuxième gène chimère dans lequel un fragment d'acide nucléique codant une protéine riche en lysine, où le pourcentage en poids de lysine est d'au moins 15%, est lié de manière opérationnelle à une séquence régulatrice spécifique à des graines végétales.

5. Le fragment d'acide nucléique de la revendication 4, dans lequel le deuxième gène chimère comprend:
un fragment d'acide nucléique codant une protéine riche en lysine, comprend une séquence d'acide nucléique codant une protéine comprenant n unités d'heptades (d e f g a b c), chaque heptade étant soit la même ou différente, où:
n est au moins 4;
a et d sont indépendamment sélectionnés parmi le groupe consistant en Met, Leu, Val, Ile et Thr;
e et g sont indépendamment sélectionnés parmi le groupe consistant en les paires d'acides/bases Glu/Lys, Lys/Glu, Arg/Glu, Arg/Asp, Lys/Asp, Glu/Arg, Asp/Arg et Asp/Lys; et
b, c et f sont indépendamment des acides aminés quelconques à l'exception de Gly ou de Pro, et au moins deux acides aminés de b, c et f dans chaque heptade sont sélectionnés parmi le groupe consistant en Glu, Lys, Asp, Arg, His, Thr, Ser, Asn, Ala, Gln et Cys,
ledit fragment d'acide nucléique est lié de manière opérationnelle à une séquence régulatrice spécifique à des graines végétales.

6. Le fragment d'acide nucléique de la revendication 4, dans lequel le deuxième gène chimère comprend un fragment d'acide nucléique codant une protéine riche en lysine comprenant une séquence d'acide nucléique codant une protéine ayant la séquence d'acides aminés (MEEKLKA)₆(MEEKMKA)₂, qui est lié de manière opérationnelle à une séquence régulatrice spécifique à des graines végétales.

7. Fragment d'acide nucléique comprenant:
(a) un premier gène chimère dans lequel un fragment d'acide nucléique codant une synthase d'acide dihydrodipicolinique qui est insensible à l'inhibition par la lysine, est lié de manière opérationnelle à une séquence transit de chloroplaste végétal et à une séquence régulatrice spécifique à des graines végétales et
(b) un deuxième gène chimère comprenant un fragment d'acide nucléique codant une lysine-cétoglutarate réductase, lié de manière opérationnelle dans l'orientation sens ou anti-sens à une séquence régulatrice spécifique à des graines végétales.

8. Plante de maïs comprenant le gène chimère de l'une quelconque des revendications 1 à 3 dans son génome.

9. Graines obtenues de la plante de maïs de la revendication 8, qui comprennent le gène chimère de la revendication 1.

10. Procédé d'obtention d'une plante de colza, de soja ou de maïs, où les graines de la plante accumulent de la lysine à un taux de dix pour cent à quatre cents pour cent plus élevé que ne le font les graines d'une plante non transformée, comprenant:
(a) transformer des cellules d'une plante de colza, de soja ou de maïs avec un gène chimère comprenant un fragment d'acide nucléique codant une synthase d'acide dihydrodipicolinique qui est insensible à l'inhibition par la lysine, lié de manière opérationnelle à une séquence transit de chloroplaste végétal et à une séquence régulatrice spécifique à des graines végétales;
(b) régénérer des plantes matures fertiles des cellules végétales transformées obtenues de l'étape (a) dans des conditions appropriées pour obtenir des graines;
(c) cribler les graines descendantes de l'étape (b) pour leur teneur en lysine; et
(d) sélectionner ces plantes dont les graines contiennent des taux accrus de lysine allant de dix pour cent à quatre cents pour cent de plus que dans des graines de plantes non transformées.

11. Le procédé de la revendication 10 pour l'obtention d'une plante de maïs où l'augmentation en lysine dans les graines est de dix pour cent à cent trente pour cent de plus que dans des graines d'une plante de maïs non transformée.

12. Le procédé de la revendication 10, dans lequel le gène chimère de l'étape (a) comprend la séquence de nucléotides présentée dans la SEQ ID NO:3 codant une synthase d'acide dihydrodipicolinique de Corynebacterium glutamicum, et dans lequel la séquence transit de chloroplaste végétal est dérivée d'un gène codant la petite sous-unité de ribulose 1,5-bisphosphate carboxylase de Glycine max, et dans lequel la séquence régulatrice spécifique à des graines provient du gène codant la sous-unité de la protéine de stockage de graines phaséoline du haricot Phaseolus vulgaris, ou bien la séquence régulatrice spécifique à des graines provient du gène de l'inhibiteur-3 de la trypsine de Kunitz de Glycine max.

13. Le procédé de la revendication 10 ou de la revendication 11, dans lequel la séquence régulatrice spécifique à des graines est un promoteur spécifique aux embryons de monocotylédone et dans lequel la cellule végétale de la revendication 10(a) provient de maïs.

14. Le procédé de la revendication 10 ou de la revendication 11, dans lequel la cellule végétale de (a) provient de maïs et dans lequel le gène chimère de l'étape (a) comprend la séquence de nucléotides présentée dans la SEQ ID NO:3 codant une synthase d'acide dihydridipicolinique de Corynebacterium glutamicum, et dans lequel la séquence transit de chloroplaste végétal est dérivée d'un gène codant la petite sous-unité de ribulose 1,5-bisphosphate carboxylase de Zea maize, et dans lequel la séquence régulatrice spécifique à des graines provient du gène de globuline-1 de Zea maize.

15. Plante de colza, de soja ou de maïs ayant le fragment d'acide nucléique de la revendication 4, 5, 6 ou 7 dans son génome.

16. Graine de colza, de soja ou de maïs contenant le fragment d'acide nucléique de la revendication 4, 5, 6 ou 7.

17. Plante de maïs transformée avec le gène chimère de l'une quelconque des revendications 1 à 3 ou plante de colza, de soja ou de maïs transformée avec le fragment d'acide nucléique de l'une quelconque des revendications 4 à 7, où les graines obtenues de la plante accumulent de la lysine à un taux de dix pour cent à quatre cents pour cent supérieur que les graines d'une plante non transformée.

18. Graines, obtenues d'une plante de maïs, transformées avec le gène chimère de l'une quelconque des revendications 1 à 3, ou obtenues d'une plante de colza, de soja ou de maïs transformée avec le fragment d'acide nucléique de l'une quelconque des revendications 4 à 7 contenant de la lysine à un taux de dix pour cent à cent trente pour cent plus élevé que ne le font les graines de plantes non transformées.

19. Cellule végétale de maïs transformée avec un gène chimère de l'une quelconque des revendications 1 à 3 ou cellule végétale de colza, de soja ou de maïs transformée avec un fragment d'acide nucléique de l'une quelconque des revendications 4 à 7, où les graines obtenues d'une plante régénérée à partir de la cellule végétale transformée accumulent de la lysine à un taux de dix pour cent à quatre cents pour cent supérieur à celui de graines obtenues d'une plante régénérée à partir d'une cellule végétale non transformée.

20. Farine de graines dérivée des graines de la revendication 18.


### SEQUENCE LISTING

(1) GENERAL INFORMATION:
   (i) APPLICANT:
      (A) NAME: E. I. DU PONT DE NEMOURS AND COMPANY
      (B) STREET: 1007 MARKET STREET
      (C) CITY: WILMINGTON
      (D) STATE: DELAWARE
      (E) COUNTRY: UNITED STATES OF AMERICA
      (F) POSTAL CODE (ZIP): 19898
      (G) TELEPHONE: 302-992-4931
      (H) TELEFAX: 302-773-0164
      (I) TELEX: 6717325
   (ii) TITLE OF INVENTION: CHIMERIC GENES AND METHODS FOR INCREASING THE LYSINE CONTENT OF THE SEEDS OF CORN, SOYBEAN AND RAPESEED PLANTS
   (iii) NUMBER OF SEQUENCES: 100
   (iv) COMPUTER READABLE FORM:
      (A) MEDIUM TYPE: DISKETTE, 3.50 INCH
      (B) COMPUTER: MACINTOSH
      (C) OPERATING SYSTEM: MACINTOSH, 6.0
      (D) SOFTWARE: MICROSOFT WORD, 4.0
   (v) CURRENT APPLICATION DATA:
      (A) APPLICATION NUMBER:
      (B) FILING DATE:
      (C) CLASSIFICATION:
   (vi) PRIOR APPLICATION DATA:
      (A) APPLICATION NUMBER: 08/160,117
      (B) FILING DATE: NOVEMBER 30, 1993
   (vii) ATTORNEY/AGENT INFORMATION:
      (A) NAME: BARBARA C. SIEGELL
      (B) REGISTRATION NUMBER: 30,684
      (C) REFERENCE/DOCKET NUMBER: BB-1055-B
(2) INFORMATION FOR SEQ ID NO:1:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
      CCCGGGCCAT GGCTACAGGT TTAACAGCTA AGACCGGAGT AGAGCACT 48
(2) INFORMATION FOR SEQ ID NO:2:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
      GATATCGAAT TCTCATTATA GAACTCCAGC TTTTTTC 37
(2) INFORMATION FOR SEQ ID NO:3:
   (i) SEQUENCE CHARACTERISTICS:
      (A) LENGTH: 917 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 3..911
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
      CTTCCCGTGA CCATGGGCCA TC 22
(2) INFORMATION FOR SEQ ID NO:5:
   (i) SEQUENCE CHARACTERISTICS:
      (A) LENGTH: 1350 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..1350
   (xi) SEQUENCE DESCRIPTION: SEQ ID NO:5:
   ATG GCT GAA ATT GTT GTC TCC AAA TTT GGC GGT ACC AGC GTA GCT GAT    48
   Met Ala Glu Ile Val Val Ser Lys Phe Gly Gly Thr Ser Val Ala Asp
     1               5                  10                  15
   TTT GAC GCC ATG AAC CGC AGC GCT GAT ATT GTG CTT TCT GAT GCC AAC    96
   Phe Asp Ala Met Asn Arg Ser Ala Asp Ile Val Leu Ser Asp Ala Asn
                20                  25                  30
   GTG CGT TTA GTT GTC CTC TCG GCT TCT GCT GGT ATC ACT AAT CTG CTG   144
   Val Arg Leu Val Val Leu Ser Ala Ser Ala Gly Ile Thr Asn Leu Leu
            35                  40                  45
   GTC GCT TTA GCT GAA GGA CTG GAA CCT GGC GAG CGA TTC GAA AAA CTC    192
   Val Ala Leu Ala Glu Gly Leu Glu Pro Gly Glu Arg Phe Glu Lys Leu
        50                  55                  60
   GAC GCT ATC CGC AAC ATC CAG TTT GCC ATT CTG GAA CGT CTG CGT TAC    240
   Asp Ala Ile Arg Asn Ile Gln Phe Ala Ile Leu Glu Arg Leu Arg Tyr
    65                  70                  75                  80
   CCG AAC GTT ATC CGT GAA GAG ATT GAA CGT CTG CTG GAG AAC ATT ACT    288
   Pro Asn Val Ile Arg Glu Glu Ile Glu Arg Leu Leu Glu Asn Ile Thr
                    85                  90                  95
   GTT CTG GCA GAA GCG GCG GCG CTG GCA ACG TCT CCG GCG CTG ACA GAT    336
   Val Leu Ala Glu Ala Ala Ala Leu Ala Thr Ser Pro Ala Leu Thr Asp
               100                 105                 110
   GAG CTG GTC AGC CAC GGC GAG CTG ATG TCG ACC CTG CTG TTT GTT GAG    384
   Glu Leu Val Ser His Gly Glu Leu Met Ser Thr Leu Leu Phe Val Glu
           115                 120                 125
   ATC CTG CGC GAA CGC GAT GTT CAG GCA CAG TGG TTT GAT GTA CGT AAA    432
   Ile Leu Arg Glu Arg Asp Val Gln Ala Gln Trp Phe Asp Val Arg Lys
       130                 135                 140
   GTG ATG CGT ACC AAC GAC CGA TTT GGT CGT GCA GAG CCA GAT ATA GCC    480
   Val Met Arg Thr Asn Asp Arg Phe Gly Arg Ala Glu Pro Asp Ile Ala
   145                 150                 155                 160
   GCG CTG GCG GAA CTG GCC GCG CTG CAG CTG CTC CCA CGT CTC AAT GAA    528
   Ala Leu Ala Glu Leu Ala Ala Leu Gln Leu Leu Pro Arg Leu Asn Glu
                   165                 170                 175
   GGC TTA GTG ATC ACC CAG GGA TTT ATC GGT AGC GAA AAT AAA GGT CGT    576
   Gly Leu Val Ile Thr Gln Gly Phe Ile Gly Ser Glu Asn Lys Gly Arg
               180                 185                 190
   ACA ACG ACG CTT GGC CGT GGA GGC AGC GAT TAT ACG GCA GCC TTG CTG    624
   Thr Thr Thr Leu Gly Arg Gly Gly Ser Asp Tyr Thr Ala Ala Leu Leu
           195                 200                 205
   GCG GAG GCT TTA CAC GCA TCT CGT GTT GAT ATC TGG ACC GAC GTC CCG    672
   Ala Glu Ala Leu His Ala Ser Arg Val Asp Ile Trp Thr Asp Val Pro
       210                 215                 220
   GGC ATC TAC ACC ACC GAT CCA CGC GTA GTT TCC GCA GCA AAA CGC ATT    720
   Gly Ile Tyr Thr Thr Asp Pro Arg Val Val Ser Ala Ala Lys Arg Ile
   225                 230                 235                 240
   GAT GAA ATC GCG TTT GCC GAA GCG GCA GAG ATG GCA ACT TTT GGT GCA    768
   Asp Glu Ile Ala Phe Ala Glu Ala Ala Glu Met Ala Thr Phe Gly Ala
                   245                 250                 255
   AAA GTA CTG CAT CCG GCA ACG TTG CTA CCC GCA GTA CGC AGC GAT ATC    816
   Lys Val Leu His Pro Ala Thr Leu Leu Pro Ala Val Arg Ser Asp Ile
               260                 265                 270
   CCG GTC TTT GTC GGC TCC AGC AAA GAC CCA CGC GCA GGT GGT ACG CTG    864
   Pro Val Phe Val Gly Ser Ser Lys Asp Pro Arg Ala Gly Gly Thr Leu
           275                 280                 285
   GTG TGC AAT AAA ACT GAA AAT CCG CCG CTG TTC CGC GCT CTG GCG CTT    912
   Val Cys Asn Lys Thr Glu Asn Pro Pro Leu Phe Arg Ala Leu Ala Leu
       290                 295                 300
   CGT CGC AAT CAG ACT CTG CTC ACT TTG CAC AGC CTG AAT ATG CTG CAT    960
   Arg Arg Asn Gln Thr Leu Leu Thr Leu His Ser Leu Asn Met Leu His
   305                 310                 315                 320
   TCT CGC GGT TTC CTC GCG GAA GTT TTC GGC ATC CTC GCG CGG CAT AAT   1008
   Ser Arg Gly Phe Leu Ala Glu Val Phe Gly Ile Leu Ala Arg His Asn
                   325                 330                 335
   ATT TCG GTA GAC TTA ATC ACC ACG TCA GAA GTG AGC GTG GCA TTA ACC   1056
   Ile Ser Val Asp Leu Ile Thr Thr Ser Glu Val Ser Val Ala Leu Thr
               340                 345                 350
   CTT GAT ACC ACC GGT TCA ACC TCC ACT GGC GAT ACG TTG CTG ACG CAA   1104
   Leu Asp Thr Thr Gly Ser Thr Ser Thr Gly Asp Thr Leu Leu Thr Gln
           355                 360                 365
   TCT CTG CTG ATG GAG CTT TCC GCA CTG TGT CGG GTG GAG GTG GAA GAA   1152
   Ser Leu Leu Met Glu Leu Ser Ala Leu Cys Arg Val Glu Val Glu Glu
       370                 375                 380
   GGT CTG GCG CTG GTC GCG TTG ATT GGC AAT GAC CTG TCA AAA GCC TGC   1200
   Gly Leu Ala Leu Val Ala Leu Ile Gly Asn Asp Leu Ser Lys Ala Cys
   385                 390                 395                 400
   GCC GTT GGC AAA GAG GTA TTC GGC GTA CTG GAA CCG TTC AAC ATT CGC   1248
   Ala Val Gly Lys Glu Val Phe Gly Val Leu Glu Pro Phe Asn Ile Arg
                   405                 410                 415
   ATG ATT TGT TAT GGC GCA TCC AGC CAT AAC CTG TGC TTC CTG GTG CCC   1296
   Met Ile Cys Tyr Gly Ala Ser Ser His Asn Leu Cys Phe Leu Val Pro
               420                 425                 430
   GGC GAA GAT GCC GAG CAG GTG GTG CAA AAA CTG CAT AGT AAT TTG TTT   1344
   Gly Glu Asp Ala Glu Gln Val Val Gln Lys Leu His Ser Asn Leu Phe
           435                 440                 445
   GAG TAA                                                           1350
   Glu  *
       450
(2) INFORMATION FOR SEQ ID NO:6:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
      GATCCATGGC TGAAATTGTT GTCTCCAAAT TTGGCG 36
(2) INFORMATION FOR SEQ ID NO:7:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
      GTACCGCCAA ATTTGGAGAC AACAATTTCA GCCATG 36
(2) INFORMATION FOR SEQ ID NO:8:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   CATGGCTGGC TTCCCCACGA GGAAGACCAA CAATGACATT ACCTCCATTG CTAGCAACGG 60
   TGGAAGAGTA CAATG 75
(2) INFORMATION FOR SEQ ID NO:9:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   CATGCATTGT ACTCTTCCAC CGTTGCTAGC AATGGAGGTA ATGTCATTGT TGGTCTTCCT 60
   CGTGGGGAAG CCAGC 75
(2) INFORMATION FOR SEQ ID NO:10:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
   CATGGCTTCC TCAATGATCT CCTCCCCAGC TGTTACCACC GTCAACCGTG CCGGTGCCGG 60
   CATGGTTGCT CCATTCACCG GCCTCAAAAG 90
(2) INFORMATION FOR SEQ ID NO:11:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   CATGCTTTTG AGGCCGGTGA ATGGAGCAAC CATGCCGGCA CCGGCACGGT TGACGGTGGT 60
   AACAGCTGGG GAGGAGATCA TTGAGGAAGC 90
(2) INFORMATION FOR SEQ ID NO:12:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
      CCGGTTTGCT GTAATAGGTA CCA 23
(2) INFORMATION FOR SEQ ID NO:13:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
      AGCTTGGTAC CTATTACAGC AAACCGGCAT G 31
(2) INFORMATION FOR SEQ ID NO:14:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
      GCTTCCTCAA TGATCTCCTC CCCAGCT 27
(2) INFORMATION FOR SEQ ID NO:15:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:15:
      CATTGTACTC TTCCACCGTT GCTAGCAA 28
(2) INFORMATION FOR SEQ ID NO:16:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..20
      (D) OTHER INFORMATION: /product= "synthetic oligonucleotide" /standard_name= "SM 70"
   (xi) SEQUENCE DESCRIPTION: SEQ ID NO:16:
      CTGACTCGCT GCGCTCGGTC 20
(2) INFORMATION FOR SEQ ID NO:17:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..24
      (D) OTHER INFORMATION: /product= "synthetic oligonucleotide" /standard_name= "SM 71"
   (xi) SEQUENCE DESCRIPTION: SEQ ID NO:17:
      TATTTTCTCC TTACGCATCT GTGC 24
(2) INFORMATION FOR SEQ ID NO:18:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..27
      (D) OTHER INFORMATION: /product= "synthetic oligonucleotide" /standard_name= "SM 78"
   (xi) SEQUENCE DESCRIPTION: SEQ ID NO:18:
      TTCATCGATA GGCGACCACA CCCGTCC 27
(2) INFORMATION FOR SEQ ID NO:19:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..27
      (D) OTHER INFORMATION: /product= "synthetic oligonucleotide" /standard_name= "SM 79"
   (xi) SEQUENCE DESCRIPTION: SEQ ID NO:19:
      AATATCGATG CCACGATGCG TCCGGCG 27
(2) INFORMATION FOR SEQ ID NO:20:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..55
      (D) OTHER INFORMATION: /product= "synthetic oligonucleotide" /standard_name= "SM 81"
   (xi) SEQUENCE DESCRIPTION: SEQ ID NO:20:
      CATGGAGGAG AAGATGAAGG CGATGGAAGA GAAGATGAAG GCGTGATAGG TACCG 55
(2) INFORMATION FOR SEQ ID NO:21:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..55
      (D) OTHER INFORMATION: /product= "synthetic oligonucleotide" /standard_name= "SM 80"
   (xi) SEQUENCE DESCRIPTION: SEQ ID NO:21:
      AATTCGGTAC CTATCACGCC TTCATCTTCT CTTCCATCGC CTTCATCTTC TCCTC 55
(2) INFORMATION FOR SEQ ID NO:22:
   (i) SEQUENCE CHARACTERISTICS:
      (A) LENGTH: 14 amino acids
      (B) TYPE: amino acid
      (C) STRANDEDNESS: unknown
      (D) TOPOLOGY: unknown
   (ii) MOLECULE TYPE: protein
   (ix) FEATURE:
      (A) NAME/KEY: Protein
      (B) LOCATION: 1..14
      (D) OTHER INFORMATION: /label= name /note= "base gene [(SSP5)2]"
   (xi) SEQUENCE DESCRIPTION: SEQ ID NO:22:
   Met Glu Glu Lys Met Lys Ala Met Glu Glu Lys Met Lys Ala
   1 5 10
(2) INFORMATION FOR SEQ ID NO:23:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 84"
   (xi) SEQUENCE DESCRIPTION: SEQ ID NO:23:
      GATGGAGGAG AAGATGAAGG C 21
(2) INFORMATION FOR SEQ ID NO:24:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 85"
   (xi) SEQUENCE DESCRIPTION: SEQ ID NO:24:
      ATCGCCTTCA TCTTCTCCTC C 21
(2) INFORMATION FOR SEQ ID NO:25:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 82"
   (xi) SEQUENCE DESCRIPTION: SEQ ID NO:25:
      GATGGAGGAG AAGCTGAAGG C 21
(2) INFORMATION FOR SEQ ID NO:26:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 83"
   (xi) SEQUENCE DESCRIPTION: SEQ ID NO:26:
      ATCGCCTTCA GCTTCTCCTC C 21
(2) INFORMATION FOR SEQ ID NO:27:
   (i) SEQUENCE CHARACTERISTICS:
      (A) LENGTH: 7 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:27:
   Met Glu Glu Lys Leu Lys Ala
   1               5
(2) INFORMATION FOR SEQ ID NO:28:
   (i) SEQUENCE CHARACTERISTICS:
      (A) LENGTH: 7 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:28:
   Met Glu Glu Lys Met Lys Ala
   1               5
(2) INFORMATION FOR SEQ ID NO:29:
   (i) SEQUENCE CHARACTERISTICS:
      (A) LENGTH: 160 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: C15
   (ix) FEATURE:
      (A) NAME/KEY: CDS
      (B) LOCATION: 2..151
      (D) OTHER INFORMATION: /function= "synthetic storage protein" /product= "protein" /gene= "ssp" /standard name= "5.7.7.7.7.7.5"
   (xi) SEQUENCE DESCRIPTION: SEQ ID NO:29:
   C ATG GAG GAG AAG ATG AAG GCG ATG GAG GAG AAG CTG AAG GCG ATG     46
     Met Glu Glu Lys Met Lys Ala Met Glu Glu Lys Leu Lys Ala Met
       1               5                  10                  15
   GAG GAG AAG CTG AAG GCG ATG GAG GAG AAG CTG AAG GCG ATG GAG GAG   94
   Glu Glu Lys Leu Lys Ala Met Glu Glu Lys Leu Lys Ala Met Glu Glu
                    20                  25                  30
   AAG CTG AAG GCG ATG GAG GAG AAG CTG AAG GCG ATG GAA GAG AAG ATG  142
   Lys Leu Lys Ala Met Glu Glu Lys Leu Lys Ala Met Glu Glu Lys Met
                35                  40                  45
   AAG GCG TGATAGGTAC CG                                            160
   Lys Ala
            50
(2) INFORMATION FOR SEQ ID NO:30:
   (i) SEQUENCE CHARACTERISTICS:
      (A) LENGTH: 49 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Met Glu Glu Lys Met Lys Ala Met Glu Glu Lys Leu Lys Ala Met Glu
    1               5                  10                  15
   Glu Lys Leu Lys Ala Met Glu Glu Lys Leu Lys Ala Met Glu Glu Lys
               20                  25                  30
   Leu Lys Ala Met Glu Glu Lys Leu Lys Ala Met Glu Glu Lys Met Lys
           35                  40                  45
   Ala
(2) INFORMATION FOR SEQ ID NO:31:
   (i) SEQUENCE CHARACTERISTICS:
      (A) LENGTH: 160 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: C20
   (ix) FEATURE:
      (A) NAME/KEY: CDS
      (B) LOCATION: 2..151
      (D) OTHER INFORMATION: /function= "synthetic storage protein" /product= "protein" /gene= "ssp" /standard name= "5.7.7.7.7̅.7.5"
   (xi) SEQUENCE DESCRIPTION: SEQ ID NO:31:
   C ATG GAG GAG AAG ATG AAG GCG ATG GAG GAG AAG CTG AAG GCG ATG      46
     Met Glu Glu Lys Met Lys Ala Met Glu Glu Lys Leu Lys Ala Met
      1               5                  10                  15
   GAG GAG AAG CTG AAG GCG ATG GAG GAG AAG CTG AAG GCG ATG GAG GAG    94
   Glu Glu Lys Leu Lys Ala Met Glu Glu Lys Leu Lys Ala Met Glu Glu
                   20                  25                  30
   AAG CTG AAG GCG ATG GAG GAG AAG CTG AAG GCG ATG GAA GAG AAG ATG   142
   Lys Leu Lys Ala Met Glu Glu Lys Leu Lys Ala Met Glu Glu Lys Met
               35                  40                  45
   AAG GCG TGATAGGTAC CG                                             160
   Lys Ala
           50
(2) INFORMATION FOR SEQ ID NO:32:
   (i) SEQUENCE CHARACTERISTICS:
      (A) LENGTH: 49 amino acids
      (B) TYPE: amino acid
      (D) TOPOLOGY: linear
   (ii) MOLECULE TYPE: protein
   (xi) SEQUENCE DESCRIPTION: SEQ ID NO:32:
   Met Glu Glu Lys Met Lys Ala Met Glu Glu Lys Leu Lys Ala Met Glu
    1               5                  10                  15
   Glu Lys Leu Lys Ala Met Glu Glu Lys Leu Lys Ala Met Glu Glu Lys
               20                  25                  30
   Leu Lys Ala Met Glu Glu Lys Leu Lys Ala Met Glu Glu Lys Met Lys
           35                  40                  45
   Ala
(2) INFORMATION FOR SEQ ID NO:33:
   (i) SEQUENCE CHARACTERISTICS:
      (A) LENGTH: 139 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: C30
   (ix) FEATURE:
      (A) NAME/KEY: CDS
      (B) LOCATION: 2..130
      (D) OTHER INFORMATION: /function= "synthetic storage protein" /product= "protein" /gene= "ssp" /standard_name= "5.7.7.7.7.5"
   (xi) SEQUENCE DESCRIPTION: SEQ ID NO:33:
   C ATG GAG GAG AAG ATG AAG GCG ATG GAG GAG AAG CTG AAG GCG ATG      46
     Met Glu Glu Lys Met Lys Ala Met Glu Glu Lys Leu Lys Ala Met
       1               5                  10                  15
   GAG GAG AAG CTG AAG GCG ATG GAG GAG AAG CTG AAG GCG ATG GAG GAG   94
   Glu Glu Lys Leu Lys Ala Met Glu Glu Lys Leu Lys Ala Met Glu Glu
                    20                  25                  30
   AAG CTG AAG GCG ATG GAA GAG AAG ATG AAG GCG TGATAGGTAC CG         139
   Lys Leu Lys Ala Met Glu Glu Lys Met Lys Ala
                35                  40
(2) INFORMATION FOR SEQ ID NO:34:
   (i) SEQUENCE CHARACTERISTICS:
      (A) LENGTH: 42 amino acids
      (B) TYPE: amino acid
      (D) TOPOLOGY: linear
   (ii) MOLECULE TYPE: protein
   (xi) SEQUENCE DESCRIPTION: SEQ ID NO:34:
   Met Glu Glu Lys Met Lys Ala Met Glu Glu Lys Leu Lys Ala Met Glu
     1               5                  10                  15
   Glu Lys Leu Lys Ala Met Glu Glu Lys Leu Lys Ala Met Glu Glu Lys
                20                  25                  30
   Leu Lys Ala Met Glu Glu Lys Met Lys Ala
            35                  40
(2) INFORMATION FOR SEQ ID NO:35:
   (i) SEQUENCE CHARACTERISTICS:
      (A) LENGTH: 97 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: D16
   (ix) FEATURE:
      (A) NAME/KEY: CDS
      (B) LOCATION: 2..88
      (D) OTHER INFORMATION: /function= "synthetic storage protein" /product= "protein" /gene= "ssp" /standard_name= "5.5.5.5"
   (xi) SEQUENCE DESCRIPTION: SEQ ID NO:35:
   C ATG GAG GAG AAG ATG AAG GCG ATG GAG GAG AAG ATG AAG GCG ATG   46
     Met Glu Glu Lys Met Lys Ala Met Glu Glu Lys Met Lys Ala Met
       1               5                  10                  15
   GAG GAG AAG ATG AAG GCG ATG GAA GAG AAG ATG AAG GCG TGATAGGTAC  95
   Glu Glu Lys Met Lys Ala Met Glu Glu Lys Met Lys Ala
                    20                  25
   CG                                                              97
(2) INFORMATION FOR SEQ ID NO:36:
   (i) SEQUENCE CHARACTERISTICS:
      (A) LENGTH: 28 amino acids
      (B) TYPE: amino acid
      (D) TOPOLOGY: linear
   (ii) MOLECULE TYPE: protein
   (xi) SEQUENCE DESCRIPTION: SEQ ID NO:36:
   Met Glu Glu Lys Met Lys Ala Met Glu Glu Lys Met Lys Ala Met Glu
     1               5                  10                  15
   Glu Lys Met Lys Ala Met Glu Glu Lys Met Lys Ala
                20                  25
(2) INFORMATION FOR SEQ ID NO:37:
   (i) SEQUENCE CHARACTERISTICS:
      (A) LENGTH: 118 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: D20
   (ix) FEATURE:
      (A) NAME/KEY: CDS
      (B) LOCATION: 2..109
      (D) OTHER INFORMATION: /function= "synthetic storage protein" /product= "protein" /gene= "ssp" /standard_name= "5.5.5.5.5"
   (xi) SEQUENCE DESCRIPTION: SEQ ID NO:37:
   C ATG GAG GAG AAG ATG AAG GCG ATG GAG GAG AAG ATG AAG GCG ATG     46
     Met Glu Glu Lys Met Lys Ala Met Glu Glu Lys Met Lys Ala Met
       1               5                  10                  15
   GAG GAG AAG ATG AAG GCG ATG GAG GAG AAG ATG AAG GCG ATG GAA GAG   94
   Glu Glu Lys Met Lys Ala Met Glu Glu Lys Met Lys Ala Met Glu Glu
                   20                  25                  30
   AAG ATG AAG GCG TGATAGGTAC CG                                    118
   Lys Met Lys Ala
               35
(2) INFORMATION FOR SEQ ID NO:38:
   (i) SEQUENCE CHARACTERISTICS:
      (A) LENGTH: 35 amino acids
      (B) TYPE: amino acid
      (D) TOPOLOGY: linear
   (ii) MOLECULE TYPE: protein
   (xi) SEQUENCE DESCRIPTION: SEQ ID NO:38:
   Met Glu Glu Lys Met Lys Ala Met Glu Glu Lys Met Lys Ala Met Glu
     1               5                  10                  15
   Glu Lys Met Lys Ala Met Glu Glu Lys Met Lys Ala Met Glu Glu Lys
               20                  25                  30
   Met Lys Ala
           35
(2) INFORMATION FOR SEQ ID NO:39:
   (i) SEQUENCE CHARACTERISTICS:
      (A) LENGTH: 97 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: D33
   (ix) FEATURE:
      (A) NAME/KEY: CDS
      (B) LOCATION: 2..88
      (D) OTHER INFORMATION: /function= "synthetic storage protein" /product= "protein" /gene= "ssp" /standard_name= "5.5.5.5"
   (xi) SEQUENCE DESCRIPTION: SEQ ID NO:39:
   C ATG GAG GAG AAG ATG AAG GCG ATG GAG GAG AAG ATG AAG GCG ATG   46
     Met Glu Glu Lys Met Lys Ala Met Glu Glu Lys Met Lys Ala Met
      1               5                  10                  15
   GAG GAG AAG ATG AAG GCG ATG GAA GAG AAG ATG AAG GCG TGATAGGTAC  95
   Glu Glu Lys Met Lys Ala Met Glu Glu Lys Met Lys Ala
                   20                  25
   CG                                                              97
(2) INFORMATION FOR SEQ ID NO:40:
   (i) SEQUENCE CHARACTERISTICS:
      (A) LENGTH: 28 amino acids
      (B) TYPE: amino acid
      (D) TOPOLOGY: linear
   (ii) MOLECULE TYPE: protein
   (xi) SEQUENCE DESCRIPTION: SEQ ID NO:40:
   Met Glu Glu Lys Met Lys Ala Met Glu Glu Lys Met Lys Ala Met Glu
    1               5                  10                  15
   Glu Lys Met Lys Ala Met Glu Glu Lys Met Lys Ala
               20                  25
(2) INFORMATION FOR SEQ ID NO:41:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 86"
   (xi) SEQUENCE DESCRIPTION: SEQ ID NO:41:
      GATGGAGGAG AAGCTGAAGA A 21
(2) INFORMATION FOR SEQ ID NO:42:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 87"
   (xi) SEQUENCE DESCRIPTION: SEQ ID NO:42:
      ATCTTCTTCA GCTTCTCCTC C 21
(2) INFORMATION FOR SEQ ID NO:43:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 88"
   (xi) SEQUENCE DESCRIPTION: SEQ ID NO:43:
      GATGGAGGAG AAGCTGAAGT G 21
(2) INFORMATION FOR SEQ ID NO:44:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 89"
   (xi) SEQUENCE DESCRIPTION: SEQ ID NO:44:
      ATCCACTTCA GCTTCTCCTC C 21
(2) INFORMATION FOR SEQ ID NO:45:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 90"
   (xi) SEQUENCE DESCRIPTION: SEQ ID NO:45:
      GATGGAGGAG AAGATGAAGA A 21
(2) INFORMATION FOR SEQ ID NO:46:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 91"
   (xi) SEQUENCE DESCRIPTION: SEQ ID NO:46:
      ATCTTCTTCA TCTTCTCCTC C 21
(2) INFORMATION FOR SEQ ID NO:47:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 92"
   (xi) SEQUENCE DESCRIPTION: SEQ ID NO:47:
      GATGGAGGAG AAGATGAAGT G 21
(2) INFORMATION FOR SEQ ID NO:48:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..21
      (D) OTHER INFORMATION: /product= "synthetic oligonucleotide" /standard_name= "SM 93"
   (xi) SEQUENCE DESCRIPTION: SEQ ID NO:48:
      ATCCACTTCA TCTTCTCCTC C 21
(2) INFORMATION FOR SEQ ID NO:49:
   (i) SEQUENCE CHARACTERISTICS:
      (A) LENGTH: 7 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:49:
   Met Glu Glu Lys Leu Lys Lys
   1               5
(2) INFORMATION FOR SEQ ID NO:50:
   (i) SEQUENCE CHARACTERISTICS:
      (A) LENGTH: 7 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:50:
   Met Glu Glu Lys Leu Lys Trp
   1               5
(2) INFORMATION FOR SEQ ID NO:51:
   (i) SEQUENCE CHARACTERISTICS:
      (A) LENGTH: 7 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:51:
   Met Glu Glu Lys Met Lys Lys
   1               5
(2) INFORMATION FOR SEQ ID NO:52:
   (i) SEQUENCE CHARACTERISTICS:
      (A) LENGTH: 7 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:52:
   Met Glu Glu Lys Met Lys Trp
   1               5
(2) INFORMATION FOR SEQ ID NO:53:
   (i) SEQUENCE CHARACTERISTICS:
      (A) LENGTH: 160 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 82-4
   (ix) FEATURE:
      (A) NAME/KEY: CDS
      (B) LOCATION: 2..151
      (D) OTHER INFORMATION: /function= "synthetic storage protein /product= "protein" /gene= "ssp" /standard_name= "7.7.7.7.7.7.5"
   (xi) SEQUENCE DESCRIPTION: SEQ ID NO:53:
   C ATG GAG GAG AAG CTG AAG GCG ATG GAG GAG AAG CTG AAG GCG ATG     46
     Met Glu Glu Lys Leu Lys Ala Met Glu Glu Lys Leu Lys Ala Met
       1               5                  10                  15
   GAG GAG AAG CTG AAG GCG ATG GAG GAG AAG CTG AAG GCG ATG GAG GAG   94
   Glu Glu Lys Leu Lys Ala Met Glu Glu Lys Leu Lys Ala Met Glu Glu
                    20                  25                  30
   AAG CTG AAG GCG ATG GAG GAG AAG CTG AAG GCG ATG GAA GAG AAG ATG  142
   Lys Leu Lys Ala Met Glu Glu Lys Leu Lys Ala Met Glu Glu Lys Met
                35                  40                  45
   AAG GCG TGATAGGTAC CG                                            160
   Lys Ala
            50
(2) INFORMATION FOR SEQ ID NO:54:
   (i) SEQUENCE CHARACTERISTICS:
      (A) LENGTH: 49 amino acids
      (B) TYPE: amino acid
      (D) TOPOLOGY: linear
   (ii) MOLECULE TYPE: protein
   (xi) SEQUENCE DESCRIPTION: SEQ ID NO:54:
   Met Glu Glu Lys Leu Lys Ala Met Glu Glu Lys Leu Lys Ala Met Glu
    1               5                  10                  15
   Glu Lys Leu Lys Ala Met Glu Glu Lys Leu Lys Ala Met Glu Glu Lys
               20                  25                  30
   Leu Lys Ala Met Glu Glu Lys Leu Lys Ala Met Glu Glu Lys Met Lys
           35                  40                  45
   Ala
(2) INFORMATION FOR SEQ ID NO:55:
   (i) SEQUENCE CHARACTERISTICS:
      (A) LENGTH: 97 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 84-H3
   (ix) FEATURE:
      (A) NAME/KEY: CDS
      (B) LOCATION: 2..88
      (D) OTHER INFORMATION: /function= "synthetic storage protein /product= "protein" /gene= "ssp" /standard_name= "5.5.5.5"
   (xi) SEQUENCE DESCRIPTION: SEQ ID NO:55:
   C ATG GAG GAG AAG ATG AAG GCG ATG GAG GAG AAG ATG AAG GCG ATG   46
     Met Glu Glu Lys Met Lys Ala Met Glu Glu Lys Met Lys Ala Met
      1               5                  10                  15
   GAG GAG AAG ATG AAG GCG ATG GAA GAG AAG ATG AAG GCG TGATAGGTAC  95
   Glu Glu Lys Met Lys Ala Met Glu Glu Lys Met Lys Ala
                   20                  25
   CG                                                              97
(2) INFORMATION FOR SEQ ID NO:56:
   (i) SEQUENCE CHARACTERISTICS:
      (A) LENGTH: 28 amino acids
      (B) TYPE: amino acid
      (D) TOPOLOGY: linear
   (ii) MOLECULE TYPE: protein
   (xi) SEQUENCE DESCRIPTION: SEQ ID NO:56:
   Met Glu Glu Lys Met Lys Ala Met Glu Glu Lys Met Lys Ala Met Glu
    1               5                  10                  15
   Glu Lys Met Lys Ala Met Glu Glu Lys Met Lys Ala
               20                  25
(2) INFORMATION FOR SEQ ID NO:57:
   (i) SEQUENCE CHARACTERISTICS:
      (A) LENGTH: 97 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 86-H23
   (ix) FEATURE:
      (A) NAME/KEY: CDS
      (B) LOCATION: 2..88
      (D) OTHER INFORMATION: /function= "synthetic storage protein /product= "protein" /gene= "ssp" /standard_name= "5.8.8.5"
   (xi) SEQUENCE DESCRIPTION: SEQ ID NO:57:
   C ATG GAG GAG AAG ATG AAG GCG ATG GAG GAG AAG CTG AAG AAG ATG    46
     Met Glu Glu Lys Met Lys Ala Met Glu Glu Lys Leu Lys Lys Met
      1               5                  10                  15
   GAG GAG AAG CTG AAG AAG ATG GAA GAG AAG ATG AAG GCG TGATAGGTAC   95
   Glu Glu Lys Leu Lys Lys Met Glu Glu Lys Met Lys Ala
               20                  25
   CG                                                               97
(2) INFORMATION FOR SEQ ID NO:58:
   (i) SEQUENCE CHARACTERISTICS:
      (A) LENGTH: 28 amino acids
      (B) TYPE: amino acid
      (D) TOPOLOGY: linear
   (ii) MOLECULE TYPE: protein
   (xi) SEQUENCE DESCRIPTION: SEQ ID NO:58:
   Met Glu Glu Lys Met Lys Ala Met Glu Glu Lys Leu Lys Lys Met Glu
    1               5                  10                  15
   Glu Lys Leu Lys Lys Met Glu Glu Lys Met Lys Ala
               20                  25
(2) INFORMATION FOR SEQ ID NO:59:
   (i) SEQUENCE CHARACTERISTICS:
      (A) LENGTH: 112 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 88-2
   (ix) FEATURE:
      (A) NAME/KEY: CDS
      (B) LOCATION: 2..103
      (D) OTHER INFORMATION: /function= "synthetic storage protein /product= "protein" /gene= "ssp" /standard name= "5.9.9.9.5"
   (xi) SEQUENCE DESCRIPTION: SEQ ID NO:59:
   C ATG GAG GAG AAG ATG AAG GCG AAG AAG CTG AAG TGG ATG GAG GAG    46
     Met Glu Glu Lys Met Lys Ala Lys Lys Leu Lys Trp Met Glu Glu
      1               5                  10                  15
   AAG CTG AAG TGG ATG GAG GAG AAG CTG AAG TGG ATG GAA GAG AAG ATG  94
   Lys Leu Lys Trp Met Glu Glu Lys Leu Lys Trp Met Glu Glu Lys Met
                   20                  25                  30
   AAG GCG TGATAGGTAC CG                                            112
   Lys Ala
(2) INFORMATION FOR SEQ ID NO:60:
   (i) SEQUENCE CHARACTERISTICS:
      (A) LENGTH: 33 amino acids
      (B) TYPE: amino acid
      (D) TOPOLOGY: linear
   (ii) MOLECULE TYPE: protein
   (xi) SEQUENCE DESCRIPTION: SEQ ID NO:60:
   Met Glu Glu Lys Met Lys Ala Lys Lys Leu Lys Trp Met Glu Glu Lys
     1               5                  10                  15
   Leu Lys Trp Met Glu Glu Lys Leu Lys Trp Met Glu Glu Lys Met Lys
                20                  25                  30
   Ala
(2) INFORMATION FOR SEQ ID NO:61:
   (i) SEQUENCE CHARACTERISTICS:
      (A) LENGTH: 118 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 90-H8
   (ix) FEATURE:
      (A) NAME/KEY: CDS
      (B) LOCATION: 2..109
      (D) OTHER INFORMATION: /function= "synthetic storage protein /product= "protein" ./gene= "ssp" /standard_name= "5.10.10.10.5"
   (xi) SEQUENCE DESCRIPTION: SEQ ID NO:61:
   C ATG GAG GAG AAG ATG AAG GCG ATG GAG GAG AAG ATG AAG AAG ATG   46
     Met Glu Glu Lys Met Lys Ala Met Glu Glu Lys Met Lys Lys Met
       1               5                  10                  15
   GAG GAG AAG ATG AAG AAG ATG GAG GAG AAG ATG AAG AAG ATG GAA GAG 94
   Glu Glu Lys Met Lys Lys Met Glu Glu Lys Met Lys Lys Met Glu Glu
                    20                  25                  30
   AAG ATG AAG GCG TGATAGGTAC CG                                118
   Lys Met Lys Ala
                35
(2) INFORMATION FOR SEQ ID NO:62:
   (i) SEQUENCE CHARACTERISTICS:
      (A) LENGTH: 35 amino acids
      (B) TYPE: amino acid
      (D) TOPOLOGY: linear
   (ii) MOLECULE TYPE: protein
   (xi) SEQUENCE DESCRIPTION: SEQ ID NO:62:
   Met Glu Glu Lys Met Lys Ala Met Glu Glu Lys Met Lys Lys Met Glu
    1               5                  10                  15
   Glu Lys Met Lys Lys Met Glu Glu Lys Met Lys Lys Met Glu Glu Lys
               20                  25                  30
   Met Lys Ala
           35
(2) INFORMATION FOR SEQ ID NO:63:
   (i) SEQUENCE CHARACTERISTICS:
      (A) LENGTH: 97 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 92-2
   (ix) FEATURE:
      (A) NAME/KEY: CDS
      (B) LOCATION: 2..88
      (D) OTHER INFORMATION: /function= "synthetic storage protein /product= "protein" /gene= "ssp" /standard_name= "5.11.11.5"
   (xi) SEQUENCE DESCRIPTION: SEQ ID NO:63:
   C ATG GAG GAG AAG ATG AAG GCG ATG GAG GAG AAG ATG AAG TGG ATG   46
     Met Glu Glu Lys Met Lys Ala Met Glu Glu Lys Met Lys Trp Met
       1               5                  10                  15
   GAG GAG AAG ATG AAG TGG ATG GAA GAG AAG ATG AAG GCG TGATAGGTAC   95
   Glu Glu Lys Met Lys Trp Met Glu Glu Lys Met Lys Ala
                    20                  25
   CG                                                                97
(2) INFORMATION FOR SEQ ID NO:64:
   (i) SEQUENCE CHARACTERISTICS:
      (A) LENGTH: 28 amino acids
      (B) TYPE: amino acid
      (D) TOPOLOGY: linear
   (ii) MOLECULE TYPE: protein
   (xi) SEQUENCE DESCRIPTION: SEQ ID NO:64:
   Met Glu Glu Lys Met Lys Ala Met Glu Glu Lys Met Lys Trp Met Glu
     1               5                  10                  15
   Glu Lys Met Lys Trp Met Glu Glu Lys Met Lys Ala
                20                  25
(2) INFORMATION FOR SEQ ID NO:65:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..84
      (D) OTHER INFORMATION: /product= "synthetic oligonucleotide" /standard_name= "SM 96"
   (xi) SEQUENCE DESCRIPTION: SEQ ID NO:65:
   GATGGAGGAA AAGATGAAGG CGATGGAGGA GAAAATGAAA GCTATGGAGG AAAAGATGAA 60
   AGCGATGGAG GAGAAAATGA AGGC                                        84
(2) INFORMATION FOR SEQ ID NO:66:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..84
      (D) OTHER INFORMATION: /product= "synthetic oligonucleotide" /standard_name= "SM 97"
   (xi) SEQUENCE DESCRIPTION: SEQ ID NO:66:
   ATCGCCTTCA TTTTCTCCTC CATCGCTTTC ATCTTTTCCT CCATAGCTTT CATTTTCTCC 60
   TCCATCGCCT TCATCTTTTC CTCC                                        84
(2) INFORMATION FOR SEQ ID NO:67:
   (i) SEQUENCE CHARACTERISTICS:
      (A) LENGTH: 28 amino acids
      (B) TYPE: amino acid
      (C) STRANDEDNESS: unknown
      (D) TOPOLOGY: unknown
   (ii) MOLECULE TYPE: protein
   (ix) FEATURE:
      (A) NAME/KEY: Protein
      (B) LOCATION: 1..28
      (D) OTHER INFORMATION: /label= name /note= "(SSP 5)4"
   (xi) SEQUENCE DESCRIPTION: SEQ ID NO:67:
   Met Glu Glu Lys Met Lys Ala Met Glu Glu Lys Met Lys Ala Met Glu
   1               5                   10                  15
   Glu Lys Met Lys Ala Met Glu Glu Lys Met Lys Ala
                   20                  25
(2) INFORMATION FOR SEQ ID NO:68:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..84
      (D) OTHER INFORMATION: /product= "synthetic oligonucleotide" /standard_name= "SM 98"
   (xi) SEQUENCE DESCRIPTION: SEQ ID NO:68:
   GATGGAGGAA AAGCTGAAAG CGATGGAGGA GAAACTCAAG GCTATGGAAG AAAAGCTTAA 60
   AGCGATGGAG GAGAAACTGA AGGC 84
(2) INFORMATION FOR SEQ ID NO:69:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..84
      (D) OTHER INFORMATION: /product= "synthetic oligonucleotide" /standard_name= "SM 99"
   (xi) SEQUENCE DESCRIPTION: SEQ ID NO:69:
   ATCGCCTTCA GTTTCTCCTC CTACGCTTTA AGCTTTTCTT CCATAGCCTT GAGTTTCTCC 60
   TCCATCGCTT TCAGCTTTTC CTCC 84
(2) INFORMATION FOR SEQ ID NO:70:
   (i) SEQUENCE CHARACTERISTICS:
      (A) LENGTH: 28 amino acids
      (B) TYPE: amino acid
      (C) STRANDEDNESS: unknown
      (D) TOPOLOGY: unknown
   (ii) MOLECULE TYPE: protein
   (ix) FEATURE:
      (A) NAME/KEY: Protein
      (B) LOCATION: 1..28
      (D) OTHER INFORMATION: /label= name /note= "(SSP 7)4"
   (xi) SEQUENCE DESCRIPTION: SEQ ID NO:70:
   Met Glu Glu Lys Leu Lys Ala Met Glu Glu Lys Leu Lys Ala Met Glu
   1               5                   10                  15
   Glu Lys Leu Lys Ala Met Glu Glu Lys Leu Lys Ala
               20                  25
(2) INFORMATION FOR SEQ ID NO:71:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..84
      (D) OTHER INFORMATION: /product= "synthetic oligonucleotide" /standard_name= "SM 100"
   (xi) SEQUENCE DESCRIPTION: SEQ ID NO:71:
   GATGGAGGAA AAGCTTAAGA AGATGGAAGA AAAGCTGAAA TGGATGGAGG AGAAACTCAA 60
   AAAGATGGAG GAAAAGCTTA AATG                                         84
(2) INFORMATION FOR SEQ ID NO:72:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..84
      (D) OTHER INFORMATION: /product= "synthetic oligonucleotide" /standard_name= "SM 101"
   (xi) SEQUENCE DESCRIPTION: SEQ ID NO:72:
   ATCCATTTAA GCTTTTCCTC CTACTTTTTG AGTTTCTCCT CCATCCATTT CAGCTTTTCT 60
   TCCATCTTCT TAAGCTTTTC CTCC                                         84
(2) INFORMATION FOR SEQ ID NO:73:
   (i) SEQUENCE CHARACTERISTICS:
      (A) LENGTH: 28 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:73:
   Met Glu Glu Lys Leu Lys Lys Met Glu Glu Lys Leu Lys Trp Met Glu
   1               5                   10                  15
   Glu Lys Leu Lys Lys Met Glu Glu Lys Leu Lys Trp
               20                  25
(2) INFORMATION FOR SEQ ID NO:74:
   (i) SEQUENCE CHARACTERISTICS:
      (A) LENGTH: 243 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 2-9
   (ix) FEATURE:
      (A) NAME/KEY: CDS
      (B) LOCATION: 2..235
      (D) OTHER INFORMATION: /function= "synthetic storage protein /product= "protein" /gene= "ssp" /standard name= "7.7.7.7.7̅.7.8.9.8.9.5"
   (xi) SEQUENCE DESCRIPTION: SEQ ID NO:74:
   C ATG GAG GAG AAG CTG AAG GCG ATG GAG GAG AAG CTG AAG GCG ATG         46
     Met Glu Glu Lys Leu Lys Ala Met Glu Glu Lys Leu Lys Ala Met
      1               5                  10                  15
   GAG GAG AAG CTG AAG GCG ATG GAG GAG AAG CTG AAG GCG ATG GAG GAG       94
   Glu Glu Lys Leu Lys Ala Met Glu Glu Lys Leu Lys Ala Met Glu Glu
                   20                  25                  30
   AAG CTG AAG GCG ATG GAG GAG AAG CTG AAG GCG ATG GAG GAA AAG CTT      142
   Lys Leu Lys Ala Met Glu Glu Lys Leu Lys Ala Met Glu Glu Lys Leu
               35                  40                  45
   AAG AAG ATG GAA GAA AAG CTG AAA TGG ATG GAG GAG AAA CTC AAA AAG      190
   Lys Lys Met Glu Glu Lys Leu Lys Trp Met Glu Glu Lys Leu Lys Lys
           50                  55                  60
   ATG GAG GAA AAG CTT AAA TGG ATG GAA GAG AAG ATG AAG GCG TGATAGGTAC 242
   Met Glu Glu Lys Leu Lys Trp Met Glu Glu Lys Met Lys Ala
       65                  70                  75
   C                                                                    243
(2) INFORMATION FOR SEQ ID NO:75:
   (i) SEQUENCE CHARACTERISTICS:
      (A) LENGTH: 77 amino acids
      (B) TYPE: amino acid
      (D) TOPOLOGY: linear
   (ii) MOLECULE TYPE: protein
   (xi) SEQUENCE DESCRIPTION: SEQ ID NO:75:
   Met Glu Glu Lys Leu Lys Ala Met Glu Glu Lys Leu Lys Ala Met Glu
    1               5                  10                  15
   Glu Lys Leu Lys Ala Met Glu Glu Lys Leu Lys Ala Met Glu Glu Lys
               20                  25                  30
   Leu Lys Ala Met Glu Glu Lys Leu Lys Ala Met Glu Glu Lys Leu Lys
           35                  40                  45
   Lys Met Glu Glu Lys Leu Lys Trp Met Glu Glu Lys Leu Lys Lys Met
       50                  55                  60
   Glu Glu Lys Leu Lys Trp Met Glu Glu Lys Met Lys Ala
    65                  70                  75
(2) INFORMATION FOR SEQ ID NO:76:
   (i) SEQUENCE CHARACTERISTICS:
      (A) LENGTH: 175 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (vii) IMMEDIATE SOURCE:
      (B) CLONE: 5-1
   (ix) FEATURE:
      (A) NAME/KEY: CDS
      (B) LOCATION: 2..172
      (D) OTHER INFORMATION: /function= "synthetic storage protein /product= "protein" /gene= "ssp" /standard name= "5.5.5.7.7̅.7.7.5"
   (xi) SEQUENCE DESCRIPTION: SEQ ID NO:76:
(2) INFORMATION FOR SEQ ID NO:77:
   (i) SEQUENCE CHARACTERISTICS:
      (A) LENGTH: 56 amino acids
      (B) TYPE: amino acid
      (D) TOPOLOGY: linear
   (ii) MOLECULE TYPE: protein
   (xi) SEQUENCE DESCRIPTION: SEQ ID NO:77:
   Met Glu Glu Lys Met Lys Ala Met Glu Glu Lys Met Lys Ala Met Glu
     1               5                  10                  15
   Glu Lys Met Lys Ala Met Glu Glu Lys Leu Lys Ala Met Glu Glu Lys
               20                  25                  30
   Leu Lys Ala Met Glu Glu Lys Leu Lys Ala Met Glu Glu Lys Leu Lys
           35                  40                  45
   Ala Met Glu Glu Lys Met Lys Ala
       50                  55
(2) INFORMATION FOR SEQ ID NO:78:
   (i) SEQUENCE CHARACTERISTICS:
      (A) LENGTH: 187 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (B) STRAIN: E. coli
      (G) CELL TYPE: DH5 alpha
   (ix) FEATURE:
      (A) NAME/KEY: CDS
      (B) LOCATION: 3..173
      (D) OTHER INFORMATION: /function= "synthetic storage protein /product= "protein" /gene= "ssp" /standard_name= "SSP-3-5"
   (xi) SEQUENCE DESCRIPTION: SEQ ID NO:78:
   CC ATG GAG GAG AAG CTG AAG GCG ATG GAG GAG AAG CTG AAG GCG ATG      47
      Met Glu Glu Lys Leu Lys Ala Met Glu Glu Lys Leu Lys Ala Met
        1               5                  10                  15
   GAG GAG AAG CTG AAG GCG ATG GAG GAG AAG CTG AAG GCG ATG GAG GAG     95
   Glu Glu Lys Leu Lys Ala Met Glu Glu Lys Leu Lys Ala Met Glu Glu
                   20                  25                  30
   AAG CTG AAG GCG ATG GAG GAG AAG CTG AAG GCG ATG GAG GAA AAG ATG    143
   Lys Leu Lys Ala Met Glu Glu Lys Leu Lys Ala Met Glu Glu Lys Met
               35                  40                  45
   AAG GCG ATG GAA GAG AAG ATG AAG GCG TGATAGGTAC CGAATTC             187
   Lys Ala Met Glu Glu Lys Met Lys Ala
           50                  55
(2) INFORMATION FOR SEQ ID NO:79:
   (i) SEQUENCE CHARACTERISTICS:
      (A) LENGTH: 56 amino acids
      (B) TYPE: amino acid
      (D) TOPOLOGY: linear
   (ii) MOLECULE TYPE: protein
   (xi) SEQUENCE DESCRIPTION: SEQ ID NO:79:
   Met Glu Glu Lys Leu Lys Ala Met Glu Glu Lys Leu Lys Ala Met Glu
    1               5                  10                  15
   Glu Lys Leu Lys Ala Met Glu Glu Lys Leu Lys Ala Met Glu Glu Lys
               20                  25                  30
   Leu Lys Ala Met Glu Glu Lys Leu Lys Ala Met Glu Glu Lys Met Lys
           35                  40                  45
   Ala Met Glu Glu Lys Met Lys Ala
       50                  55
(2) INFORMATION FOR SEQ ID NO:80:
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..61
      (D) OTHER INFORMATION: /product= "synthetic oligonucleotide" /standard_name= "SM 107"
   (xi) SEQUENCE DESCRIPTION: SEQ ID NO:80:
   CATGGAGGAG AAGATGAAAA AGCTCGAAGA GAAGATGAAG GTCATGAAGT GATAGGTACC 60
   G                                                                 61
(2) INFORMATION FOR SEQ ID NO:81:
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..61
      (D) OTHER INFORMATION: /product= "synthetic oligonucleotide" /standard_name= "SM 106"
   (xi) SEQUENCE DESCRIPTION: SEQ ID NO:81:
   AATTCGGTAC CTATCACTTC ATGACCTTCA TCTTCTCTTC GAGCTTTTTC ATCTTCTCCT 60
   C                                                                 61
(2) INFORMATION FOR SEQ ID NO:82:
   (i) SEQUENCE CHARACTERISTICS:
      (A) LENGTH: 16 amino acids
      (B) TYPE: amino acid
      (C) STRANDEDNESS: unknown
      (D) TOPOLOGY: unknown
   (ii) MOLECULE TYPE: protein
   (ix) FEATURE:
      (A) NAME/KEY: Protein
      (B) LOCATION: 1..16
      (D) OTHER INFORMATION: /label= name /note= "pSK34 base gene"
   (xi) SEQUENCE DESCRIPTION: SEQ ID NO:82:
   Met Glu Glu Lys Met Lys Lys Leu Glu Glu Lys Met Lys Val Met Lys
   1               5                   10                  15
(2) INFORMATION FOR SEQ ID NO:83:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..63
      (D) OTHER INFORMATION: /product= "synthetic oligonucleotide" /standard_name= "SM 110"
   (xi) SEQUENCE DESCRIPTION: SEQ ID NO:83:
   GCTGGAAGAA AAGATGAAGG CTATGGAGGA CAAGATGAAA TGGCTTGAGG AAAAGATGAA 60
   GAA 63
(2) INFORMATION FOR SEQ ID NO:84:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..63
      (D) OTHER INFORMATION: /product= "synthetic oligonucleotide" /standard_name= "SM 111"
   (xi) SEQUENCE DESCRIPTION: SEQ ID NO:84:
   AGCTTCTTCA TCTTTTCCTC AAGCCATTTC ATCTTGTCCT CCATAGCCTT CATCTTTTCT 60
   TCC 63
(2) INFORMATION FOR SEQ ID NO:85:
   (i) SEQUENCE CHARACTERISTICS:
      (A) LENGTH: 37 amino acids
      (B) TYPE: amino acid
      (D) TOPOLOGY: linear
   (ii) MOLECULE TYPE: protein
   (xi) SEQUENCE DESCRIPTION: SEQ ID NO:85:
   Met Glu Glu Lys Met Lys Lys Leu Glu Glu Lys Met Lys Ala Met Glu
     1               5                  10                  15
   Asp Lys Met Lys Trp Leu Glu Glu Lys Met Lys Lys Leu Glu Glu Lys
               20                  25                  30
   Met Lys Val Met Lys
           35
(2) INFORMATION FOR SEQ ID NO:86:
   (i) SEQUENCE CHARACTERISTICS:
      (A) LENGTH: 37 amino acids
      (B) TYPE: amino acid
      (D) TOPOLOGY: linear
   (ii) MOLECULE TYPE: protein
   (xi) SEQUENCE DESCRIPTION: SEQ ID NO:86:
   Met Glu Glu Lys Met Lys Lys Leu Glu Glu Lys Met Lys Ala Met Glu
    1               5                  10                  15
   Asp Lys Met Lys Trp Leu Glu Glu Lys Met Lys Lys Leu Glu Glu Lys
               20                  25                  30
   Met Lys Val Met Lys
           35
(2) INFORMATION FOR SEQ ID NO:87:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..62
      (D) OTHER INFORMATION: /product= "synthetic oligonucletide" /standard_name= "SM 112"
   (xi) SEQUENCE DESCRIPTION: SEQ ID NO:87:
   GCTCGAAGAA AGATGAAGGC AATGGAAGAC AAAATGAAGT GGCTTGAGGA GAAAATGAAG 60
   AA                                                                62
(2) INFORMATION FOR SEQ ID NO:88:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..62
      (D) OTHER INFORMATION: /product= "synthetic oligonucleotide" /standard_name= "SM 113"
   (xi) SEQUENCE DESCRIPTION: SEQ ID NO:88:
   AGCTTCTTCA TTTTCTCCTC AAGCCACTTC ATTTTGTCTT CCATTGCCTT CATCTTTCTT 60
   CG                                                                62
(2) INFORMATION FOR SEQ ID NO:89:
   (i) SEQUENCE CHARACTERISTICS:
      (A) LENGTH: 37 amino acids
      (B) TYPE: amino acid
      (D) TOPOLOGY: linear
   (ii) MOLECULE TYPE: protein
   (xi) SEQUENCE DESCRIPTION: SEQ ID NO:89:
   Met Glu Glu Lys Met Lys Lys Leu Lys Glu Glu Met Ala Lys Met Lys
    1               5                  10                  15
   Asp Glu Met Trp Lys Leu Lys Glu Glu Met Lys Lys Leu Glu Glu Lys
               20                  25                  30
   Met Lys Val Met Lys
           35
(2) INFORMATION FOR SEQ ID NO:90:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..63
      (D) OTHER INFORMATION: /product= "synthetic oligonucleotide" /standard_name= "SM 114"
   (xi) SEQUENCE DESCRIPTION: SEQ ID NO:90:
   GCTCAAGGAG GAAATGGCTA AGATGAAAGA CGAAATCTGG AAACTGAAAG AGGAAATGAA 60
   GAA                                                               63
(2) INFORMATION FOR SEQ ID NO:91:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 1..63
      (D) OTHER INFORMATION: /product= "synthetic oligonucleotide" /standard_name= "SM 115"
   (xi) SEQUENCE DESCRIPTION: SEQ ID NO:91:
   AGCTTCTTCA TTTCCTCTTT CAGTTTCCAC ATTTCGTCTT TCATCTTAGC CATTTCCTCC 60
   TTG                                                               63
(2) INFORMATION FOR SEQ ID NO:92:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:92:
   Met Glu Glu Lys Met Lys Lys Leu Lys Glu Glu Met Ala Lys Met Lys
    1               5                  10                  15
   Asp Glu Met Trp Lys Leu Lys Glu Glu Met Lys Lys Leu Glu Glu Lys
               20                  25                  30
   Met Lys Val Met Glu Glu Lys Met Lys Lys Leu Glu Glu Lys Met Lys
           35                  40                  45
   Ala Met Glu Asp Lys Met Lys Trp Leu Glu Glu Lys Met Lys Lys Leu
       50                  55                  60
   Glu Glu Lys Met Lys Val Met Glu Glu Lys Met Lys Lys Leu Glu Glu
   65                  70                  75                  80
   Lys Met Lys Ala Met Glu Asp Lys Met Lys Trp Leu Glu Glu Lys Met
                   85                  90                  95
   Lys Lys Leu Glu Glu Lys Met Lys Val Met Lys
               100                 105
(2) INFORMATION FOR SEQ ID NO:93:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:93:
      CTAGAAGCCT CGGCAACGTC AGCAACGGCG GAAGAATCCG GTG 43
(2) INFORMATION FOR SEQ ID NO:94:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:94:
      CATGCACCGG ATTCTTCCGC CGTTGCTGAC GTTGCCGAGG CTT 43
(2) INFORMATION FOR SEQ ID NO:95:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:95:
      GATCCCATGG CGCCCCTTAA GTCCACCGCC AGCCTCCCCG TCGCCCGCCG CTCCT 55
(2) INFORMATION FOR SEQ ID NO:96:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:96:
      CTAGAGGAGC GGCGGGCGAC GGGGAGGCTG GCGGTGGACT TAAGGGGCGC CATGG 55
(2) INFORMATION FOR SEQ ID NO:97:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:97:
      CATGGCGCCC ACCGTGATGA TGGCCTCGTC GGCCACCGCC GTCGCTCCGT TCCAGGGGC 59
(2) INFORMATION FOR SEQ ID NO:98:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:98:
      TTAAGCCCCT GGAACGGAGC GACGGCGGTG GCCGACGAGG CCATCATCAC GGTGGGCGC 59
(2) INFORMATION FOR SEQ ID NO:99:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:99:
      GCGCCCACCG TGATGA 16
(2) INFORMATION FOR SEQ ID NO:100:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:100:
      CACCGGATTC TTCCGC 16